# EUROPEAN PATENT APPLICATION

(11) **EP 3 042 963 A1**
(43) Date of publication of application: **13.07.2016**
(21) Application number: 15194063.2
(22) Date of filing: 19.06.2006
(51) Int. Cl.: C12Q 1/68, C07H 21/04, B01L 3/00

(54) **METHODS OF DETECTING NUCLEIC ACIDS IN INDIVIDUAL CELLS AND OF IDENTIFYING RARE CELLS FROM LARGE HETEROGENEOUS CELL POPULATIONS**

(30) Priority: 20.06.2005 US 691834 P
(62) Divisional of application: 06785111.3
(71) Applicant: Advanced Cell Diagnostics, Inc., Hayward CA 94545 (US)
(72) Inventor: Luo, Yuling, San Ramon, CA 94582 (US); Chen, Shiping, Fremont, CA 94539 (US)
(74) Representative: Jones Day

(57) **Abstract**

The invention provides a method of detecting a nucleic acid target within a cell in a sample, the method comprising labeling the nucleic acid using at least two capture probes that capture a label probe to the nucleic acid target. The signal may be amplified with a help of one or more amplifiers and preamplifiers. The method is useful *inter alia* for identifying a cell of a certain type in a mixture of cells based. The invention further provides a kit for carrying out the method of the invention as well as a composition and a tissue section comprising a fixed and permeabilized cell with the labeling system as identified for the method of the invention.

## Description

### CROSS-REFERENCE TO RELATED APPLICATIONS

This application is a non-provisional utility patent application claiming priority to and benefit of the following prior provisional patent application: USSN 60/691,834, filed June 20, 2005, entitled "Method of Detecting and Enumerating Rare Cells from Large Heterogeneous Cell Populations" by Luo and Chen, which is incorporated herein by reference in its entirety for all purposes.

### FIELD OF THE INVENTION

The invention relates generally to nucleic acid chemistry and biochemical assays. More particularly, the invention relates to methods for in situ detection of nucleic acid analytes in single cells. The invention also relates to detection and identification of single cells, particularly rare cells.

### BACKGROUND OF THE INVENTION

Ample evidence has demonstrated that cancer cells can dissociate from the primary tumor and circulate in the lymph node, bone marrow, peripheral blood or other body fluids. These circulating tumor cells (CTC) have been shown to reflect the biological characteristics of the primary tumors, including the potential for metastasis development and tumor recurrence. Therefore, the detection of CTC may indicate disease recurrence, tumor cell spreading, and a high potential for distant metastasis. All of these are significant informative clinical factors in identifying high-risk cancer patients' disease status (e.g. Vogel et al., 2002; Gilbey et al., 2004; Molnar et al., 2003; Vlems et al., 2003; Ma et al., 2003).

Validation of the clinical utility of CTC detection as a prognostic indicator has not been progressing as fast as expected, in large part due to lack of suitable detection technologies. One key difficulty in detecting CTC in peripheral blood or other body fluids is that CTC are present in the circulation in extremely low concentrations, estimated to be in the range of one tumor cell among 10⁶-10⁷ normal white blood cells. As a result, any detection technology for this application has to exhibit exceptional sensitivity and specificity in order to limit both false negative and false positive rate to an acceptable level.

One existing approach incorporates immunomagnetic separation technology in detection of intact CTC (US 6,365,362; US 6,645,731). Using this technology, a blood sample from a cancer patient is incubated with magnetic beads coated with antibodies directed against an epithelial surface antigen as for example EpCAM (Cristofanilli et al., 2004). The magnetically labeled cells are then isolated using a magnetic separator. The immunomagnetically-enriched fraction is further processed for downstream analysis for CTC identification. Using this technology, it was shown in a prospective study that the number of CTC after treatment is an independent predictor of progression-free survival and overall survival in patients with metastatic breast cancer (Cristofanilli et al., 2004). Although this technology has reported high sensitivity, its applicability is limited by the availability of detection antibodies that are highly sensitive and specific to particular types of CTC. The antibodies can exhibit non-specific binding to other cellular components which can lead to low signal to noise ratio and impair later detection. The antibodies binding to CTC may also bind to antigen present in other types of cells at low level, resulting in a high level of false positives.

Another approach for determining the presence of CTC has been to test for the tumor cell specific expression of messenger RNA in blood. Real time reverse transcription-polymerase chain reaction (QPCR) has been used to correlate the detection of CTC with patient prognosis. Real-time RT-PCR has been used for detecting CEA mRNA in peripheral blood of colorectal cancer patients (Ito et al., 2002). Disease free survival of patients with positive CEA mRNA in post-operative blood was significantly shorter than in cases that were negative for CEA mRNA. These results suggest that tumor cells were shed into the bloodstream and resulted in poor patient outcomes in patients with colorectal cancer. Another report demonstrated the clinical utility of molecular detection of CTC in high-risk AJCC stage IIBC and IIIAB melanoma patients using multiple mRNA markers by QPCR (Mocellin et al., 2004). The advantage of detecting tumor specific mRNA expression is that any tumor-specific gene can be used to serve as a diagnostic/prognostic marker. However, the QPCR approach requires the laborious procedure of mRNA isolation from the blood sample and reverse transcription before the PCR reaction. False positives are often observed using this technique due to sample contamination by chromosomal DNA or low-level expression of the chosen marker gene in normal blood cells (Fava et al. 2001). In addition, the limit of detection sensitivity of this technique is at most about one tumor cell per 1 ml of blood, and the technology cannot provide an accurate count of CTC numbers.

It is highly desirable to detect and quantitate tumor cell specific mRNA expression at a single cell level in blood or other body fluids. A technology that can detect expression of multiple specific mRNAs in individual cells in suspension would allow both sensitive and specific detection and enumeration of CTC in blood or other body fluids. In addition, such technology could enable the collection of CTC cells for downstream cytological and molecular analysis. Currently available techniques do not fulfill these needs.

In situ hybridization (ISH) technology is an established method of localizing and detecting specific mRNA sequences in morphologically preserved tissue sections or cell preparations (Hicks et al., 2004). The most common specimens used are frozen sections, paraffin embedded sections or suspension cells that were cytospun onto glass slides and fixed with methanol. Detection is carried out using nucleic acid probes that are complementary to and hybridize with specific nucleotide sequences within cells and tissues. The sensitivity of the technique is such that threshold levels of detection are in the range of 10-20 copies of mRNA per cell.

However, ISH technology faces a number of technical challenges that limit its wide use. First of all, cells immobilized on solid surface exhibit poor hybridization kinetics. Secondly, assay optimization is generally required for a target mRNA in probe selection, labeling, and detection, for each tissue section in fixation and permeabilization, and in hybridization and washing. In addition, various experiments need to be performed to control for the specificity of the probe, for tissue mRNA quality, and for the hybridization efficacy of the experimental procedure. In addition to technical issues, current ISH technology has relatively low performance standards in term of its detection sensitivity and reproducibility. The false positive rate is still high unless the relevant cells are re-examined manually using their morphology, which is time and labor-intensive. Current ISH technology also does not have the capability to quantitatively determine the mRNA expression level or to simultaneously measure the expression of multiple target mRNA within cells, which may provide clinical valuable information such as increased detection sensitivity and specificity, and the identification of primary tumor type, source and stage.

There are four main types of probes that are typically used in performing in situ hybridization within cells: oligonucleotide probes (usually 20-40 bases in length), single-stranded DNA probes (200-500 bases in length), double stranded DNA probes, or RNA probes (200-5000 bases in length). RNA probes are currently the most widely used probes for in situ hybridization as they have the advantage that RNA-RNA hybrids are very thermostable and are resistant to digestion by RNases. RNA probe is a direct labeling method that suffers a number of difficulties. First, separate labeled probes have to be prepared for detecting each mRNA of interest. Second, it is technically difficult to detect the expression of multiple mRNA of interest in situ at the same time. As a result, only sequential detection of multiple mRNAs using different labeling methods has recently been reported (Schrock et al, 1996; Kosman et al, 2004). Furthermore, with direct labeling methods, there is no good way to control for potential cross-hybridization with non-specific sequences in cells. Branched DNA (bDNA) in situ hybridization is an indirect labeling method for detecting mRNA in single cells (Player et al, 2001). This method uses a series of oligonucleotide probes that have one portion hybridizing to the specific mRNA of interest and another portion hybridizing to the bDNA for signal amplification and detection. bDNA ISH has the advantage of using unlabeled oligonucleotide probes for detecting every mRNA of interest and the signal amplification and detection are generic components in the assay. However, the gene specific probes in the bDNA ISH need to be theoretically screened against possible non-specific hybridization interactions with other mRNA sequences in the cells. The nonspecific hybridization of the oligonucleotide probes in bDNA ISH can become a serious problem when multiple of those probes have to be used for the detection of low abundance mRNAs. Similarly, although use of bDNA ISH to detect or quantitate multiple mRNAs is desirable, such nonspecific hybridization of the oligonucleotide probes is a potential problem.

The present invention overcomes the above noted difficulties and provides methods for detecting nucleic acids in and for identifying individual cells. A complete understanding of the invention will be obtained upon review of the following.

### SUMMARY OF THE INVENTION

Methods of detecting nucleic acid targets in single cells, including methods of detecting multiple targets in a single cell, are provided. Methods of detecting individual cells, particularly rare cells from large heterogeneous cell populations, through detection of nucleic acids are described. Related compositions, systems, and kits are also described.

A first general class of embodiments includes methods of detecting two or more nucleic acid targets in an individual cell. In the methods, a sample comprising the cell is provided. The cell comprises, or is suspected of comprising, a first nucleic acid target and a second nucleic acid target. A first label probe comprising a first label and a second label probe comprising a second label, wherein a first signal from the first label is distinguishable from a second signal from the second label, are provided. At least a first capture probe and at least a second capture probe are also provided.

The first capture probe is hybridized, in the cell, to the first nucleic acid target (when the first nucleic acid target is present in the cell), and the second capture probe is hybridized, in the cell, to the second nucleic acid target (when the second nucleic acid target is present in the cell). The first label probe is captured to the first capture probe and the second label probe is captured to the second capture probe, thereby capturing the first label probe to the first nucleic acid target and the second label probe to the second nucleic acid target. The first signal from the first label and the second signal from the second label are then detected. Since the first and second labels are associated with their respective nucleic acid targets through the capture probes, presence of the label(s) in the cell indicates the presence of the corresponding nucleic acid target(s) in the cell. The methods are optionally quantitative. Thus, an intensity of the first signal and an intensity of the second signal can be measured, and the intensity of the first signal can be correlated with a quantity of the first nucleic acid target in the cell while the intensity of the second signal is correlated with a quantity of the second nucleic acid target in the cell.

In one aspect, the label probes bind directly to the capture probes. For example, in one class of embodiments, a single first capture probe and a single second capture probe are provided, the first label probe is hybridized to the first capture probe, and the second label probe is hybridized to the second capture probe. In a related class of embodiments, two or more first capture probes and two or more second capture probes are provided, as are a plurality of the first label probes (e.g., two or more identical first label probes) and a plurality of the second label probes (e.g., two or more identical second label probes). The two or more first capture probes are hybridized to the first nucleic acid target, and the two or more second capture probes are hybridized to the second nucleic acid target. A single first label probe is hybridized to each of the first capture probes, and a single second label probe is hybridized to each of the second capture probes.

In another aspect, the label probes are captured to the capture probes indirectly, for example, through binding of preamplifiers and/or amplifiers. In one class of embodiments in which amplifiers are employed, a single first capture probe, a single second capture probe, a plurality of the first label probes, and a plurality of the second label probes are provided. A first amplifier is hybridized to the first capture probe and to the plurality of first label probes, and a second amplifier is hybridized to the second capture probe and to the plurality of second label probes. In another class of embodiments, two or more first capture probes, two or more second capture probes, a plurality of the first label probes, and a plurality of the second label probes are provided. The two or more first capture probes are hybridized to the first nucleic acid target, and the two or more second capture probes are hybridized to the second nucleic acid target. A first amplifier is hybridized to each of the first capture probes, and the plurality of first label probes is hybridized to the first amplifiers. A second amplifier is hybridized to each of the second capture probes, and the plurality of second label probes is hybridized to the second amplifiers.

In one class of embodiments in which preamplifiers are employed, a single first capture probe, a single second capture probe, a plurality of the first label probes, and a plurality of the second label probes are provided. A first preamplifier is hybridized to the first capture probe, a plurality of first amplifiers is hybridized to the first preamplifier, and the plurality of first label probes is hybridized to the first amplifiers. A second preamplifier is hybridized to the second capture probe, a plurality of second amplifiers is hybridized to the second preamplifier, and the plurality of second label probes is hybridized to the second amplifiers. In another class of embodiments, two or more first capture probes, two or more second capture probes, a plurality of the first label probes, and a plurality of the second label probes are provided. The two or more first capture probes are hybridized to the first nucleic acid target, and the two or more second capture probes are hybridized to the second nucleic acid target. A first preamplifier is hybridized to each of the first capture probes, a plurality of first amplifiers is hybridized to each of the first preamplifiers, and the plurality of first label probes is hybridized to the first amplifiers. A second preamplifier is hybridized to each of the second capture probes, a plurality of second amplifiers is hybridized to each of the second preamplifiers, and the plurality of second label probes is hybridized to the second amplifiers.

In embodiments in which two or more first capture probes and/or two or more second capture probes are employed, the capture probes preferably hybridize to nonoverlapping polynucleotide sequences in their respective nucleic acid target.

In one class of embodiments, a plurality of the first label probes and a plurality of the second label probes are provided. A first amplified polynucleotide is produced by rolling circle amplification of a first circular polynucleotide hybridized to the first capture probe. The first circular polynucleotide comprises at least one copy of a polynucleotide sequence identical to a polynucleotide sequence in the first label probe, and the first amplified polynucleotide thus comprises a plurality of copies of a polynucleotide sequence complementary to the polynucleotide sequence in the first label probe. The plurality of first label probes is then hybridized to the first amplified polynucleotide. Similarly, a second amplified polynucleotide is produced by rolling circle amplification of a second circular polynucleotide hybridized to the second capture probe. The second circular polynucleotide comprises at least one copy of a polynucleotide sequence identical to a polynucleotide sequence in the second label probe, and the second amplified polynucleotide thus comprises a plurality of copies of a polynucleotide sequence complementary to the polynucleotide sequence in the second label probe. The plurality of second label probes is then hybridized to the second amplified polynucleotide. The amplified polynucleotides remain associated with the capture probe(s), and the label probes are thus captured to the nucleic acid targets.

The methods are useful for multiplex detection of nucleic acids, including simultaneous detection of more than two nucleic acid targets. Thus, the cell optionally comprises or is suspected of comprising a third nucleic acid target, and the methods optionally include: providing a third label probe comprising a third label, wherein a third signal from the third label is distinguishable from the first and second signals, providing at least a third capture probe, hybridizing in the cell the third capture probe to the third nucleic acid target (when present in the cell), capturing the third label probe to the third capture probe, and detecting the third signal from the third label. Fourth, fifth, sixth, etc. nucleic acid targets are similarly simultaneously detected in the cell if desired. Each hybridization or capture step is preferably accomplished for all of the nucleic acid targets at the same time.

A nucleic acid target can be essentially any nucleic acid that is desirably detected in the cell. For example, a nucleic acid target can be a DNA, a chromosomal DNA, an RNA, an mRNA, a microRNA, a ribosomal RNA, or the like. The nucleic acid target can be a nucleic acid endogenous to the cell. As another example, the target can be a nucleic acid introduced to or expressed in the cell by infection of the cell with a pathogen, for example, a viral or bacterial genomic RNA or DNA, a plasmid, a viral or bacterial mRNA, or the like.

The first and second (and/or optional third, fourth, etc.) nucleic acid targets can be part of a single nucleic acid molecule, or they can be separate molecules. In one class of embodiments, the first nucleic acid target is a first mRNA and the second nucleic acid target is a second mRNA. In another class of embodiments, the first nucleic acid target comprises a first region of an mRNA and the second nucleic acid target comprises a second region of the same mRNA. In another class of embodiments, the first nucleic acid target comprises a first chromosomal DNA polynucleotide sequence and the second nucleic acid target comprises a second chromosomal DNA polynucleotide sequence. The first and second chromosomal DNA polynucleotide sequences are optionally located on the same chromosome, e.g., within the same gene, or on different chromosomes.

In one aspect, the signal(s) from nucleic acid target(s) are normalized. In one class of embodiments, the second nucleic acid target comprises a reference nucleic acid, and the method includes normalizing the first signal to the second signal. The label (first, second, third, etc.) can be essentially any convenient label that directly or indirectly provides a detectable signal. In one aspect, the first label is a first fluorescent label and the second label is a second fluorescent label.

The methods can be used to detect the presence of the nucleic acid targets in cells from essentially any type of sample. For example, the sample can be derived from a bodily fluid such as blood. The methods for detecting nucleic acid targets in cells can be used to identify the cells. For example, a cell can be identified as being of a desired type based on which nucleic acids, and in what levels, it contains. Thus, in one class of embodiments, the methods include identifying the cell as a desired target cell based on detection of the first and second signals (and optional third, fourth, etc. signals) from within the cell. As just a few examples, the cell can be a circulating tumor cell, a virally infected cell, a fetal cell in maternal blood, a bacterial cell or other microorganism in a biological sample, or an endothelial cell, precursor endothelial cell, or myocardial cell in blood.

The cell is typically fixed and permeabilized before hybridization of the capture probes, to retain the nucleic acid targets in the cell and to permit the capture probes, label probes, etc. to enter the cell. The cell is optionally washed to remove materials not captured to one of the nucleic acid targets. The cell can be washed after any of various steps, for example, after hybridization of the capture probes to the nucleic acid targets to remove unbound capture probes, after hybridization of the preamplifiers, amplifiers, and/or label probes to the capture probes, and/or the like. It will be evident that double-stranded nucleic acid target(s) are preferably denatured, e.g., by heat, prior to hybridization of the corresponding capture probe(s) to the target(s).

Preferably, the cell is in suspension for all or most of the steps of the method. Thus, in one class of embodiments, the cell is in suspension in the sample comprising the cell, and/or the cell is in suspension during the hybridizing, capturing, and/or detecting steps. In other embodiments, the cell is in suspension in the sample comprising the cell, and the cell is fixed on a substrate during the hybridizing, capturing, and/or detecting steps. For example, the cell can be in suspension during the hybridization, capturing, and optional washing steps and immobilized on a substrate during the detection step. In embodiments in which the cell is in suspension, the first and second (and optional third, etc.) signals can be conveniently detected by flow cytometry. Signals from the labels are typically detected in a single operation.

One general class of embodiments provides methods of assaying a relative level of one or more target nucleic acids in an individual cell. In the methods, a sample comprising the cell is provided. The cell comprises or is suspected of comprising a first, target nucleic acid, and it comprises a second, reference nucleic acid. A first label probe comprising a first label and a second label probe comprising a second label, wherein a first signal from the first label is distinguishable from a second signal from the second label, are also provided. In the cell, the first label probe is captured to the first, target nucleic acid (when present in the cell) and the second label probe is captured to the second, reference nucleic acid. The first signal from the first label and the second signal from the second label are then detected in the individual cell, and the intensity of each signal is measured. The intensity of the first signal is normalized to the intensity of the second (reference) signal. The level of the first, target nucleic acid relative to the level of the second, reference nucleic acid in the cell is thereby assayed, since the first and second labels are associated with their respective nucleic acids. The methods are optionally quantitative, permitting measurement of the amount of the first, target nucleic acid relative to the amount of the second, reference nucleic acid in the cell. Thus, the intensity of the first signal normalized to that of the second signal can be correlated with a quantity of the first, target nucleic acid present in the cell.

The label probes can bind directly to the nucleic acids. For example, the first label probe can hybridize to the first, target nucleic acid and/or the second label probe can hybridize to the second, reference nucleic acid. Alternatively, the label probes can be bound indirectly to the nucleic acids, e.g., via capture probes. In one class of embodiments, at least a first capture probe and at least a second capture probe are provided. In the cell, the first capture probe is hybridized to the first, target nucleic acid and the second capture probe is hybridized to the second, reference nucleic acid. The first label probe is captured to the first capture probe and the second label probe is captured to the second capture probe, thereby capturing the first label probe to the first, target nucleic acid and the second label probe to the second, reference nucleic acid. The features described for the methods above apply to these embodiments as well, with respect to configuration and number of the label and capture probes, optional use of preamplifiers and/or amplifiers, rolling circle amplification of circular polynucleotides, and the like.

The methods can be used for multiplex detection of nucleic acids, including simultaneous detection of two or more target nucleic acids. Thus, the cell optionally comprises or is suspected of comprising a third, target nucleic acid, and the methods optionally include: providing a third label probe comprising a third label, wherein a third signal from the third label is distinguishable from the first and second signals; capturing, in the cell, the third label probe to the third, target nucleic acid (when present in the cell); detecting the third signal from the third label, which detecting comprises measuring an intensity of the third signal; and normalizing the intensity of the third signal to the intensity of the second signal. Fourth, fifth, sixth, etc. nucleic acids are similarly simultaneously detected in the cell if desired.

The methods for assaying relative levels of target nucleic acids in cells can be used to identify the cells. For example, a cell can be identified as being of a desired type based on which nucleic acids, and in what levels, it contains. Thus, in one class of embodiments, the methods include identifying the cell as a desired target cell based on the normalized first signal (and optional normalized third, fourth, etc. signals).

Essentially all of the features noted for the methods above apply to these embodiments as well, as relevant; for example, with respect to type of target and reference nucleic acids, cell type, source of sample, fixation and permeabilization of the cell, washing the cell, denaturation of double-stranded target and reference nucleic acids, type of labels, use of optional blocking probes, detection of signals, detection (and intensity measurement) by flow cytometry or microscopy, presence of the cell in suspension or immobilized on a substrate, and/or the like.

Another general class of embodiments provides methods of performing comparative gene expression analysis in single cells. In the methods, a first mixed cell population comprising one or more cells of a specified type is provided. An expression level of one or more target nucleic acids relative to a reference nucleic acid is measured in the cells of the specified type of the first population, to provide a first expression profile. A second mixed cell population comprising one or more cells of the specified type is also provided, and an expression level of the one or more target nucleic acids relative to the reference nucleic acid is measured in the cells of the specified type of the second population, to provide a second expression profile. The first and second expression profiles are then compared.

Essentially all of the features noted for the methods above apply to these embodiments as well, as relevant; for example, with respect to type of target and reference nucleic acids, cell type, source of sample, fixation and permeabilization of the cell, washing the cell, denaturation of double-stranded target and reference nucleic acids, type of labels, use and configuration of label probes, capture probes, preamplifiers and/or amplifiers, use of optional blocking probes, detection of signals, detection (and intensity measurement) by flow cytometry or microscopy, presence of the cell in suspension or immobilized on a substrate, and/or the like.

In one aspect, the invention provides methods that facilitate association of a high density of labels to target nucleic acids in cells. One general class of embodiments provides methods of detecting two or more nucleic acid targets in an individual cell. In the methods, a sample comprising the cell is provided. The cell comprises or is suspected of comprising a first nucleic acid target and a second nucleic acid target. In the cell, a first label is captured to the first nucleic acid target (when present in the cell) and a second label is captured to the second nucleic acid target (when present in the cell). A first signal from the first label is distinguishable from a second signal from the second label. As noted, the labels are captured at high density. Thus, an average of at least one copy of the first label per nucleotide of the first nucleic acid target is captured to the first nucleic acid target over a region that spans at least 20 contiguous nucleotides of the first nucleic acid target, and an average of at least one copy of the second label per nucleotide of the second nucleic acid target is captured to the second nucleic acid target over a region that spans at least 20 contiguous nucleotides of the second nucleic acid target. The first signal from the first label and the second signal from the second label are detected.

In one class of embodiments, an average of at least four, eight, or twelve copies of the first label per nucleotide of the first nucleic acid target are captured to the first nucleic acid target over a region that spans at least 20 contiguous nucleotides of the first nucleic acid target, and an average of at least four, eight, or twelve copies of the second label per nucleotide of the second nucleic acid target are captured to the second nucleic acid target over a region that spans at least 20 contiguous nucleotides of the second nucleic acid target. In one embodiment, an average of at least sixteen copies of the first label per nucleotide of the first nucleic acid target are captured to the first nucleic acid target over a region that spans at least 20 contiguous nucleotides of the first nucleic acid target, and an average of at least sixteen copies of the second label per nucleotide of the second nucleic acid target are captured to the second nucleic acid target over a region that spans at least 20 contiguous nucleotides of the second nucleic acid target.

Essentially all of the features noted for the methods above apply to these embodiments as well, as relevant, for example, with respect to type of labels, detection of signals, type, treatment, and suspension of the cell, and/or the like. A like density of third, fourth, fifth, sixth, etc. labels is optionally captured to third, fourth, fifth, sixth, etc. nucleic acid targets.

Another general class of embodiments provides methods of detecting an individual cell of a specified type. In the methods, a sample comprising a mixture of cell types including at least one cell of the specified type is provided. A first label probe comprising a first label and a second label probe comprising a second label, wherein a first signal from the first label is distinguishable from a second signal from the second label, are provided. In the cell, the first label probe is captured to a first nucleic acid target (when the first nucleic acid target is present in the cell) and the second label probe is captured to a second nucleic acid target (when the second nucleic acid target is present in the cell). The first signal from the first label and the second signal from the second label are detected and correlated with the presence, absence, or amount of the corresponding, first and second nucleic acid targets in the cell. The cell is identified as being of the specified type based on detection of the presence, absence, or amount of both the first and second nucleic acid targets within the cell, where the specified type of cell is distinguishable from the other cell type(s) in the mixture on the basis of either the presence, absence, or amount of the first nucleic acid target or the presence, absence, or amount of the second nucleic acid target in the cell (that is, the nucleic acid targets are redundant markers for the specified cell type). An intensity of the first signal and an intensity of the second signal are optionally measured and correlated with a quantity of the corresponding nucleic acid present in the cell. In one class of embodiments, the cell comprises a first nucleic acid target and a second nucleic acid target, and the cell is identified as being of the specified type based on detection of the presence or amount of both the first and second nucleic acid targets within the cell, where the specified type of cell is distinguishable from the other cell type(s) in the mixture on the basis of either the presence or amount of the first nucleic acid target or the presence or amount of the second nucleic acid target in the cell.

The label probes can bind directly to the nucleic acid targets. For example, the first label probe can hybridize to the first nucleic acid target and/or the second label probe can hybridize to the second nucleic acid target. The label probes are optionally captured to the nucleic acid targets via capture probes. In one class of embodiments, at least a first capture probe and at least a second capture probe are provided. In the cell, the first capture probe is hybridized to the first nucleic acid target and the second capture probe is hybridized to the second nucleic acid target. The first label probe is captured to the first capture probe and the second label probe is captured to the second capture probe, thereby capturing the first label probe to the first nucleic acid target and the second label probe to the second nucleic acid target. The features described for the methods above apply to these embodiments as well, with respect to configuration and number of the label and capture probes, optional use of preamplifiers and/or amplifiers, rolling circle amplification of circular polynucleotides, and the like.

Third, fourth, fifth, etc. nucleic acid targets are optionally detected in the cell. For example, the method optionally includes: providing a third label probe comprising a third label, wherein a third signal from the third label is distinguishable from the first and second signals, capturing in the cell the third label probe to a third nucleic acid target (when the third target is present in the cell), and detecting the third signal from the third label. The third, fourth, fifth, etc. label probes are optionally hybridized directly to their corresponding nucleic acid, or they can be captured indirectly via capture probes as described for the first and second label probes.

The first and/or second signal can be normalized to the third signal. Thus, in some embodiments, the cell comprises the third nucleic acid target, and the methods include identifying the cell as being of the specified type based on the normalized first and/or second signal, e.g., in embodiments in which the target cell type is distinguishable from the other cell type(s) in the mixture based on the copy number of the first and/or second nucleic acid targets, rather than purely on their presence in the target cell type and not in the other cell type(s).

As another example, the third nucleic acid target can serve as a third redundant marker for the target cell type, e.g., to improve specificity of the assay for the desired cell type. Thus, in one class of embodiments, the methods include correlating the third signal detected from the cell with the presence, absence, or amount of the third nucleic acid target in the cell, and identifying the cell as being of the specified type based on detection of the presence, absence, or amount of the first, second, and third nucleic acid targets within the cell, wherein the specified type of cell is distinguishable from the other cell type(s) in the mixture on the basis of either presence, absence, or amount of the first nucleic acid target, presence, absence, or amount of the second nucleic acid target, or presence, absence, or amount of the third nucleic acid target in the cell.

The methods can be applied to detection and identification of even rare cell types. For example, the ratio of cells of the specified type to cells of all other type(s) in the mixture is optionally less than 1:1x10⁴, less than 1:1x10⁵, less than 1:1x10⁶, less than 1:1x10⁷, less than 1:1x10⁸, or even less than 1:1x10⁹.

Essentially all of the features noted for the methods above apply to these embodiments as well, as relevant; for example, with respect to type of nucleic acid targets, cell type, source of sample, fixation and permeabilization of the cell, washing the cell, denaturation of double-stranded nucleic acids, type of labels, use of optional blocking probes, detection of signals, detection (and intensity measurement) of signals from the individual cell by flow cytometry or microscopy, presence of the cell in suspension or immobilized on a substrate, and/or the like.

The invention also provides compositions useful in practicing or produced by the methods. One exemplary class of embodiments provides a composition that includes a fixed and permeabilized cell, which cell comprises or is suspected of comprising a first nucleic acid target and a second nucleic acid target, at least a first capture probe capable of hybridizing to the first nucleic acid target, at least a second capture probe capable of hybridizing to the second nucleic acid target, a first label probe comprising a first label, and a second label probe comprising a second label. A first signal from the first label is distinguishable from a second signal from the second label. The cell optionally comprises the first and second capture probes and label probes. The first and second capture probes are optionally hybridized to their respective nucleic acid targets in the cell.

The features described for the methods above for indirect capture of the label probes to the nucleic acid targets apply to these embodiments as well, for example, with respect to configuration and number of the label and capture probes, optional use of preamplifiers and/or amplifiers, and the like.

In one class of embodiments, the composition comprises a plurality of the first label probes, a plurality of the second label probes, a first amplified polynucleotide produced by rolling circle amplification of a first circular polynucleotide hybridized to the first capture probe, and a second amplified polynucleotide produced by rolling circle amplification of a second circular polynucleotide hybridized to the second capture probe. The first circular polynucleotide comprises at least one copy of a polynucleotide sequence identical to a polynucleotide sequence in the first label probe, and the first amplified polynucleotide comprises a plurality of copies of a polynucleotide sequence complementary to the polynucleotide sequence in the first label probe. The second circular polynucleotide comprises at least one copy of a polynucleotide sequence identical to a polynucleotide sequence in the second label probe, and the second amplified polynucleotide comprises a plurality of copies of a polynucleotide sequence complementary to the polynucleotide sequence in the second label probe. The composition can also include reagents necessary for producing the amplified polynucleotides, for example, an exogenously supplied nucleic acid polymerase, an exogenously supplied nucleic acid ligase, and/or exogenously supplied nucleoside triphosphates (e.g., dNTPs).

The cell optionally includes additional nucleic acid targets, and the composition (and cell) can include reagents for detecting these targets. For example, the cell can comprise or be suspected of comprising a third nucleic acid target, and the composition can include at least a third capture probe capable of hybridizing to the third nucleic acid target and a third label probe comprising a third label. A third signal from the third label is distinguishable from the first and second signals. The cell optionally includes fourth, fifth, sixth, etc. nucleic acid targets, and the composition optionally includes fourth, fifth, sixth, etc. label probes and capture probes.

The cell can be present in a mixture of cells, for example, a complex heterogeneous mixture. In one class of embodiments, the cell is of a specified type, and the composition comprises one or more other types of cells. These other cells can be present in excess, even large excess, of the cell. For example, the ratio of cells of the specified type to cells of all other type(s) in the composition is optionally less than 1:1x10⁴, less than 1:1x10⁵, less than 1:1x10⁶, less than 1:1x10⁷, less than 1:1x10⁸, or even less than 1:1x10⁹.

Essentially all of the features noted for the methods above apply to these embodiments as well, as relevant; for example, with respect to type of nucleic acid target, type and source of cell, location of various targets on a single molecule or on different molecules, type of labels, inclusion of optional blocking probes, and/or the like. The cell is optionally in suspension in the composition.

One general class of embodiments provides a composition comprising a cell, which cell includes a first nucleic acid target, a second nucleic acid target, a first label wnose presence in the cell is indicative of the presence of the first nucleic acid target in the cell, and a second label whose presence in the cell is indicative of the presence of the second nucleic acid target in the cell, wherein a first signal from the first label is distinguishable from a second signal from the second label. An average of at least one copy of the first label is present in the cell per nucleotide of the first nucleic acid target over a region that spans at least 20 contiguous nucleotides of the first nucleic acid target, and an average of at least one copy of the second label is present in the cell per nucleotide of the second nucleic acid target over a region that spans at least 20 contiguous nucleotides of the second nucleic acid target.

In one class of embodiments, the copies of the first label are physically associated with the first nucleic acid target, and the copies of the second label are physically associated with the second nucleic acid target. For example, the first label can be part of a first label probe and the second label part of a second label probe, where the label probes are captured to the target nucleic acids.

Essentially all of the features noted for the embodiments above apply to these embodiments as well, as relevant, for example, with respect to type and number of labels, suspension of the cell, and/or the like. A like density of labels is optionally present for third, fourth, fifth, sixth, etc. nucleic acid targets.

Another aspect of the invention provides kits useful for practicing the methods. One general class of embodiments provides a kit for detecting a first nucleic acid target and a second nucleic acid target in an individual cell. The kit includes at least one reagent for fixing and/or permeabilizing the cell, at least a first capture probe capable of hybridizing to the first nucleic acid target, at least a second capture probe capable of hybridizing to the second nucleic acid target, a first label probe comprising a first label, and a second label probe comprising a second label, wherein a first signal from the first label is distinguishable from a second signal from the second label, packaged in one or more containers.

Essentially all of the features noted for the embodiments above apply to these embodiments as well, as relevant; for example, with respect to number of nucleic acid targets, configuration and number of the label and capture probes, inclusion of preamplifiers and/or amplifiers, inclusion of blocking probes, inclusion of amplification reagents, type of nucleic acid target, location of various targets on a single molecule or on different molecules, type of labels, inclusion of optional blocking probes, and/or the like.

Another general class of embodiments provides a kit for detecting an individual cell of a specified type from a mixture of cell types by detecting a first nucleic acid target and a second nucleic acid target. The kit includes at least one reagent for fixing and/or permeabilizing the cell, a first label probe comprising a first label, and a second label probe comprising a second label, wherein a first signal from the first label is distinguishable from a second signal from the second label, packaged in one or more containers. The specified type of cell is distinguishable from the other cell type(s) in the mixture by presence, absence, or amount of the first nucleic acid target in the cell or by presence, absence, or amount of the second nucleic acid target in the cell.

Essentially all of the features noted for the embodiments above apply to these embodiments as well, as relevant; for example, with respect to number of nucleic acid targets, inclusion of capture probes, configuration and number of the label and/or capture probes, inclusion of preamplifiers and/or amplifiers, inclusion of blocking probes, inclusion of amplification reagents, type of nucleic acid target, location of various targets on a single molecule or on different molecules, type of labels, inclusion of optional blocking probes, and/or the like.

### BRIEF DESCRIPTION OF THE DRAWINGS

**Figure 1** schematically illustrates QMAGEX technology workflow for an exemplary embodiment.
**Figure 2** schematically illustrates a direct labeling approach in which label probes are hybridized to the target nucleic acid.
**Figure 3** schematically illustrates an indirect labeling approach in which label probes are hybridized to capture probes hybridized to the target nucleic acid.
**Figure 4** schematically illustrates an indirect labeling capture probe design approach that utilizes a pair of independent capture probes to enhance the specificity of the label probe capture to the target nucleic acid.
**Figure 5** schematically illustrates an indirect labeling capture probe design approach that utilizes three or more independent capture probes to enhance the specificity of the label probe capture to the target nucleic acid.
**Figure 6** schematically illustrates probe design approaches to detect multiple target molecules in parallel using either direct labeling **(Panel A)** or indirect labeling with two independent capture probes **(Panel B).**

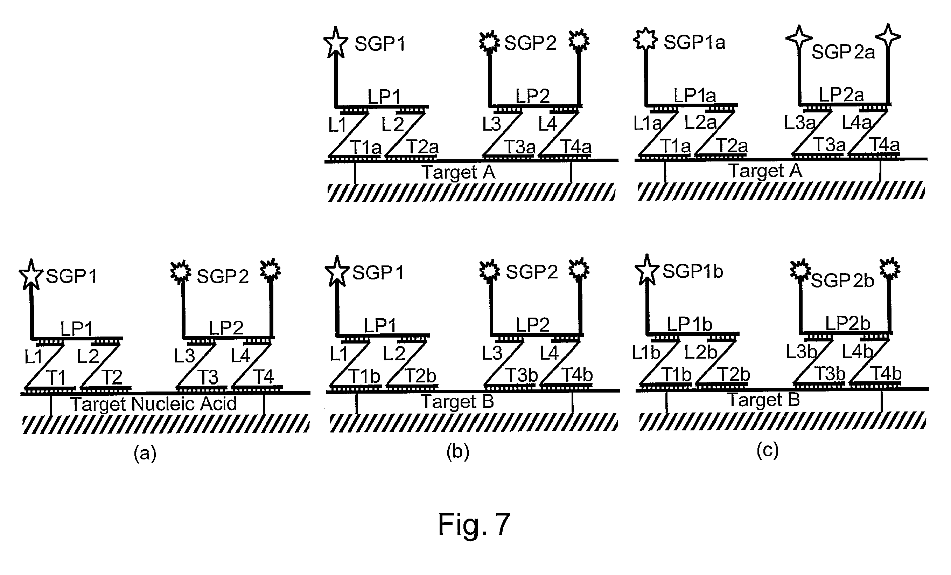

**Figure 7** schematically illustrates probe design approaches to reducing false positive rates in rare cell identification by attaching multiple types of signal-generating particles (labels) to the same target molecule. **Panel A** shows multiple types of signal-generating particles (labels) on one target. **Panel B** shows multiple types of signal-generating particles (labels) on more than one target, where the relative signal strengths of the particle set are maintained across all targets. **Panel C** shows a set of signal-generating particles (labels) on a target molecule, where different targets have distinctively different sets.
**Figure 8** **Panels A-D** schematically illustrate different structures of exemplary amplifiers.
**Figure 9** schematically illustrates utilizing rolling circle amplification to amplify signal. As shown in **Panel A,** a circular nucleotide molecule is attached to capture probe(s). As shown in **Panel B,** a long chain molecule with many repeated sequences appears as a result of rolling circle amplification. As shown in **Panel C,** many signal probes can be hybridized to the repeated sequences to achieve signal amplification.
**Figure 10** schematically illustrates one embodiment of the assay instrument configuration.

Schematic figures are not necessarily to scale.

### DEFINITIONS

Unless defined otherwise, all technical and scientific terms used herein have the same meaning as commonly understood by one of ordinary skill in the art to which the invention pertains. The following definitions supplement those in the art and are directed to the current application and are not to be imputed to any related or unrelated case, e.g., to any commonly owned patent or application. Although any methods and materials similar or equivalent to those described herein can be used in the practice for testing of the present invention, the preferred materials and methods are described herein. Accordingly, the terminology used herein is for the purpose of describing particular embodiments only, and is not intended to be limiting.

As used in this specification and the appended claims, the singular forms "a," "an" and "the" include plural referents unless the context clearly dictates otherwise. Thus, for example, reference to "a molecule" includes a plurality of such molecules, and the like.

The term "about" as used herein indicates the value of a given quantity varies by +/-10% of the value, or optionally +/- 5% of the value, or in some embodiments, by +/1% of the value so described.

The term "polynucleotide" (and the equivalent term "nucleic acid") encompasses any physical string of monomer units that can be corresponded to a string of nucleotides, including a polymer of nucleotides (e.g., a typical DNA or RNA polymer), peptide nucleic acids (PNAs), modified oligonucleotides (e.g., oligonucleotides comprising nucleotides that are not typical to biological RNA or DNA, such as 2'-O-methylated oligonucleotides), and the like. The nucleotides of the polynucleotide can be deoxyribonucleotides, ribonucleotides or nucleotide analogs, can be natural or non-natural, and can be unsubstituted, unmodified, substituted or modified. The nucleotides can be linked by phosphodiester bonds, or by phosphorothioate linkages, methylphosphonate linkages, boranophosphate linkages, or the like. The polynucleotide can additionally comprise non-nucleotide elements such as labels, quenchers, blocking groups, or the like. The polynucleotide can be, e.g., single-stranded or double-stranded.

A "nucleic acid target" or "target nucleic acid" refers to a nucleic acid, or optionally a region thereof, that is to be detected.

A "polynucleotide sequence" or "nucleotide sequence" is a polymer of nucleotides (an oligonucleotide, a DNA, a nucleic acid, etc.) or a character string representing a nucleotide polymer, depending on context. From any specified polynucleotide sequence, either the given nucleic acid or the complementary polynucleotide sequence (e.g., the complementary nucleic acid) can be determined.

The term "gene" is used broadly to refer to any nucleic acid associated with a biological function. Genes typically include coding sequences and/or the regulatory sequences required for expression of such coding sequences. The term gene can apply to a specific genomic sequence, as well as to a cDNA or an mRNA encoded by that genomic sequence. Genes also include non-expressed nucleic acid segments that, for example, form recognition sequences for other proteins. Non-expressed regulatory sequences include promoters and enhancers, to which regulatory proteins such as transcription factors bind, resulting in transcription of adjacent or nearby sequences.

Two polynucleotides "hybridize" when they associate to form a stable duplex, e.g., under relevant assay conditions. Nucleic acids hybridize due to a variety of well characterized physico-chemical forces, such as hydrogen bonding, solvent exclusion, base stacking and the like. An extensive guide to the hybridization of nucleic acids is found in Tijssen (1993) Laboratory Techniques in Biochemistry and Molecular Biology-Hybridization with Nucleic Acid Probes, part I chapter 2, "Overview of principles of hybridization and the strategy of nucleic acid probe assays" (Elsevier, New York), as well as in Ausubel, *infra.*

A first polynucleotide "capable of hybridizing" to a second polynucleotide contains a first polynucleotide sequence that is complementary to a second polynucleotide sequence in the second polynucleotide. The first and second polynucleotides are able to form a stable duplex, e.g., under relevant assay conditions.

The "Tₘ" (melting temperature) of a nucleic acid duplex under specified conditions (e.g., relevant assay conditions) is the temperature at which half of the base pairs in a population of the duplex are disassociated and half are associated. The Tₘ for a particular duplex can be calculated and/or measured, e.g., by obtaining a thermal denaturation curve for the duplex (where the Tₘ is the temperature corresponding to the midpoint in the observed transition from double-stranded to single-stranded form).

The term "complementary" refers to a polynucleotide that forms a stable duplex with its "complement," e.g., under relevant assay conditions. Typically, two polynucleotide sequences that are complementary to each other have mismatches at less than about 20% of the bases, at less than about 10% of the bases, preferably at less than about 5% of the bases, and more preferably have no mismatches.

A "label" is a moiety that facilitates detection of a molecule. Common labels in the context of the present invention include fluorescent, luminescent, light-scattering, and/or colorimetric labels. Suitable labels include enzymes and fluorescent moieties, as well as radionuclides, substrates, cofactors, inhibitors, chemiluminescent moieties, magnetic particles, and the like. Patents teaching the use of such labels include U.S. Pat. Nos. 3,817,837; 3,850,752; 3,939,350; 3,996,345; 4,277,437; 4,275,149; and 4,366,241. Many labels are commercially available and can be used in the context of the invention.

The term "label probe" refers to an entity that binds to a target molecule, directly or indirectly, and enables the target to be detected, e.g., by a readout instrument. A label probe (or "LP") is typically a single-stranded polynucleotide that comprises at least one label which directly or indirectly provides a detectable signal. The label can be covalently attached to the polynucleotide, or the polynucleotide can be configured to bind to the label (e.g., a biotinylated polynucleotide can bind a streptavidin-associated label). The label probe can, for example, hybridize directly to a target nucleic acid, or it can hybridize to a nucleic acid that is in turn hybridized to the target nucleic acid or to one or more other nucleic acids that are hybridized to the nucleic acid. Thus, the label probe can comprise a polynucleotide sequence that is complementary to a polynucleotide sequence of the target nucleic acid, or it can comprise at least one polynucleotide sequence that is complementary to a polynucleotide sequence in a capture probe, amplifier, or the like.

A "capture probe" is a polynucleotide that is capable of hybridizing to a target nucleic acid and capturing a label probe to that target nucleic acid. The capture probe can hybridize directly to the label probe, or it can hybridize to one or more nucleic acids that in turn hybridize to the label probe; for example, the capture probe can hybridize to an amplifier or a preamplifier. The capture probe thus includes a first polynucleotide sequence that is complementary to a polynucleotide sequence of the target nucleic acid and a second polynucleotide sequence that is complementary to a polynucleotide sequence of the label probe, amplifier, preamplifier, or the like. The capture probe is preferably single-stranded.

An "amplifier" is a molecule, typically a polynucleotide, that is capable of hybridizing to multiple label probes. Typically, the amplifier hybridizes to multiple identical label probes. The amplifier also hybridizes to at least one capture probe or nucleic acid bound to a capture probe. For example, the amplifier can hybridize to at least one capture probe and to a plurality of label probes, or to a preamplifier and a plurality of label probes. The amplifier can be, e.g., a linear, forked, comb-like, or branched nucleic acid. As noted for all polynucleotides, the amplifier can include modified nucleotides and/or nonstandard internucleotide linkages as well as standard deoxyribonucleotides, ribonucleotides, and/or phosphodiester bonds. Suitable amplifiers are described, for example, in USPN 5,635,352, USPN 5,124,246, USPN 5,710,264, and USPN 5,849,481.

A "preamplifier" is a molecule, typically a polynucleotide, that serves as an intermediate between one or more capture probes and amplifiers. Typically, the preamplifier hybridizes simultaneously to one or more capture probes and to a plurality of amplifiers. Exemplary preamplifiers are described, for example, in USPN 5,635,352 and USPN 5,681,697.

A "pathogen" is a biological agent, typically a microorganism, that causes disease or illness to its host.

A "microorganism" is an organism of microscopic or submicroscopic size. Examples include, but are not limited to, bacteria, fungi, yeast, protozoans, microscopic algae (e.g., unicellular algae), viruses (which are typically included in this category although they are incapable of growth and reproduction outside of host cells), subviral agents, viroids, and mycoplasma.

A variety of additional terms are defined or otherwise characterized herein.

### DETAILED DESCRIPTION

Among other aspects, the present invention provides multiplex assays that can be used for simultaneous detection, and optionally quantitation, of two or more nucleic acid targets in a single cell. A related aspect of the invention provides methods for detecting the level of one or more target nucleic acids relative to that of a reference nucleic acid in an individual cell.

In general, in the assays of the invention, a label probe is captured to each target nucleic acid. The label probe can be captured to the target through direct binding of the label probe to the target. Preferably, however, the label probe is captured indirectly through binding to capture probes, amplifiers, and/or preamplifiers that bind to the target. Use of the optional amplifiers and preamplifiers facilitates capture of multiple copies of the label probe to the target, thus amplifying signal from the target without requiring enzymatic amplification of the target itself. Binding of the capture probes is optionally cooperative, reducing background caused by undesired cross hybridization of capture probes to nontarget nucleic acids (a greater problem in multiplex assays than singleplex assays since more probes must be used in multiplex assays, increasing the likelihood of cross hybridization).

One aspect of the invention relates to detection of single cells, including detection of rare cells from a heterogeneous mixture of cells. Individual cells are detected through detection of nucleic acids whose presence, absence, copy number, or the like are characteristic of the cell.

Compositions, kits, and systems related to the methods are also provided.

### METHODS OF DETECTING NUCLEIC ACIDS AND CELLS

### Multiplex detection of nucleic acids

As noted, one aspect of the invention provides multiplex nucleic acid assays in single cells. Thus, one general class of embodiments includes methods of detecting two or more nucleic acid targets in an individual cell. In the methods, a sample comprising the cell is provided. The cell comprises, or is suspected of comprising, a first nucleic acid target and a second nucleic acid target. A first label probe comprising a first label and a second label probe comprising a second label, wherein a first signal from the first label is distinguishable from a second signal from the second label, are provided. At least a first capture probe and at least a second capture probe are also provided.

The first capture probe is hybridized, in the cell, to the first nucleic acid target (when the first nucleic acid target is present in the cell), and the second capture probe is hybridized, in the cell, to the second nucleic acid target (when the second nucleic acid target is present in the cell). The first label probe is captured to the first capture probe and the second label probe is captured to the second capture probe, thereby capturing the first label probe to the first nucleic acid target and the second label probe to the second nucleic acid target. The first signal from the first label and the second signal from the second label are then detected. Since the first and second labels are associated with their respective nucleic acid targets through the capture probes, presence of the label(s) in the cell indicates the presence of the corresponding nucleic acid target(s) in the cell. The methods are optionally quantitative. Thus, an intensity of the first signal and an intensity of the second signal can be measured, and the intensity of the first signal can be correlated with a quantity of the first nucleic acid target in the cell while the intensity of the second signal is correlated with a quantity of the second nucleic acid target in the cell.

In one aspect, the label probes bind directly to the capture probes. For example, in one class of embodiments, a single first capture probe and a single second capture probe are provided, the first label probe is hybridized to the first capture probe, and the second label probe is hybridized to the second capture probe. In a related class of embodiments, two or more first capture probes and two or more second capture probes are provided, as are a plurality of the first label probes (e.g., two or more identical first label probes) and a plurality of the second label probes (e.g., two or more identical second label probes). The two or more first capture probes are hybridized to the first nucleic acid target, and the two or more second capture probes are hybridized to the second nucleic acid target. A single first label probe is hybridized to each of the first capture probes, and a single second label probe is hybridized to each of the second capture probes.

In another aspect, the label probes are captured to the capture probes indirectly, for example, through binding of preamplifiers and/or amplifiers. Use of amplifiers and preamplifiers can be advantageous in increasing signal strength, since they can facilitate binding of large numbers of label probes to each nucleic acid target.

In one class of embodiments in which amplifiers are employed, a single first capture probe, a single second capture probe, a plurality of the first label probes, and a plurality of the second label probes are provided. A first amplifier is hybridized to the first capture probe and to the plurality of first label probes, and a second amplifier is hybridized to the second capture probe and to the plurality of second label probes. In another class of embodiments, two or more first capture probes, two or more second capture probes, a plurality of the first label probes, and a plurality of the second label probes are provided. The two or more first capture probes are hybridized to the first nucleic acid target, and the two or more second capture probes are hybridized to the second nucleic acid target. A first amplifier is hybridized to each of the first capture probes, and the plurality of first label probes is hybridized to the first amplifiers. A second amplifier is hybridized to each of the second capture probes, and the plurality of second label probes is hybridized to the second amplifiers.

In one class of embodiments in which preamplifiers are employed, a single first capture probe, a single second capture probe, a plurality of the first label probes, and a plurality of the second label probes are provided. A first preamplifier is hybridized to the first capture probe, a plurality of first amplifiers is hybridized to the first preamplifier, and the plurality of first label probes is hybridized to the first amplifiers. A second preamplifier is hybridized to the second capture probe, a plurality of second amplifiers is hybridized to the second preamplifier, and the plurality of second label probes is hybridized to the second amplifiers. In another class of embodiments, two or more first capture probes, two or more second capture probes, a plurality of the first label probes, and a plurality of the second label probes are provided. The two or more first capture probes are hybridized to the first nucleic acid target, and the two or more second capture probes are hybridized to the second nucleic acid target. A first preamplifier is hybridized to each of the first capture probes, a plurality of first amplifiers is hybridized to each of the first preamplifiers, and the plurality of first label probes is hybridized to the first amplifiers. A second preamplifier is hybridized to each of the second capture probes, a plurality of second amplifiers is hybridized to each of the second preamplifiers, and the plurality of second label probes is hybridized to the second amplifiers. Optionally, additional preamplifiers can be used as intermediates between a preamplifier hybridized to the capture probe(s) and the amplifiers.

In the above classes of embodiments, one capture probe hybridizes to each label probe, amplifier, or preamplifier. In alternative classes of related embodiments, two or more capture probes hybridize to the label probe, amplifier, or preamplifier. See, e.g., the section below entitled "Implementation, applications, and advantages."

In embodiments in which two or more first capture probes and/or two or more second capture probes are employed, the capture probes preferably hybridize to nonoverlapping polynucleotide sequences in their respective nucleic acid target. The capture probes can, but need not, cover a contiguous region of the nucleic acid target. Blocking probes, polynucleotides which hybridize to regions of the nucleic acid target not occupied by capture probes, are optionally provided and hybridized to the target. For a given nucleic acid target, the corresponding capture probes and blocking probes are preferably complementary to physically distinct, nonoverlapping sequences in the nucleic acid target, which nonoverlapping sequences are preferably, but not necessarily, contiguous. Having the capture probes and optional blocking probes be contiguous with each other can in some embodiments enhance hybridization strength, remove secondary structure, and ensure more consistent and reproducible signal.

In many embodiments, such as those above, enzymatic manipulation is not required to capture the label probes to the capture probes. In other embodiments, however, enzymatic manipulation, particularly amplification of nucleic acids intermediate between the capture probes and the label probes, facilitates detection of the nucleic acid targets. For example, in one class of embodiments, a plurality of the first label probes and a plurality of the second label probes are provided. A first amplified polynucleotide is produced by rolling circle amplification of a first circular polynucleotide hybridized to the first capture probe. The first circular polynucleotide comprises at least one copy of a polynucleotide sequence identical to a polynucleotide sequence in the first label probe, and the first amplified polynucleotide thus comprises a plurality of copies of a polynucleotide sequence complementary to the polynucleotide sequence in the first label probe. The plurality of first label probes is then hybridized to the first amplified polynucleotide. Similarly, a second amplified polynucleotide is produced by rolling circle amplification of a second circular polynucleotide hybridized to the second capture probe (preferably, at the same time the first amplified polynucleotide is produced). The second circular polynucleotide comprises at least one copy of a polynucleotide sequence identical to a polynucleotide sequence in the second label probe, and the second amplified polynucleotide thus comprises a plurality of copies of a polynucleotide sequence complementary to the polynucleotide sequence in the second label probe. The plurality of second label probes is then hybridized to the second amplified polynucleotide. The amplified polynucleotides remain associated (e.g., covalently) with the capture probe(s), and the label probes are thus captured to the nucleic acid targets. A circular polynucleotide can be provided and hybridized to the capture probe, or a linear polynucleotide that is circularized by ligation after it binds to the capture probe (e.g., a padlock probe) can be employed. Techniques for rolling circle amplification, including use of padlock probes, are well known in the art. See, e.g., Larsson et al. (2004) "In situ genotyping individual DNA molecules by target-primed rolling-circle amplification of padlock probes" Nat Methods. 1(3):227-32, Nilsson et al. (1994) Science 265:2085-2088, and Antson et al. (2000) "PCR-generated padlock probes detect single nucleotide variation in genomic DNA" Nucl Acids Res 28(12):E58.

Potential capture probe sequences are optionally examined for possible interactions with non-corresponding nucleic acid targets, the preamplifiers, the amplifiers, the label probes, and/or any relevant genomic sequences, for example. Sequences expected to cross-hybridize with undesired nucleic acids are typically not selected for use in the capture probes. Examination can be, e.g., visual (e.g., visual examination for complementarity), computational (e.g., a BLAST search of the relevant genomic database, or computation and comparison of binding free energies), and/or experimental (e.g., cross-hybridization experiments). Label probe sequences are preferably similarly examined, to help minimize potential undesirable cross-hybridization.

A capture probe, preamplifier, amplifier, and/or label probe optionally comprises at least one non-natural nucleotide. For example, a capture probe and a preamplifier (or amplifier or label probe) that hybridizes to it optionally comprise, at complementary positions, at least one pair of non-natural nucleotides that base pair with each other but that do not Watson-Crick base pair with the bases typical to biological DNA or RNA (i.e., A, C, G, T, or U). Examples of nonnatural nucleotides include, but are not limited to, Locked NucleicAcid™ nucleotides (available from Exiqon A/S, (www.) exiqon.com; see, e.g., SantaLucia Jr. (1998) Proc Natl Acad Sci 95:1460-1465) and isoG, isoC, and other nucleotides used in the AEGIS system (Artificially Expanded Genetic Information System, available from EraGen Biosciences, (www.) eragen.com; see, e.g., USPN 6,001,983, USPN 6,037,120, and USPN 6,140,496). Use of such non-natural base pairs (e.g., isoG-isoC base pairs) in the probes can, for example, reduce background and/or simplify probe design by decreasing cross hybridization, or it can permit use of shorter probes when the non-natural base pairs have higher binding affinities than do natural base pairs.

As noted, the methods are useful for multiplex detection of nucleic acids, including simultaneous detection of more than two nucleic acid targets. Thus, the cell optionally comprises or is suspected of comprising a third nucleic acid target, and the methods optionally include: providing a third label probe comprising a third label, wherein a third signal from the third label is distinguishable from the first and second signals, providing at least a third capture probe, hybridizing in the cell the third capture probe to the third nucleic acid target (when the third target is present in the cell), capturing the third label probe to the third capture probe, and detecting the third signal from the third label. Fourth, fifth, sixth, etc. nucleic acid targets are similarly simultaneously detected in the cell if desired.

A nucleic acid target can be essentially any nucleic acid that is desirably detected in the cell. For example, a nucleic acid target can be a DNA, a chromosomal DNA, an RNA, an mRNA, a microRNA, a ribosomal RNA, or the like. The nucleic acid target can be a nucleic acid endogenous to the cell. As another example, the target can be a nucleic acid introduced to or expressed in the cell by infection of the cell with a pathogen, for example, a viral or bacterial genomic RNA or DNA, a plasmid, a viral or bacterial mRNA, or the like.

The first and second (and/or optional third, fourth, etc.) nucleic acid targets can be part of a single nucleic acid molecule, or they can be separate molecules. Various advantages and applications of both approaches are discussed in greater detail below and in the section entitled "Implementation, applications, and advantages." In one class of embodiments, the first nucleic acid target is a first mRNA and the second nucleic acid target is a second mRNA. In another class of embodiments, the first nucleic acid target comprises a first region of an mRNA and the second nucleic acid target comprises a second region of the same mRNA; this approach can increase specificity of detection of the mRNA. In another class of embodiments, the first nucleic acid target comprises a first chromosomal DNA polynucleotide sequence and the second nucleic acid target comprises a second chromosomal DNA polynucleotide sequence. The first and second chromosomal DNA polynucleotide sequences are optionally located on the same chromosome, e.g., within the same gene, or on different chromosomes.

In one aspect, the signal(s) from nucleic acid target(s) are normalized. In one class of embodiments, the second nucleic acid target comprises a reference nucleic acid, and the method includes normalizing the first signal to the second signal. The reference nucleic acid is a nucleic acid selected as a standard of comparison. It will be evident that choice of the reference nucleic acid can depend on the desired application. For example, for gene expression analysis, where the first and optional third, fourth, etc. nucleic acid targets are mRNAs whose expression levels are to be determined, the reference nucleic acid can be an mRNA transcribed from a housekeeping gene. As another example, the first nucleic acid target can be an mRNA whose expression is altered in a pathological state, e.g., an mRNA expressed in a tumor cell and not a normal cell or expressed at a higher level in a tumor cell than in a normal cell, while the second nucleic acid target is an mRNA expressed from a housekeeping gene or similar gene whose expression is not altered in the pathological state. As yet another example, the first nucleic acid target can be a chromosomal DNA sequence that is amplified or deleted in a tumor cell, while the second nucleic acid target is another chromosomal DNA sequence that is maintained at its normal copy number in the tumor cell. Exemplary reference nucleic acids are described herein, and many more are well known in the art.

Optionally, results from the cell are compared with results from a reference cell. That is, the first and second targets are also detected in a reference cell, for example, a non-tumor, uninfected, or other healthy normal cell, chosen as a standard of comparison depending on the desired application. The signals can be normalized to a reference nucleic acid as noted above. As just one example, the first nucleic acid target can be the Her-2 gene, with the goal of measuring Her-2 gene amplification. Signal from Her-2 can be normalized to that from a reference gene, whose copy number is stably maintained in the genomic DNA. The normalized signal for the Her-2 gene from a target cell (e.g., a tumor cell or suspected tumor cell) can be compared to the normalized signal from a reference cell (e.g., a normal cell), to determine copy number in the cancer cell in comparison to normal cells.

The label (first, second, third, etc.) can be essentially any convenient label that directly or indirectly provides a detectable signal. In one aspect, the first label is a first fluorescent label and the second label is a second fluorescent label. Detecting the signal from the labels thus comprises detecting fluorescent signals from the labels. A variety of fluorescent labels whose signals can be distinguished from each other are known, including, e.g., fluorophores and quantum dots. As other examples, the label can be a luminescent label, a light-scattering label (e.g., colloidal gold particles), or an enzyme (e.g., alkaline phosphatase or horseradish peroxidase).

The methods can be used to detect the presence of the nucleic acid targets in cells from essentially any type of sample. For example, the sample can be derived from a bodily fluid, a bodily waste, blood, bone marrow, sputum, urine, lymph node, stool, vaginal secretions, cervical pap smear, oral swab or other swab or smear, spinal fluid, saliva, sputum, ejaculatory fluid, semen, lymph fluid, an intercellular fluid, a tissue (e.g., a tissue homogenate), a biopsy, and/or a tumor. The sample and/or the cell can be derived from one or more of a human, an animal, a plant, and a cultured cell. Samples derived from even relatively large volumes of materials such as bodily fluid or bodily waste can be screened in the methods of the invention, and removal of such materials is relatively non-invasive. Samples are optionally taken from a patient, following standard laboratory methods after informed consent.

The methods for detecting nucleic acid targets in cells can be used to identify the cells. For example, a cell can be identified as being of a desired type based on which nucleic acids, and in what levels, it contains. Thus, in one class of embodiments, the methods include identifying the cell as a desired target cell based on detection of the first and second signals (and optional third, fourth, etc. signals) from within the cell. The cell can be identified on the basis of the presence or absence of one or more of the nucleic acid targets. Similarly, the cell can be identified on the basis of the relative signal strength from or expression level of one or more of the nucleic acid targets. Signals are optionally normalized as noted above and/or compared to those from a reference cell.

The methods can be applied to detection and identification of even rare cell types. Thus, the sample including the cell can be a mixture of desired target cells and other, nontarget cells, which can be present in excess of the target cells. For example, the ratio of target cells to cells of all other type(s) in the sample is optionally less than 1:1x10⁴, less than 1:1x10⁵, less than 1:1x10⁶, less than 1:1x10⁷, less than 1:1x10⁸, or even less than 1:1x10⁹.

Essentially any type of cell that can be differentiated based on its nucleic acid content (presence, absence, expression level or copy number of one or more nucleic acids) can be detected and identified using the methods and a suitable choice of nucleic acid targets. As just a few examples, the cell can be a circulating tumor cell, a virally infected cell, a fetal cell in maternal blood, a bacterial cell or other microorganism in a biological sample (e.g., blood or other body fluid), an endothelial cell, precursor endothelial cell, or myocardial cell in blood, a stem cell, or a T-cell. Rare cell types can be enriched prior to performing the methods, if necessary, by methods known in the art (e.g., lysis of red blood cells, isolation of peripheral blood mononuclear cells, further enrichment of rare target cells through magnetic-activated cell separation (MACS), etc.). The methods are optionally combined with other techniques, such as DAPI staining for nuclear DNA. It will be evident that a variety of different types of nucleic acid markers are optionally detected simultaneously by the methods and used to identify the cell. For example, a cell can be identified based on the presence or relative expression level of one nucleic acid target in the cell and the absence of another nucleic acid target from the cell; e.g., a circulating tumor cell can be identified by the presence or level of one or more markers found in the tumor cell and not found (or found at different levels) in blood cells, and its identity can be confirmed by the absence of one or more markers present in blood cells and not circulating tumor cells. The principle may be extended to using any other type of markers such as protein based markers in single cells.

The cell is typically fixed and permeabilized before hybridization of the capture probes, to retain the nucleic acid targets in the cell and to permit the capture probes, label probes, etc. to enter the cell. The cell is optionally washed to remove materials not captured to one of the nucleic acid targets. The cell can be washed after any of various steps, for example, after hybridization of the capture probes to the nucleic acid targets to remove unbound capture probes, after hybridization of the preamplifiers, amplifiers, and/or label probes to the capture probes, and/or the like.

The various capture and hybridization steps can be performed simultaneously or sequentially, in essentially any convenient order. Preferably, a given hybridization step is accomplished for all of the nucleic acid targets at the same time. For example, all the capture probes (first, second, etc.) can be added to the cell at once and permitted to hybridize to their corresponding targets, the cell can be washed, amplifiers (first, second, etc.) can be hybridized to the corresponding capture probes, the cell can be washed, the label probes (first, second, etc.) can be hybridized to the corresponding amplifiers, and the cell can then be washed again prior to detection of the labels. As another example, the capture probes can be hybridized to the targets, the cell can be washed, amplifiers and label probes can be added together and hybridized, and the cell can then be washed prior to detection. It will be evident that double-stranded nucleic acid target(s) are preferably denatured, e.g., by heat, prior to hybridization of the corresponding capture probe(s) to the target(s).

Preferably, the cell is in suspension for all or most of the steps of the method, for ease of handling. However, the methods are also applicable to cells in solid tissue samples (e.g., tissue sections) and/or cells immobilized on a substrate (e.g., a slide or other surface). Thus, in one class of embodiments, the cell is in suspension in the sample comprising the cell, and/or the cell is in suspension during the hybridizing, capturing, and/or detecting steps. For example, the cell can be in suspension in the sample and during the hybridization, capture, optional washing, and detection steps. In other embodiments, the cell is in suspension in the sample comprising the cell, and the cell is fixed on a substrate during the hybridizing, capturing, and/or detecting steps. For example, the cell can be in suspension during the hybridization, capture, and optional washing steps and immobilized on a substrate during the detection step.

Signals from the labels can be detected, and their intensities optionally measured, by any of a variety of techniques well known in the art. For example, in embodiments in which the cell is in suspension, the first and second (and optional third, etc.) signals can be conveniently detected by flow cytometry. In embodiments in which cells are immobilized on a substrate, the first and second (and optional third etc.) signals can be detected, for example, by laser scanner or microscope, e.g., a fluorescent or automated scanning microscope. As noted, detection is at the level of individual, single cells. Signals from the labels are typically detected in a single operation (e.g., a single flow cytometry run or a single microscopy or scanning session), rather than sequentially in separate operations for each label. Such a single detection operation can, for example, involve changing optical filters between detection of the different labels, but it does not involve detection of the first label followed by capture of the second label and then detection of the second label. In some embodiments, the first and second (and optional third etc.) labels are captured to their respective targets simultaneously but are detected in separate detection steps or operations.

Additional features described herein, e.g., in the section below entitled "Implementation, applications, and advantages," can be applied to the methods, as relevant. For example, as described in greater detail below, a label probe can include more than one label, identical or distinct. Signal strength is optionally adjusted between targets depending on their expected copy numbers, if desired; for example, the signal for an mRNA expressed at low levels can be amplified to a greater degree (e.g., by use of more labels per label probe and/or use of preamplifiers and amplifiers to capture more label probes per copy of the target) than the signal for a highly expressed mRNA.

In another aspect of the invention, two or more nucleic acids are detected by PCR amplification of the nucleic acids in situ in individual cells. To prevent leakage of the resulting amplicons out of the cells, a water-oil emulsion can be made as mentioned in Li et al. (2006) "BEAMing up for detection and quantification of rare sequence variants" Nature Methods 3(2):95-7 that separates single cells into different compartments.

### Detection of relative levels by normalization to reference nucleic acids

As discussed briefly above, the signal detected for a nucleic acid of interest can be normalized to that of a standard, reference nucleic acid. One general class of embodiments thus provides methods of assaying a relative level of one or more target nucleic acids in an individual cell. In the methods, a sample comprising the cell is provided. The cell comprises or is suspected of comprising a first, target nucleic acid, and it comprises a second, reference nucleic acid. A first label probe comprising a first label and a second label probe comprising a second label, wherein a first signal from the first label is distinguishable from a second signal from the second label, are also provided. In the cell, the first label probe is captured to the first, target nucleic acid (when the first, target nucleic acid is present in the cell) and the second label probe is captured to the second, reference nucleic acid. The first signal from the first label and the second signal from the second label are then detected in the individual cell, and the intensity of each signal is measured. The intensity of the first signal is normalized to the intensity of the second (reference) signal. The level of the first, target nucleic acid relative to the level of the second, reference nucleic acid in the cell is thereby assayed, since the first and second labels are associated with their respective nucleic acids. The methods are optionally quantitative, permitting measurement of the amount of the first, target nucleic acid relative to the amount of the second, reference nucleic acid in the cell. Thus, the intensity of the first signal normalized to that of the second signal can be correlated with a quantity of the first, target nucleic acid present in the cell.

The label probes can bind directly to the nucleic acids. For example, the first label probe can hybridize to the first, target nucleic acid and/or the second label probe can hybridize to the second, reference nucleic acid. Alternatively, some or all of the label probes can be indirectly bound to their corresponding nucleic acids, e.g., through capture probes. For example, the first and second label probes can bind directly to the nucleic acids, or one can bind directly while the other binds indirectly, or both can bind indirectly.

The label probes are optionally captured to the nucleic acids via capture probes. In one class of embodiments, at least a first capture probe and at least a second capture probe are provided. In the cell, the first capture probe is hybridized to the first, target nucleic acid and the second capture probe is hybridized to the second, reference nucleic acid. The first label probe is captured to the first capture probe and the second label probe is captured to the second capture probe, thereby capturing the first label probe to the first, target nucleic acid and the second label probe to the second, reference nucleic acid. The features described for the methods above apply to these embodiments as well, with respect to configuration and number of the label and capture probes, optional use of preamplifiers and/or amplifiers, rolling circle amplification of circular polynucleotides, and the like.

The methods can be used for multiplex detection of nucleic acids, including simultaneous detection of two or more target nucleic acids. Thus, the cell optionally comprises or is suspected of comprising a third, target nucleic acid, and the methods optionally include: providing a third label probe comprising a third label, wherein a third signal from the third label is distinguishable from the first and second signals; capturing, in the cell, the third label probe to the third, target nucleic acid (when present in the cell); detecting the third signal from the third label, which detecting comprises measuring an intensity of the third signal; and normalizing the intensity of the third signal to the intensity of the second signal. Alternatively, the third signal can be normalized to that from a different reference nucleic acid. Fourth, fifth, sixth, etc. nucleic acids are similarly simultaneously detected in the cell if desired. The third, fourth, fifth, etc. label probes are optionally hybridized directly to their corresponding nucleic acid, or they can be captured indirectly via capture probes as described for the first and second label probes.

The methods can be used for gene expression analysis, detection of gene amplification or deletion, or detection or diagnosis of disease, as just a few examples. A target nucleic acid can be essentially any nucleic acid that is desirably detected in the cell. For example, a target nucleic acid can be a DNA, a chromosomal DNA, an RNA, an mRNA, a microRNA, a ribosomal RNA, or the like. The target nucleic acid can be a nucleic acid endogenous to the cell, or as another example, the target can be a nucleic acid introduced to or expressed in the cell by infection of the cell with a pathogen, for example, a viral or bacterial genomic RNA or DNA, a plasmid, a viral or bacterial mRNA, or the like. The reference nucleic acid can similarly be a DNA, an mRNA, a chromosomal DNA, an mRNA, an RNA endogenous to the cell, or the like.

As described above, choice of the reference nucleic acid can depend on the desired application. For example, for gene expression analysis, where the first and optional third, fourth, etc. target nucleic acids are mRNAs whose expression levels are to be determined, the reference nucleic acid can be an mRNA transcribed from a housekeeping gene. As another example, the first, target nucleic acid can be an mRNA whose expression is altered in a pathological state, e.g., an mRNA expressed in a tumor cell and not a normal cell or expressed at a higher level in a tumor cell than in a normal cell, while the reference nucleic acid is an mRNA expressed from a housekeeping gene or similar gene whose expression is not altered in the pathological state. In a similar example, the target nucleic acid can be a viral or bacterial nucleic acid while the reference nucleic acid is endogenous to the cell. As yet another example, the first, target nucleic acid can be a chromosomal DNA sequence that is amplified or deleted in a tumor cell, while the reference nucleic acid is another chromosomal DNA sequence that is maintained at its normal copy number in the tumor cell. Exemplary reference nucleic acids are described herein, and many more are well known in the art.

In one class of embodiments, the first, target nucleic acid is a first mRNA and the second, reference nucleic acid is a second mRNA. In another class of embodiments, the first, target nucleic acid comprises a first chromosomal DNA polynucleotide sequence and the second, reference nucleic acid comprises a second chromosomal DNA polynucleotide sequence. The first and second chromosomal DNA polynucleotide sequences are optionally located on the same chromosome or on different chromosomes.

Optionally, normalized results from the cell are compared with normalized results from a reference cell. That is, the target and reference nucleic acids are also detected in a reference cell, for example, a non-tumor, uninfected, or other healthy normal cell, chosen as a standard of comparison depending on the desired application. As just one example, the first, target nucleic acid can be the Her-2 gene, with the goal of measuring Her-2 gene amplification. Signal from Her-2 can be normalized to that from a reference gene whose copy number is stably maintained in the genomic DNA. The normalized signal for the Her-2 gene from a target cell (e.g., a tumor cell or suspected tumor cell) can be compared to the normalized signal from a reference cell (e.g., a normal cell), to determine copy number in the cancer cell in comparison to normal cells.

Signal strength is optionally adjusted between the target and reference nucleic acids depending on their expected copy numbers, if desired. For example, the signal for a target mRNA expressed at low levels can be amplified to a greater degree (e.g., by use of more labels per label probe and/or use of capture probes, preamplifiers and amplifiers to capture more label probes per copy of the target) than the signal for a highly expressed mRNA (which can, e.g., be detected by direct binding of the label probe to the reference nucleic acid, by use of capture probes and amplifier without a preamplifier, or the like).

The methods for assaying relative levels of target nucleic acids in cells can be used to identify the cells. For example, a cell can be identified as being of a desired type based on which nucleic acids, and in what levels, it contains. Thus, in one class of embodiments, the methods include identifying the cell as a desired target cell based on the normalized first signal (and optional normalized third, fourth, etc. signals). As described herein, the cell can be identified on the basis of the presence or absence of one or more of the target nucleic acids. Similarly, the cell can be identified on the basis of the relative signal strength from or expression level of one or more target nucleic acids. Signals are optionally compared to those from a reference cell.

The methods can be applied to detection and identification of even rare cell types. Thus, the sample including the cell can be a mixture of desired target cells and other, nontarget cells, which can be present in excess of the target cells. For example, the ratio of target cells to cells of all other type(s) in the sample is optionally less than 1:1x10⁴, less than 1:1x10⁵, less than 1:1x10⁶, less than 1:1x10⁷, less than 1:1x10⁸, or even less than 1:1x10⁹.

Essentially any type of cell that can be differentiated based on its nucleic acid content (presence, absence, or copy number of one or more nucleic acids) can be detected and identified using the methods and a suitable choice of target and reference nucleic acids. As just a few examples, the cell can be a circulating tumor cell, a virally infected cell, a fetal cell in maternal blood, a bacterial cell or other microorganism in a biological sample (e.g., blood or other body fluid), or an endothelial cell, precursor endothelial cell, or myocardial cell in blood. Rare cell types can be enriched prior to performing the methods, if necessary, by methods known in the art (e.g., lysis of red blood cells, isolation of peripheral blood mononuclear cells, etc.). The methods are optionally combined with other techniques, such as DAPI staining for nuclear DNA. It will be evident that a variety of different types of nucleic acid markers are optionally detected simultaneously by the methods and used to identify the cell. For example, a cell can be identified based on the presence or relative expression level of one target nucleic acid in the cell and the absence of another target nucleic acid from the cell; e.g., a circulating tumor cell can be identified by the presence or level of one or more markers found in the tumor cell and not found (or found at different levels) in blood cells, and by the absence of one or more markers present in blood cells and not circulating tumor cells. The principle may be extended to using any other type of markers such as protein based markers in single cells.

Essentially all of the features noted for the methods above apply to these embodiments as well, as relevant; for example, with respect to source of sample, fixation and permeabilization of the cell, washing the cell, denaturation of double-stranded target and reference nucleic acids, type of labels, use of optional blocking probes, detection of signals, detection (and intensity measurement) by flow cytometry or microscopy, presence of the cell in suspension or immobilized on a substrate, and/or the like. Also, additional features described herein, e.g., in the section entitled "Implementation, applications, and advantages," can be applied to the methods, as relevant.

The methods of the invention can be used for gene expression analysis in single cells. Currently, gene expression analysis deals with heterogeneous cell populations such as blood or tumor specimens. Blood contains various subtypes of leukocytes, and when changes in gene expression of whole blood or RNA isolated from blood are measured, it is not known what subtype of blood cells actually changed their gene expression. It is possible that gene expression of only a certain subtype of blood cells is affected in a disease state or by drug treatment, for example. Technology that can measure gene expression in single cells, so changes of gene expression in single cells can be examined, is thus desirable. Similarly, a tumor specimen contains a heterogeneous cell population including tumor cells, normal cells, stromal cells, immune cells, etc. Current technology looks at the sum of the expression of all those cells through total RNA or cell lysate. However, the overall expression change may not be representative of that in target tumor cells. So again, it would be useful to look at the expression changes in single cells so that the target tumor cells can be examined specifically, to see how the target cells change in gene expression and how they respond to drug treatment, for example.

In one aspect, the present invention provides methods for gene expression analysis in single cells. Single cell gene expression analysis can be accomplished by measuring expression of a target gene and normalizing against the expression of a housekeeping gene, as described above. As just a couple of examples, the normalized expression in a disease state can be compared to that in the normal state, or the expression in a drug treated state can be compared to that in the normal state. The change of expression level in single cells may have biological significance indicating disease progression, drug therapeutic efficacy and/or toxicity, tumor staging and classification, etc.

Accordingly, one general class of embodiments provides methods of performing comparative gene expression analysis in single cells. In the methods, a first mixed cell population comprising one or more cells of a specified type is provided. A second mixed cell population comprising one or more cells of the specified type is also provided. An expression level of one or more target nucleic acids relative to a reference nucleic acid is measured in the cells of the specified type of the first population, to provide a first expression profile. An expression level of the one or more target nucleic acids relative to the reference nucleic acid is measured in the cells of the specified type of the second population, to provide a second expression profile. The first and second expression profiles are compared.

In one class of embodiments, the one or more target nucleic acids are one or more mRNAs, e.g., two or more, three or more, four or more, etc. mRNAs. The expression level of each mRNA can be determined relative to that of a housekeeping gene whose mRNA serves as the reference nucleic acid.

The first and/or second mixed cell population contains at least one other type of cell in addition to the specified type, more typically at least two or more other types of cells, and optionally several to many other types of cells (e.g., as is found in whole blood, a tumor, or other complex biological sample). The ratio of cells of the specified type to cells of all other type(s) in the first or second mixed cell population is optionally less than 1:1x10⁴, less than 1:1x10⁵, less than 1:1x10⁶, less than 1:1x10⁷, less than 1:1x10⁸, or even less than 1:1x10⁹.

As will be evident, a change in gene expression profile between the two populations may indicate a disease state or progression, a drug response, a therapeutic efficacy, etc. Thus, for example, the first mixed cell population can be from a patient who has been diagnosed or who is to be diagnosed with a particular disease or disorder, while the second mixed population is from a healthy individual. Similarly, the first and second mixed populations can be from a single individual but taken at different time points, for example, to follow disease progression or to assess response to drug treatment. Accordingly, the first mixed cell population can be taken from an individual (e.g., a human) before treatment is initiated with a drug or other compound, while the second population is taken at a specified time after treatment is initiated. As another example, the first mixed population can be from a treated individual while the second mixed population is from an untreated individual.

Essentially all of the features noted for the methods above apply to these embodiments as well, as relevant; for example, with respect to type of target and reference nucleic acids, cell type, source of sample, fixation and permeabilization of the cell, washing the cell, denaturation of double-stranded target and reference nucleic acids, type of labels, use and configuration of label probes, capture probes, preamplifiers and/or amplifiers, use of optional blocking probes, detection of signals, detection (and intensity measurement) by flow cytometry or microscopy, presence of the cell in suspension or immobilized on a substrate, and/or the like. Exemplary target and reference nucleic acids are described herein.

In another aspect, the methods can be used to compare copy number in single cells from a first population (e.g., tumor cells) with copy number in single cells from a second population (e.g., normal cells used as a reference). The nucleic acid target(s) can be transcripts or genomic DNA, where, for example, the degree of amplification or deletion of genes such as her-2 can correlate with tumor progression. In another aspect, the methods can be applied to gene expression analysis in single cells in even a single population, including, for example, cells of the same type but at different stages of the cell cycle.

### Label density

The methods of the invention permit far more labels to be captured to small regions of target nucleic acids than do currently existing techniques. For example, standard FISH techniques typically use probes that cover 20 kb or more, and a probe typically has fluorophores chemically conjugated at a density of approximately one fluorescent molecule per seven nucleotides of the probe. When molecular beacon target detection is employed, one label pair is captured to the target in the region covered by the beacon, typically about 40 nucleotides. For additional discussion of exemplary current techniques, see, e.g., U.S. patent application publications 2004/0091880 and 2005/0181463, USPN 6,645,731, and international patent application publications WO 95/09245 and 03/019141.

Methods described herein, in comparison, readily permit capture of hundreds of labels (e.g., 400 or more) to the region of the target covered by a single capture probe, e.g., 20-25 nucleotides or more. The theoretical degree of amplification achieved from a single capture probe is readily calculated for any given configuration of capture probes, amplifiers, etc; for example, the theoretical degree of amplification achieved from a single capture probe, and thus the number of labels per length in nucleotides of the capture probe, can be equal to the number of preamplifiers bound to the capture probe times the number of amplifiers that bind each preamplifier times the number of label probes that bind each preamplifier times the number of labels per label probe.

Thus, in one aspect, the invention provides methods that facilitate association of a high density of labels to target nucleic acids in cells. One general class of embodiments provides methods of detecting two or more nucleic acid targets in an individual cell. In the methods, a sample comprising the cell is provided. The cell comprises or is suspected of comprising a first nucleic acid target and a second nucleic acid target. In the cell, a first label is captured to the first nucleic acid target (when present in the cell) and a second label is captured to the second nucleic acid target (when present in the cell). A first signal from the first label is distinguishable from a second signal from the second label. As noted, the labels are captured at high density. Thus, an average of at least one copy of the first label per nucleotide of the first nucleic acid target is captured to the first nucleic acid target over a region that spans at least 20 contiguous nucleotides of the first nucleic acid target, and an average of at least one copy of the second label per nucleotide of the second nucleic acid target is captured to the second nucleic acid target over a region that spans at least 20 contiguous nucleotides of the second nucleic acid target. The first signal from the first label and the second signal from the second label are detected.

In one class of embodiments, an average of at least four, eight, or twelve copies of the first label per nucleotide of the first nucleic acid target are captured to the first nucleic acid target over a region that spans at least 20 contiguous nucleotides of the first nucleic acid target, and an average of at least four, eight, or twelve copies of the second label per nucleotide of the second nucleic acid target are captured to the second nucleic acid target over a region that spans at least 20 contiguous nucleotides of the second nucleic acid target. In one embodiment, an average of at least sixteen copies of the first label per nucleotide of the first nucleic acid target are captured to the first nucleic acid target over a region that spans at least 20 contiguous nucleotides of the first nucleic acid target, and an average of at least sixteen copies of the second label per nucleotide of the second nucleic acid target are captured to the second nucleic acid target over a region that spans at least 20 contiguous nucleotides of the second nucleic acid target.

Essentially all of the features noted for the methods above apply to these embodiments as well, as relevant, for example, with respect to type of labels, detection of signals, type, treatment, and suspension of the cell, and/or the like. The regions of the first and second nucleic acid targets optionally span at least 25,50,100,200, or more contiguous nucleotides and/or at most 2000, 1000, 500, 200, 100, 50, or fewer nucleotides. A like density of third, fourth, fifth, sixth, etc. labels is optionally present for (e.g., captured to) third, fourth, fifth, sixth, etc. nucleic acid targets.

### Detection of target cells

As described above, cells can be detected and identified by detecting their constituent nucleic acids. For certain applications, for example, detection of rare cells from large heterogeneous mixtures of cells, detection of multiple, redundant nucleic acid markers in order to detect the rare cell is advantageous. The following hypothetical example illustrates one advantage of detecting redundant markers.

Say that circulating tumor cells (CTC) are to be detected from a blood sample in which the CTC concentration is one in 10⁶ normal white blood cells. If a single nucleic acid marker for the CTC (e.g., a nucleic acid whose presence or copy number can uniquely and sufficiently distinguish the cell from the rest of the cell population) has a detection specificity of 1 in 10³, 1000 cells will be mistakenly identified as "CTC" when 10⁶ cells are counted. (Such false positives can result from random background signal generated by nonspecific binding of the relevant probe(s) or from similar factors.) If an additional independent marker is included which, on its own, also has a detection specificity of 1 in 10³, and if a cell is identified as a CTC only if both markers are positive, the combined detection specificity is now theoretically dramatically increased, to 1 in 10³x10³=10⁶. This specificity is sufficient for direct CTC detection in normal white blood cells under these assumptions. Similarly, if three independent redundant markers are used for identification of CTC, the detection specificity can be boosted to 1 in 10⁹. Use of two or more redundant markers thus reduces the number of false positives and facilitates detection of even rare cells from complex samples.

Accordingly, one general class of embodiments provides methods of detecting an individual cell of a specified type. In the methods, a sample comprising a mixture of cell types including at least one cell of the specified type is provided. A first label probe comprising a first label and a second label probe comprising a second label, wherein a first signal from the first label is distinguishable from a second signal from the second label, are provided. In the cell, the first label probe is captured to a first nucleic acid target (when the first nucleic acid target is present in the cell) and the second label probe is captured to a second nucleic acid target (when the second nucleic acid target is present in the cell). The first signal from the first label and the second signal from the second label are detected and correlated with the presence, absence, or amount of the corresponding, first and second nucleic acid targets in the cell. The cell is identified as being of the specified type based on detection of the presence, absence, or amount (e.g., a non-zero amount) of both the first and second nucleic acid targets within the cell, where the specified type of cell is distinguishable from the other cell type(s) in the mixture on the basis of either the presence, absence, or amount of the first nucleic acid target or the presence, absence, or amount of the second nucleic acid target in the cell (that is, the nucleic acid targets are redundant markers for the specified cell type). An intensity of the first signal and an intensity of the second signal are optionally measured and correlated with a quantity of the corresponding nucleic acid present in the cell.

Each nucleic acid target that serves as a marker for the specified cell type can distinguish the cell type by its presence in the cell, by its amount (copy number, e.g., its genomic copy number or its transcript expression level), or by its absence from the cell (a negative marker). A set of nucleic acid targets can include different types of such markers; that is, one nucleic acid target can serve as a positive marker, distinguishing the cell by its presence or non-zero amount in the cell, while another serves as a negative marker, distinguishing the cell by its absence from the cell. For example, in one class of embodiments, the cell comprises a first nucleic acid target and a second nucleic acid target, and the cell is identified as being of the specified type based on detection of the presence or amount of both the first and second nucleic acid targets within the cell, where the specified type of cell is distinguishable from the other cell type(s) in the mixture on the basis of either the presence or amount of the first nucleic acid target or the presence or amount of the second nucleic acid target in the cell.

The label probes can bind directly to the nucleic acid targets. For example, the first label probe can hybridize to the first nucleic acid target and/or the second label probe can hybridize to the second nucleic acid target. Alternatively, some or all of the label probes can be indirectly bound to their corresponding nucleic acid targets, e.g., through capture probes. For example, the first and second label probes can bind directly to the nucleic acid targets, or one can bind directly while the other binds indirectly, or both can bind indirectly.

The label probes are optionally captured to the nucleic acid targets via capture probes. In one class of embodiments, at least a first capture probe and at least a second capture probe are provided. In the cell, the first capture probe is hybridized to the first nucleic acid target and the second capture probe is hybridized to the second nucleic acid target. The first label probe is captured to the first capture probe and the second label probe is captured to the second capture probe, thereby capturing the first label probe to the first nucleic acid target and the second label probe to the second nucleic acid target. The features described for the methods above apply to these embodiments as well, with respect to configuration and number of the label and capture probes, optional use of preamplifiers and/or amplifiers, rolling circle amplification of circular polynucleotides, and the like.

Third, fourth, fifth, etc. nucleic acid targets are optionally detected in the cell. For example; the method optionally includes: providing a third label probe comprising a third label, wherein a third signal from the third label is distinguishable from the first and second signals, capturing in the cell the third label probe to a third nucleic acid target (when present in the cell), and detecting the third signal from the third label. The third, fourth, fifth, etc. label probes are optionally hybridized directly to their corresponding nucleic acid, or they can be captured indirectly via capture probes as described for the first and second label probes.

The additional markers can be used in any of a variety of ways. For example, the cell can comprise the third nucleic acid target, and the first and/or second signal can be normalized to the third signal. The methods can include identifying the cell as being of the specified type based on the normalized first and/or second signal, e.g., in embodiments in which the target cell type is distinguishable from the other cell type(s) in the mixture based on the copy number of the first and/or second nucleic acid targets, rather than purely on their presence in the target cell type and not in the other cell type(s). Examples include cells detectable based on a pattern of differential gene expression, CTC or other tumor cells detectable by overexpression of one or more specific mRNAs, and CTC or other tumor cells detectable by amplification or deletion of one or more specific chromosomal regions.

As another example, the third nucleic acid target can serve as a third redundant marker for the target cell type, e.g., to improve specificity of the assay for the desired cell type. Thus, in one class of embodiments, the methods include correlating the third signal detected from the cell with the presence, absence, or amount of the third nucleic acid target in the cell, and identifying the cell as being of the specified type based on detection of the presence, absence, or amount of the first, second, and third nucleic acid targets within the cell, wherein the specified type of cell is distinguishable from the other cell type(s) in the mixture on the basis of either presence, absence, or amount of the first nucleic acid target, presence, absence, or amount of the second nucleic acid target, or presence, absence, or amount of the third nucleic acid target in the cell.

As yet another example, the additional markers can assist in identifying the cell type. For example, the presence, absence, or amount of the first and third markers may suffice to identify the cell type, as could the presence, absence, or amount of the second and fourth markers; all four markers could be detected to provide two redundant sets of markers and therefore increased specificity of detection. As another example, one or more additional markers can be used in negative selection against undesired cell types; for example, identity of a cell as a CTC can be further verified by the absence from the cell of one or more markers present in blood cells and not circulating tumor cells.

Detection of additional nucleic acid targets can also provide further information useful in diagnosis, outcome prediction or the like, regardless of whether the targets serve as markers for the particular cell type. For example, additional nucleic acid targets can include markers for proliferating potential, apoptosis, or other metastatic, genetic, or epigenetic changes.

Signals from the additional targets are optionally normalized to a reference nucleic acid as described above. Signal strength is optionally adjusted between targets depending on their expected copy numbers, if desired. Signals from the target nucleic acids in the cell are optionally compared to those from a reference cell, as noted above.

A nucleic acid target can be essentially any nucleic acid that is desirably detected in the cell. For example, a nucleic acid target can be a DNA, a chromosomal DNA, an RNA, an mRNA, a microRNA, a ribosomal RNA, or the like. The nucleic acid target can be a nucleic acid endogenous to the cell. As another example, the target can be a nucleic acid introduced to or expressed in the cell by infection of the cell with a pathogen, for example, a viral or bacterial genomic RNA or DNA, a plasmid, a viral or bacterial mRNA, or the like.

The first and second (and/or optional third, fourth, etc.) nucleic acid targets can be part of a single nucleic acid molecule, or they can be separate molecules. Various advantages and applications of both approaches are discussed in greater detail below, e.g., in the section entitled "Implementation, applications, and advantages." In one class of embodiments, the first nucleic acid target is a first mRNA and the second nucleic acid target is a second mRNA. In another class of embodiments, the first nucleic acid target comprises a first region of an mRNA and the second nucleic acid target comprises a second region of the same mRNA. In another class of embodiments, the first nucleic acid target comprises a first chromosomal DNA polynucleotide sequence and the second nucleic acid target comprises a second chromosomal DNA polynucleotide sequence. The first and second chromosomal DNA polynucleotide sequences are optionally located on the same chromosome, e.g., within the same gene, or on different chromosomes.

The methods can be applied to detection and identification of even rare cell types. For example, the ratio of cells of the specified type to cells of all other type(s) in the mixture is optionally less than 1:1x10⁴, less than 1:1x10⁵, less than 1:1x10⁶, less than 1:1x10⁷, less than 1:1x10⁸, or even less than 1:1x10⁹.

Essentially any type of cell that can be differentiated based on suitable markers (or redundant regions of a single marker, e.g., a single mRNA or amplified/deleted chromosomal region) can be detected and identified using the methods. As just a few examples, the cell can be a circulating tumor cell, a virally infected cell, a fetal cell in maternal blood, a bacterial cell or other microorganism in a biological sample (e.g., blood or other body fluid), an endothelial cell, precursor endothelial cell, or myocardial cell in blood, stem cell, or T-cell. Rare cell types can be enriched prior to performing the methods, if necessary, by methods known in the art (e.g., lysis of red blood cells, isolation of peripheral blood mononuclear cells, etc.).

Essentially all of the features noted for the methods above apply to these embodiments as well, as relevant; for example, with respect to source of sample, fixation and permeabilization of the cell, washing the cell, denaturation of double-stranded nucleic acids, type of labels, use of optional blocking probes, detection of signals, detection (and intensity measurement) of signals from the individual cell by flow cytometry or microscopy, presence of the cell in suspension or immobilized on a substrate, and/or the like. Also, additional features described herein, e.g., in the section entitled "Implementation, applications, and advantages," can be applied to the methods, as relevant.

In another aspect, detection of individual cells of a specified type is performed as described above, but the first and second nucleic acid targets need not be redundant markers for that cell type. The nucleic acid targets can be essentially any desired nucleic acids, including, for example, redundant and/or non-redundant markers for the cell type.

### COMPOSITIONS AND KITS

The invention also provides compositions useful in practicing or produced by the methods. One exemplary class of embodiments provides a composition that includes a fixed and permeabilized cell, which cell comprises or is suspected of comprising a first nucleic acid target and a second nucleic acid target, at least a first capture probe capable of hybridizing to the first nucleic acid target, at least a second capture probe capable of hybridizing to the second nucleic acid target, a first label probe comprising a first label, and a second label probe comprising a second label. A first signal from the first label is distinguishable from a second signal from the second label. The cell optionally comprises the first and second capture probes and label probes. The first and second capture probes are optionally hybridized to their respective nucleic acid targets in the cell.

The features described for the methods above for indirect capture of the label probes to the nucleic acid targets apply to these embodiments as well. For example, the label probes can hybridize to the capture probes. In one class of embodiments, the composition includes a single first capture probe and a single second capture probe, where the first label probe is capable of hybridizing to the first capture probe and the second label probe is capable of hybridizing to the second capture probe. In another class of embodiments, the composition includes two or more first capture probes, two or more second capture probes, a plurality of the first label probes, and a plurality of the second label probes. A single first label probe is capable of hybridizing to each of the first capture probes, and a single second label probe is capable of hybridizing to each of the second capture probes.

In another aspect, amplifiers can be employed to increase the number of label probes captured to each target. For example, in one class of embodiments, the composition includes a single first capture probe, a single second capture probe, a plurality of the first label probes, a plurality of the second label probes, a first amplifier, and a second amplifier. The first amplifier is capable of hybridizing to the first capture probe and to the plurality of first label probes, and the second amplifier is capable of hybridizing to the second capture probe and to the plurality of second label probes. In another class of embodiments, the composition includes two or more first capture probes, two or more second capture probes, a multiplicity of the first label probes, a multiplicity of the second label probes, a first amplifier, and a second amplifier. The first amplifier is capable of hybridizing to one of the first capture probes and to a plurality of first label probes, and the second amplifier is capable of hybridizing to one of the second capture probes and to a plurality of second label probes.

In another aspect, preamplifiers and amplifiers are employed to capture the label probes to the targets. In one class of embodiments, the composition includes a single first capture probe, a single second capture probe, a multiplicity of the first label probes, a multiplicity of the second label probes, a plurality of first amplifiers, a plurality of second amplifiers, a first preamplifier, and a second preamplifier. The first preamplifier is capable of hybridizing to the first capture probe and to the plurality of first amplifiers, and the second preamplifier is capable of hybridizing to the second capture probe and to the plurality of second amplifiers. The first amplifier is capable of hybridizing to the first preamplifier and to a plurality of first label probes, and the second amplifier is capable of hybridizing to the second preamplifier and to a plurality of second label probes. In a related class of embodiments, the composition includes two or more first capture probes, two or more second capture probes, a multiplicity of the first label probes, a multiplicity of the second label probes, a multiplicity of first amplifiers, a multiplicity of second amplifiers, a plurality of first preamplifiers, and a plurality of second preamplifiers. The first preamplifier is capable of hybridizing to one of the first capture probes and to a plurality of first amplifiers, the second preamplifier is capable of hybridizing to one of the second capture probes and to a plurality of second amplifiers, the first amplifier is capable of hybridizing to the first preamplifier and to a plurality of first label probes, and the second amplifier is capable of hybridizing to the second preamplifier and to a plurality of second label probes. Optionally, additional preamplifiers can be used as intermediates between a preamplifier hybridized to the capture probe(s) and the amplifiers.

In the above classes of embodiments, one capture probe hybridizes to each label probe, amplifier, or preamplifier. In alternative classes of related embodiments, two or more capture probes hybridize to the label probe, amplifier, or preamplifier.

In one class of embodiments, the composition comprises a plurality of the first label probes, a plurality of the second label probes, a first amplified polynucleotide produced by rolling circle amplification of a first circular polynucleotide hybridized to the first capture probe, and a second amplified polynucleotide produced by rolling circle amplification of a second circular polynucleotide hybridized to the second capture probe. The first circular polynucleotide comprises at least one copy of a polynucleotide sequence identical to a polynucleotide sequence in the first label probe, and the first amplified polynucleotide comprises a plurality of copies of a polynucleotide sequence complementary to the polynucleotide sequence in the first label probe (and can thus hybridize to a plurality of the label probes). The second circular polynucleotide comprises at least one copy of a polynucleotide sequence identical to a polynucleotide sequence in the second label probe, and the second amplified polynucleotide comprises a plurality of copies of a polynucleotide sequence complementary to the polynucleotide sequence in the second label probe. The composition can also include reagents necessary for producing the amplified polynucleotides, for example, an exogenously supplied nucleic acid polymerase, an exogenously supplied nucleic acid ligase, and/or exogenously supplied nucleoside triphosphates (e.g., dNTPs).

The cell optionally includes additional nucleic acid targets, and the composition (and cell) can include reagents for detecting these targets. For example, the cell can comprise or be suspected of comprising a third nucleic acid target, and the composition can include at least a third capture probe capable of hybridizing to the third nucleic acid target and a third label probe comprising a third label. A third signal from the third label is distinguishable from the first and second signals. The cell optionally includes fourth, fifth, sixth, etc. nucleic acid targets, and the composition optionally includes fourth, fifth, sixth, etc. label probes and capture probes.

Essentially all of the features noted for the methods above apply to these embodiments as well, as relevant; for example, with respect to type of nucleic acid target, location of various targets on a single molecule or on different molecules, type of labels, inclusion of optional blocking probes, and/or the like. For example, it is worth noting that the second nucleic acid target optionally comprises a reference nucleic acid. In other embodiments, the first and second nucleic acid targets serve as markers for a specified cell type, e.g., redundant markers.

The cell can be essentially any type of cell from any source, particularly a cell that can be differentiated based on its nucleic acid content (presence, absence, or copy number of one or more nucleic acids). As just a few examples, the cell can be a circulating tumor cell, a virally infected cell, a fetal cell in maternal blood, a bacterial cell or other microorganism in a biological sample (e.g., blood or other body fluid), or an endothelial cell, precursor endothelial cell, or myocardial cell in blood. For example, the cell can be derived from a bodily fluid, blood, bone marrow, sputum, urine, lymph node, stool, cervical pap smear, oral swab or other swab or smear, spinal fluid, saliva, sputum, semen, lymph fluid, an intercellular fluid, a tissue (e.g., a tissue homogenate), a biopsy, and/or a tumor. The cell can be derived from one or more of a human, an animal, a plant, and a cultured cell.

The cell can be present in a mixture of cells, for example, a complex heterogeneous mixture. In one class of embodiments, the cell is of a specified type, and the composition comprises one or more other types of cells. These other cells can be present in excess, even large excess, of the cell. For example, the ratio of cells of the specified type to cells of all other type(s) in the composition is optionally less than 1:1x10⁴, less than 1:1x10⁵, less than 1:1x10⁶, less than 1:1x10⁷, less than 1:1x10⁸, or even less than 1:1x10⁹.

The cell is optionally immobilized on a substrate, present in a tissue section, or the like. Preferably, however, the cell is in suspension in the composition. The composition can be contained in a flow cytometer or similar instrument. Additional features described herein, e.g., in the section entitled "Implementation, applications, and advantages," can be applied to the compositions, as relevant.

Another aspect of the invention provides compositions in which a large number of labels are correlated with each target nucleic acid. One general class of embodiments thus provides a composition comprising a cell, which cell includes a first nucleic acid target, a second nucleic acid target, a first label whose presence in the cell is indicative of the presence of the first nucleic acid target in the cell, and a second label whose presence in the cell is indicative of the presence of the second nucleic acid target in the cell, wherein a first signal from the first label is distinguishable from a second signal from the second label. An average of at least one copy of the first label is present in the cell per nucleotide of the first nucleic acid target over a region that spans at least 20 contiguous nucleotides of the first nucleic acid target, and an average of at least one copy of the second label is present in the cell per nucleotide of the second nucleic acid target over a region that spans at least 20 contiguous nucleotides of the second nucleic acid target.

In one class of embodiments, the copies of the first label are physically associated with the first nucleic acid target, and the copies of the second label are physically associated with the second nucleic acid target. For example, the first label can be part of a first label probe and the second label part of a second label probe, where the label probes are captured to the target nucleic acids.

In one class of embodiments, an average of at least four, eight, or twelve copies of the first label are present in the cell per nucleotide of the first nucleic acid target over a region that spans at least 20 contiguous nucleotides of the first nucleic acid target, and an average of at least four, eight, or twelve copies of the second label are present in the cell per nucleotide of the second nucleic acid target over a region that spans at least 20 contiguous nucleotides of the second nucleic acid target. In one embodiment, an average of at least sixteen copies of the first label are present in the cell per nucleotide of the first nucleic acid target over a region that spans at least 20 contiguous nucleotides of the first nucleic acid target, and an average of at least sixteen copies of the second label are present in the cell per nucleotide of the second nucleic acid target over a region that spans at least 20 contiguous nucleotides of the second nucleic acid target.

Essentially all of the features noted for the embodiments above apply to these embodiments as well, as relevant, for example, with respect to type of labels, suspension of the cell, and/or the like. The regions of the first and second nucleic acid targets are typically regions covered by a probe, primer, or similar polynucleotide employed to detect the respective target. The regions of the first and second nucleic acid targets optionally span at least 25, 50, 100, 200, or more contiguous nucleotides and/or at most 2000, 1000, 500, 200, 100, 50, or fewer nucleotides. A like density of labels is optionally captured to third, fourth, fifth, sixth, etc. nucleic acid targets. The composition optionally includes PCR primers, a thermostable polymerase, and/or the like, in embodiments in which the targets are detected by multiplex in situ PCR.

Another aspect of the invention provides kits useful for practicing the methods. One general class of embodiments provides a kit for detecting a first nucleic acid target and a second nucleic acid target in an individual cell. The kit includes at least one reagent for fixing and/or permeabilizing the cell, at least a first capture probe capable of hybridizing to the first nucleic acid target, at least a second capture probe capable of hybridizing to the second nucleic acid target, a first label probe comprising a first label, and a second label probe comprising a second label, wherein a first signal from the first label is distinguishable from a second signal from the second label, packaged in one or more containers.

Essentially all of the features noted for the embodiments above apply to these embodiments as well, as relevant; for example, with respect to number of nucleic acid targets, configuration and number of the label and capture probes, inclusion of preamplifiers and/or amplifiers, inclusion of blocking probes, inclusion of amplification reagents, type of nucleic acid target, location of various targets on a single molecule or on different molecules, type of labels, inclusion of optional blocking probes, and/or the like. The kit optionally also includes instructions for detecting the nucleic acid targets in the cell and/or identifying the cell as being of a specified type, one or more buffered solutions (e.g., diluent, hybridization buffer, and/or wash buffer), reference cell(s) comprising one or more of the nucleic acid targets, and/or the like.

Another general class of embodiments provides a kit for detecting an individual cell of a specified type from a mixture of cell types by detecting a first nucleic acid target and a second nucleic acid target. The kit includes at least one reagent for fixing and/or permeabilizing the cell, a first label probe comprising a first label (for detection of the first nucleic acid target), and a second label probe comprising a second label (for detection of the second nucleic acid target), wherein a first signal from the first label is distinguishable from a second signal from the second label, packaged in one or more containers. The specified type of cell is distinguishable from the other cell type(s) in the mixture by presence, absence, or amount of the first nucleic acid target in the cell or by presence, absence, or amount of the second nucleic acid target in the cell (that is, the two targets are redundant markers for the specified cell type).

Essentially all of the features noted for the embodiments above apply to these embodiments as well, as relevant; for example, with respect to number of nucleic acid targets, inclusion of capture probes, configuration and number of the label and/or capture probes, inclusion of preamplifiers and/or amplifiers, inclusion of blocking probes, inclusion of amplification reagents, type of nucleic acid target, location of various targets on a single molecule or on different molecules, type of labels, inclusion of optional blocking probes, and/or the like. The kit optionally also includes instructions for identifying the cell as being of the specified type, one or more buffered solutions (e.g., diluent, hybridization buffer, and/or wash buffer), reference cell(s) comprising one or more of the nucleic acid targets, and/or the like.

### IMPLEMENTATION, APPLICATIONS, AND ADVANTAGES

Various aspects of the invention are described in additional detail below. Exemplary embodiments and applications are also described.

The new technology (methods, compositions, systems, and kits), QMAGEX (Quantitative Multiplex Analysis of Gene Expression in Single Cell), disclosed herein is capable of detection and quantification of multiple nucleic acids within individual cells. The technology is significantly different from existing ISH technology in several aspects, although they both can measure mRNA expression in individual cells. First, cells preferably remain in suspension status during all or at least most of the assay steps in the assays of the present invention, which greatly improves assay hybridization kinetics, resulting in better reproducibility and shorter assay time. Second, the instant technology has the capability for analyzing the expression of multiple mRNA transcripts within cells simultaneously and quantitatively. This is highly desirable, since, for example, detection of multiple tumor marker genes could greatly improve the accuracy of CTC identification (Mocellin et al., 2004) and greatly reduce the false positive rate. Quantitative analysis of gene expression level could not only further aid in discriminating the CTC from other types of cells but also could help in distinguishing the type and source of primary tumors as well as the stages of tumor progression. Third, the instant technology enables the use of a flow cytometer as the base for detection, which, compared with microscope-based detection instruments, offers higher throughput. In addition, the flow cytometer is capable of sorting out cells, e.g., tumor cells, for further study. Subsequent to the detection and quantification of mRNA expression, isolation of the CTC or other cells may be advantageous for further identity confirmation or for additional cytological and molecular analysis. Fourth, the instant technology has vastly improved detection sensitivity and reproducibility, and is capable of single copy gene detection and quantification. In addition, the instant technology uses a standard, generic set of probe labeling and detection technology (e.g., the same set of preamplifiers, amplifiers, and label probes can be used to detect multiple different sets of nucleic acid targets, requiring only synthesis of a new set of capture probes for each new set of nucleic acid targets), and optionally uses standardized procedures for cell fixation and permeation and for hybridization and washing. Furthermore, the technology can include built-in internal controls for assay specificity and efficiency.

The instant technology can be used not only for the detection and enumeration of rare CTC in blood samples or other body fluids, but also for any type of rare cell identification and enumeration events. Applications include, but are not limited to: detection of minimal residual disease in leukemia and lymphoma; recurrence monitoring after chemotherapy treatment (Hess et al.); detection of other pre-cancerous cells, such as the detection of HPV-containing cervical cells in body fluids; detection of viral or bacterial nucleic acid in an infected cell; detection of fetal cells in maternal blood; detection of micro-tumor lesions during early stage of tumor growth; or detection of residual tumor cells after surgery for margin management. In all of these cases, target cell specific gene expression is likely to be buried in the background of large numbers of heterogeneous cell populations. As a result, microarray or RT-PCR based expression analysis, which require the isolation of mRNA from a large population of cells, will have difficulty detecting the presence of those rare cell events accurately or reliably, whereas the invented technology can readily be applied.

It should also be noted that although single cell detection and quantification of multiple mRNA transcripts is illustrated here as the main application, such technology is equally applicable to detection of other rare cell events that include changes in chromosomal DNA or cellular nucleic acid content. Examples include, but are not limited to, detection of her-2/neu gene amplification, detection of Rb gene deletion, detection of somatic mutations, detection of chromosome translocation such as in chronic myelogenous leukemia (BCR-ABL), or detection of HPV insertion to chromosomal DNA of cervical cancer cells.

Finally, the probe design, multiplexing and amplification aspects of the instant technology can be applied in quantitative, multiplex gene expression analysis and in measuring chromosomal DNA changes at a single cell level in solid tissue sections, such as formalin-fixed, paraffin embedded (FFPE) tissue samples.

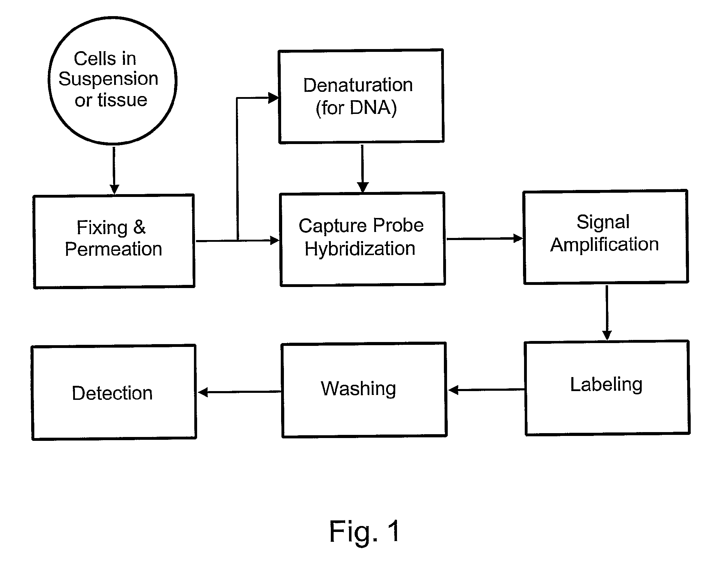

The QMAGEX technology comprises an assay and optional associated apparatus to implement the assay in an automated fashion. **Figure 1** illustrates major elements of the QMAGEX assay work flow, which, for one exemplary embodiment in which amplifiers are employed, include:
Fixation and Permeation: Cells in the sample are fixed and permeated (permeabilized) in suspension. The fixation step immobilizes nucleic acids (e.g., mRNA or chromosomal DNA) and cross-links them to the cellular structure. Then the cell membrane is permeabilized so that target-specific nucleic acid probes and signal-generating particles, such as fluorescently labeled nucleic acid probes, can enter the cell and bind to the target.
Denaturation: If the detection target is double-stranded chromosomal DNA, a denaturation step is added to convert the double-stranded target into single-stranded DNA, ready to be bound with the target-specific probes.
Capture Probe Hybridization: Carefully selected target-specific capture probes or probe sets are hybridized to the target nucleic acids. The capture probes serve to link the target molecules specifically to signal-generating particles. The technology enables multiple target genes in the cell to be recognized by different probe sets simultaneously and with a high degree of specificity.
Signal Amplification: Signals from target molecules are amplified by binding a large scaffold molecule, an amplifier, to the capture probes or probe sets. Each scaffold has multiple locations to accept label probes and signal-generating particles. In a multiplex assay, multiple distinct amplifiers are used.
Labeling: Label probes, to which signal generating particles (labels) are attached, hybridize to the amplifier in this step. In a multiplex assay, multiple distinct label probes are used.
Washing: The excess probes or signal generating particles that are not bound or that are nonspecifically bound to the cells are removed through a washing step, which reduces background noise and improves the detection signal to noise ratio. Additional washing steps may be added during the capture probe hybridization or signal amplification steps to further enhance the assay performance.
Detection: The labeled suspension cells are detected using Fluorescent Activated Cell Sorting (FACS) or a flow cytometer, or are immobilized on a solid surface and detected using a microscope or scanner based instrument.

In the following section, major elements of the QMAGEX technology will be described in detail. In the following, the term label probe refers to an entity that binds to the target molecule, directly or indirectly, and enables the target to be detected by a readout instrument. The label probe, in general, comprises a nucleic acid or modified nucleic acid molecule that binds to the target, directly or indirectly, and one or more "signal generating particle" (i.e., label) that produces the signal recognizable by the readout instrument. In indirect mode, the label probe can either be attached to the target molecule through binding to a capture probe directly or through binding to an amplifier that is in turn linked to a capture probe. Exemplary signal-generating particles (labels) include, but are not limited to, fluorescent molecules, nano-particles, radioactive isotopes, chemiluminescent molecules (e.g., digoxigenin, dinitrophenyl). Fluorescent molecules include, but are not limited to, fluorescein (FITC), cy3, cy5, alexa dyes, phycoerythrin, etc. Nano-particles include, but are not limited to, fluorescent quantum dots, scattering particles, etc. The term capture probe refers to a nucleic acid or a modified nucleic acid that links the target to a specific type of label probe, directly or indirectly. The term "capture probe set" refers to multiple nucleic acids or modified nucleic acids that link a target to a specific type of label probe, directly or indirectly, for increased assay sensitivity. The term amplifier refers to a large scaffold molecule(s) that binds to one or more capture probes or to a preamplifier on one side and to multiple label probes on another side.

### Fixation

In this step, the nucleic acids are immobilized within cells by cross-linking them within the cellular structure. There are a variety of well known methods to fix cells in suspension with a fixative reagent and to block the endogenous RNase activities, which can be adapted for use in the present invention. Fixative reagents include formalin (formaldehyde), paraformaldehyde, gluteraldehyde, ethanol, methanol, etc. One common fixative solution for tissue sections includes 0.25% gluteraldehyde and 4% paraformaldehyde in phosphate buffer. Another common fixative solution for tissue sections includes 50% ethanol, 10% formalin (containing 37% formaldehyde), and 5% acetic acid. Different combinations of the fixative reagents at various concentrations are optionally tested to find the optimal composition for fixing cells in suspension, using techniques well known in the art. Duration of the fixing treatment can also be optimized. A number of different RNase inhibitors can be included in the fixative solution, such as RNAlater (Ambion), citric acid or LiCl, etc.

### Permeation

Fixation results in cross-linking of the target nucleic acids with proteins or other cellular components within cells, which may hinder or prevent infiltration of the capture probes into the cells and mask the target molecules for hybridization. The assays of the invention thus typically include a follow-on permeation step to enable in-cell hybridization. One technique involves the application of heat for varying lengths of time to break the cross-linking. This has been demonstrated to increase the accessibility of the mRNA in the cells for hybridization. Detergents (e.g., Triton X-100 or SDS) and Proteinase K can also be used to increase the permeability of the fixed cells. Detergent treatment, usually with Triton X-100 or SDS, is frequently used to permeate the membranes by extracting the lipids. Proteinase K is a nonspecific protease that is active over a wide pH range and is not easily inactivated. It is used to digest proteins that surround the target mRNA. Again, optimal concentrations and duration of treatment can be experimentally determined as is well known in the art. A cell washing step can follow, to remove the dissolved materials produced in the permeation step.

Optionally, prior to fixation and permeation, cells in suspension are collected and treated to inactivate RNase and/or to reduce autofluorescence. DEPC treatment (e.g. Braissant and Wahli (1988) "A simplified in situ hybridization protocol using non-radioactively labeled probes to detect abundant and rare mRNAs on tissue sections" Biochemica 1:10-16) and RNAlater (Ambion, Inc.) have been demonstrated to be effective in stabilizing and protecting cellular RNA. Sodium borohydride and high heat have also been shown to preserve the integrity of RNA and to reduce autofluorescence, facilitating the detection of genes expressed at a low level (Capodieci et al. (2005) "Gene expression profiling in single cells within tissue" Nat Methods 2(9):663-5). Other methods of reducing cellular autofluorescence such as trypan blue (Mosiman et al. (1997) "Reducing cellular autofluorescence in flow cytometry: an in situ method" Cytometry 30(3):151-6) or singly labeled quencher oligonucleotide probe (Nolan et al. (2003) "A simple quenching method for fluorescence background reduction and its application to the direct, quantitative detection of specific mRNA" Anal Chem. 2003 75(22):6236-43) are optionally employed.

### Capture Probe Hybridization

In this assay step, the capture probe or capture probe set binds to the intended target molecule by hybridization. One indicator for a successful target hybridization is specificity, i.e. the capture probes or probe sets should substantially only link the label probes to the specific target molecule of interest, not to any other molecules. Probe selection and design are important in achieving specific hybridization.

### Probe Selection and Design

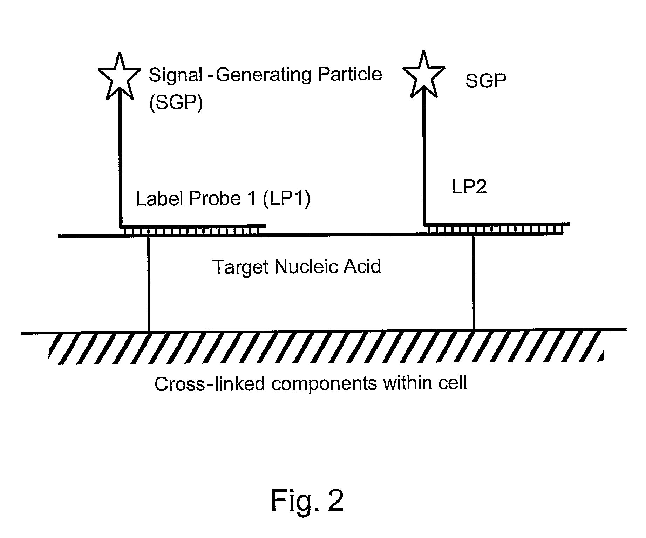

The assays of the invention employ two types of approaches in probe design to link the target nucleic acids in cells to signal generating particles: "direct labeling" and "indirect labeling". In the direct labeling approach, the target molecule hybridizes to or captures one or more label probes (LP) directly. The LPs contain the signal-generating particles (SGP), as shown in **Figure 2****.** A different LP needs to be used to attach additional SGP at different positions on the target molecule. In order to ensure hybridization specificity, the label probe is preferably stringently selected to ensure that it does not cross-hybridize with nonspecific nucleic acid sequences.

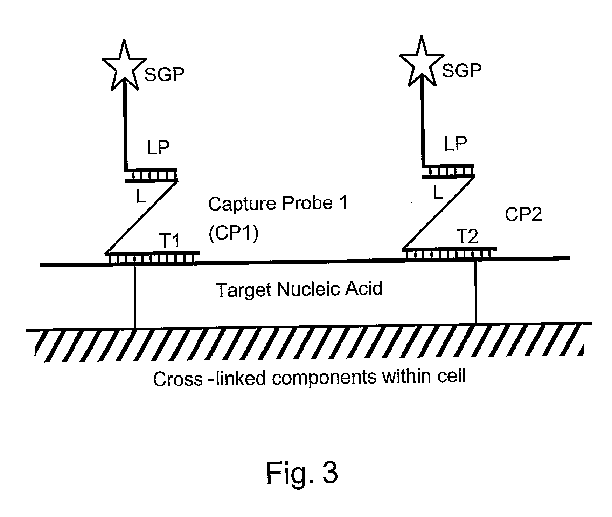

In the indirect labeling approach, an additional capture probe (CP) is employed. An example is shown in **Figure 3****.** The target molecule captures the label probe through the capture probe. In each capture probe, there is at least one section, T, complementary to a section on the target molecule, and another section, L, complementary to a section on the label probe. The T and L sections are connected by a section C. To attach more SGPs to different positions on the same target molecule, different capture probes are needed, but the label probe can remain the same. The sequence of L is carefully selected to ensure that it does not cross-hybridize substantially with any sequences in the nucleic acids in cells. In a further embodiment, the L portion of the capture probe and the label probe contain chemically modified or nonnatural nucleotides that do not hybridize with natural nucleotides in cells. In another embodiment, L and the label probe (or a portion thereof) are not even nucleic acid sequences. For example, L can be a weak affinity binding antibody that recognizes the signal-generating probe, which in this case is or includes an antigen; L can be covalently conjugated to an oligonucleotide that comprises the T section of the capture probe. Optionally, for two adjacent capture probes, the T sections hybridize to the target and two of the low affinity binding antibody binds to the antigen on the label probe at the same time, which results in strong affinity binding of the antigen. The capture and label probes are specific for a target gene of interest. Multiple capture probes (probe set) can be bound to the same target gene of interest in order to attach more signal-generating particles for higher detection sensitivity. In this situation, the probe set for the same target gene can share the same label probe.

Although both approaches can be used in the instant technology, the indirect capture approach is preferred because it enables the label probe to be target independent and further disclosure will show that it can offer better specificity and sensitivity.

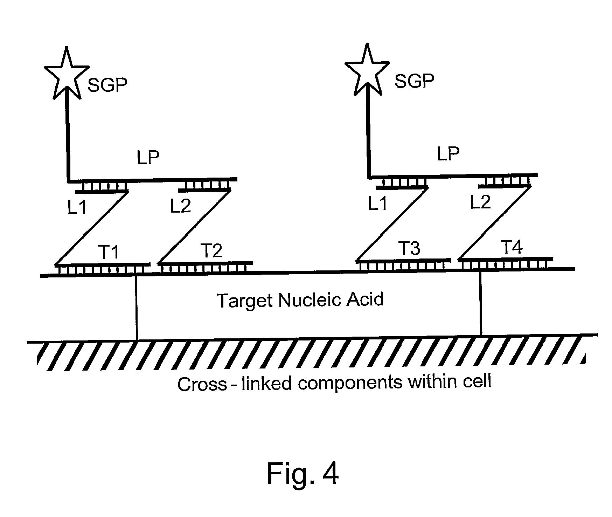

In a further indirect capture embodiment shown in **Figure 4****,** two adjacent capture probes are incorporated in a probe set targeting a gene of interest. T₁ and T₂ are designed to be complementary to two unique and adjacent sections on the target nucleic acid. L₁ and L₂, which can be different or the same, are complementary to two adjacent sections on the label probe. Their binding sections, T, L or both, are designed so that the linkage between the label probe and the target is unstable and tends to fall off at hybridization temperature when only one of the capture probes is in place. Such a design should enable exceptional specificity because the signal-generating label probe can only be attached to the target gene of interest when two independent capture probes both recognize the target and bind to the adjacent sequences or in very close proximity of the target gene. In a further embodiment, the melting temperature, Tₘ, of the T sections of the two capture probes are designed to be significantly above the hybridization temperature while the Tₘ of the L sections is below the hybridization temperature. As a result, T sections bind to the target molecule strongly and stably during hybridization, while L sections bind to the label probe weakly and unstably if only one of the capture probes is present. However, if both capture probes are present, the combination of L₁ and L₂ holds the label probe strongly and stably during hybridization. In another embodiment, Tₘ of the T sections is below hybridization temperature while Tₘ of the L sections is substantially above. In the same way, the linkage between the label probe and the target can only survive the hybridization when both capture probes are hybridized to the target in a cooperative fashion.

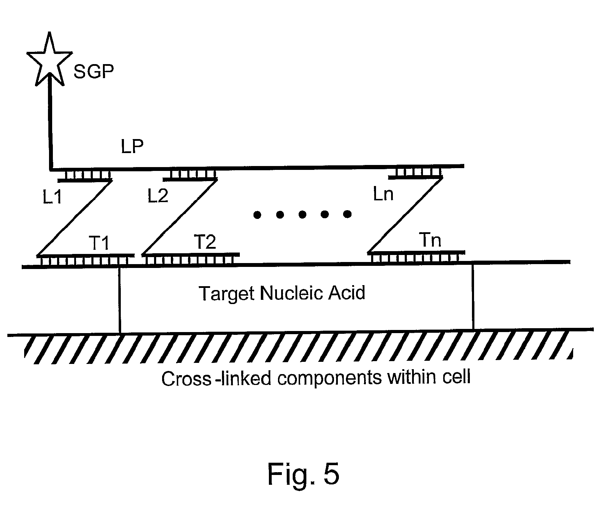

In another embodiment, three or more of the target nucleic acid specific, neighboring capture probes are used for the stable capture of one label probe within cells **(****Figure 5****).** The basic design of the probes is the same as discussed above, but the capture of one signal-generating probe should have even higher specificity than when two neighboring probes are used since now three independent probes have to bind to the same target molecule of interest in neighboring positions in order to generate signal.

### Multiplexing

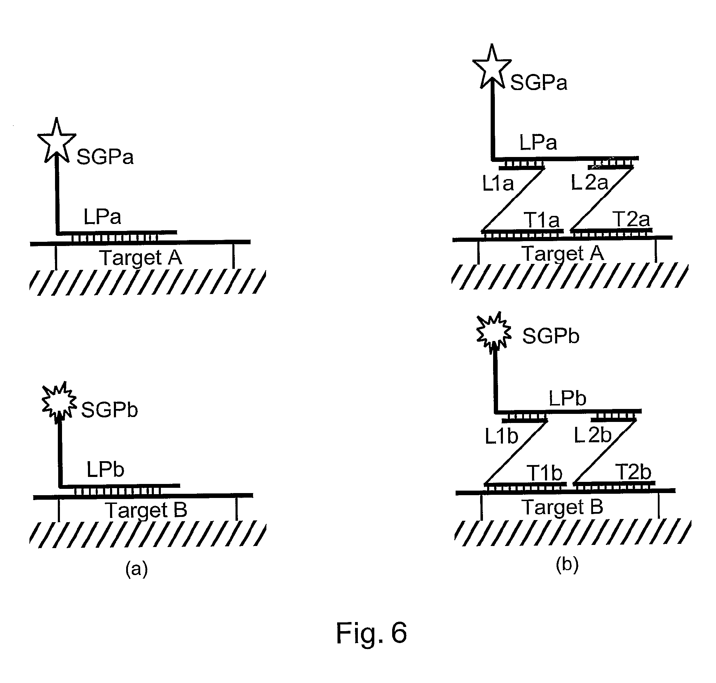

To perform multiplexed detection for more than one target gene, e.g., as shown in **Figure 6****,** each target gene has to be specifically bound by different capture and label probes. In addition, the signal generating particle (the label) attached to the label probe should provide distinctively different signals for each target that can be read by the detection instrument. In the direct labeling approach (e.g., **Figure 6** **Panel A**), suitable label probes with minimal cross-hybridization can be harder to find because each label probe has to be able to bind to the target strongly but not cross-hybridize to any other nucleic acid molecules in the system. For this approach to provide optimal results, the target binding portion of the label probe should be judiciously designed so that it does not substantially cross-hybridize with nonspecific sequences. In the indirect labeling approach (e.g., **Figure 6** **Panel B),** because of the unique multiple capture probe design approach, even when one capture probe binds to a nonspecific target, it will not result in the binding of the label probe to the nonspecific target. The assay specificity can be greatly improved. Thus the capture probe design illustrated in **Figure 4** and **Figure 5** is typically preferred in some multiplex assay applications. In one class of embodiments, the signal-generating particles attached to different target genes are different fluorescent molecules with distinctive emission spectra.

The capacity of the instant technology to measure more than one parameter simultaneously can enable detection of rare cells in a large heterogeneous cell population. As noted above, the concentration of CTC is estimated to be in the range of one tumor cell among every 10⁶-10⁷ normal blood cells. In existing FACS based immunoassays, on the other hand, random dye aggregation in cells may produce one false positive cell count in every ten thousand cells. Such an assay can thus not be used for CTC detection due to the unacceptably high false positive rates. This problem can be solved elegantly using the instant technology. In one particular embodiment, expression of more than one tumor genes are used as the targets for multiplex detection. Only cells that express all the target genes are counted as tumor cells. In this way, the false positive rate of the CTC detection can be dramatically reduced. For example, since dye aggregation in cells is a random event, if the false positive rate of a single color detection is 10⁻⁴, the false positive rate for two color or three color detection can be as low as 10⁻⁸ or 10⁻¹², respectively. In situations where the relative levels of expression of the target genes are known, these relative levels can be measured using the multiplex detection methods disclosed herein and the information can be used to further reduce the false positive rate of the detection.

In another embodiment, schematically illustrated in **Figure 7** **Panel A,** more than one signal-generating particles are linked to the same target nucleic acid. These particles generate distinct signals in the detection instrument. The relative strengths of these signals can be pre-determined by designing the number of each type of particles attached to the target. The number of signal-generating particles on a target can be controlled in probe design by changing the number of probe sets or employing different signal amplification methods, e.g., as described in the following section. The rare cells are identified only when the relative signal strengths of these particles measured by the detection instrument equal the pre-determined values. This embodiment is useful when there are not enough suitable markers or when their expression levels are unknown in a particular type of rare cells. In yet another embodiment, shown in **Figure 7** **Panel B,** the same set of signal-generating particles are attached to more than one target. The relative signal strengths of the particle set are controlled to be the same on all selected targets. This embodiment is useful in situations in which the rare cell is identified when any of the target molecules are present. In yet another embodiment, depicted in **Figure 7** **Panel C,** each target molecule has a set of signal generating particles attached to it, but the particle sets are distinctively different from target to target.

The detection of multiple target nucleic acid species of interest can be applied to quantitative measurement of one target. Due to different sample and experimental conditions, the abundance of a particular target molecule in a cell normally cannot be determined precisely through the detection of the signal level associated with the target. However, more precise measurement can be accomplished by normalizing the signal of a gene of interest to that of a reference/housekeeping gene. A reference/housekeeping gene is defined as a gene that is generally always present or expressed in cells. The expression of the reference/housekeeping gene is generally constitutive and tends not to change under different biological conditions. 18S, 28S, GAPD, ACTB, PPIB etc. have generally been considered as reference or housekeeping genes, and they have been used in normalizing gene expression data generated from different samples and/or under varying assay conditions.

In another embodiment, a special label probe set can be designed that does not bind to any capture probe or target specifically. The signal associated to this label probe can be used to establish the background of hybridization signal in individual cells. Thus the abundance of a particular target molecule can be quantitatively determined by first subtracting the background hybridization signal, then normalizing against the background subtracted reference/housekeeping gene hybridization signal.

In yet another embodiment, two or more chromosomal DNA sequences of interest can be detected simultaneously in cells. In the detection of multiple DNA sequences in cells, the label probes for the DNA sequences are distinct from each other and they do not cross-hybridize with each other. In embodiments in which cooperative indirect capture is employed, because of the design scheme, even when one probe binds to a nonspecific DNA sequence, it will not result in the capture of the signal-generating probe to the nonspecific DNA sequences.

In yet another embodiment, the detection of multiple target chromosomal DNA sequences of interest enables quantitative analysis of gene amplification, gene deletion, or gene translocations in single cells. This is accomplished by normalizing the signal of a gene of interest to that of a reference gene. The signal ratio of the gene of interest to the reference gene for a particular cell of interest is compared with the ratio in reference cells. A reference gene is defined as a gene that stably maintains its copy numbers in the genomic DNA. A reference cell is defined as a cell that contains the normal copy number of the gene of interest and the reference gene. If the signal ratio is higher in the cells of interest in comparison to the reference cells, gene amplification is detected. If the ratio is lower in the cells of interest in comparison to the reference cells, then gene deletion is detected.

### Signal Amplification & Labeling

The direct labeling approach depicted in **Figure 2** and **Figure 6** **Panel A** offers only limited sensitivity because only a relatively small number of signal-generating particles (labels) can be attached to each label probe. One way to increase sensitivity is to use in vitro transcribed RNA that incorporates signal-generating particles, but specificity will suffer as a result.

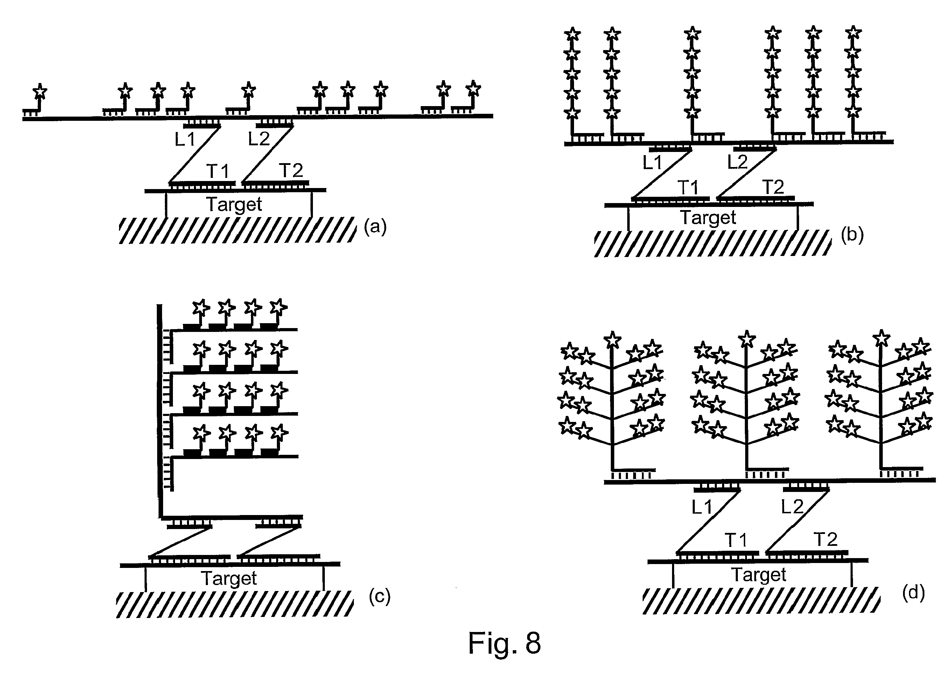

The "indirect labeling" approach not only can improve specificity as described above but also can be used to improve the detection sensitivity. In this approach, the label probe is hybridized or connected to an amplifier molecule, which provides many more attachment locations for label probes. The structure and attachment method of the amplifier can take many forms. **Figure 8** **Panels A-D** show a number of amplification schemes as illustrative examples. In **Panel A,** multiple singly-labeled label probes bind to the amplifier. In **Panel B,** multiple multiply-labeled label probes bind to the amplifier. In **Panel C,** multiple singly-labeled label probes bind to the amplifier, and multiple copies of the amplifier are bound to a preamplifier. In one particular embodiment, the amplifier is one or multiple branched DNA molecules **(Panel D).** The sequence of the label probe is preferably selected carefully so that it does not substantially cross-hybridize with any endogenous nucleic acids in the cell. In fact, the label probe does not have to be a natural polynucleotide molecule. Chemical modification of the molecule, for example, inclusion of nonnatural nucleotides, can ensure that the label probe only hybridizes to the amplifier and not to nucleic acid molecules naturally occurring in the cells. In multiplex assays, distinct amplifiers and label probes will be designed and used for the different targets.

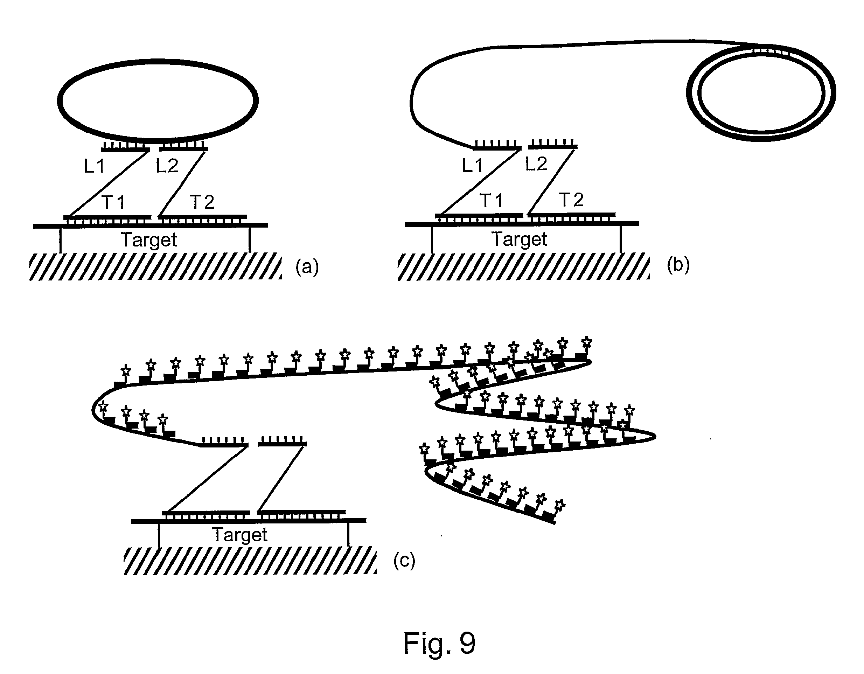

In one embodiment, as schematically illustrated in **Figure 9****,** a circular polynucleotide molecule is captured by the capture probe set. Along the circle, there can be one sequence or more than one repeat of the same sequence that binds to label probe **(****Figure 9** **Panel A).** In the signal amplification step of the assay, a rolling circle amplification procedure (Larsson et al, 2004) is carried out. As the result of this procedure, a long chain polynucleotide molecule attached to the capture probes is produced **(****Figure 9** **Panel B).** There are many repeating sequences along the chain, on which label probes can be attached by hybridization **(****Figure 9** **Panel C).** In multiplex assays, distinct capture probes, rolling circles, and label probes will be designed and used.

In one embodiment, a portion of the signal-generating probe can be PCR-amplified. In another embodiment, each portion of multiple signal-generating probes can be PCR-amplified simultaneously.

Although a specific capture approach (indirect labeling with capture probe pairs) has been used to illustrate the labeling and amplification schemes in **Figures 8** and **9****,** it is important to note that any other probe capture approaches, direct or indirect, described in previous sections can be used in combination with the labeling and amplification schemes described in these sections. The capture probe, labeling methods, and amplifier configurations described above are independent of each other and can be used in any combination in a particular assay design.

### Hybridization Conditions

The composition of the hybridization solution can affect efficiency of the hybridization process. Hybridization typically depends on the ability of the oligonucleotide to anneal to a complementary mRNA strand below its melting point (Tₘ). The value of the Tₘ is the temperature at which half of the oligonucleotide duplex is present in a single stranded form. The factors that influence the hybridization of the oligonucleotide probes to the target nucleic acids can include temperature, pH, monovalent cation concentration, presence of organic solvents, etc. A typical hybridization solution can contain some or all of the following reagents, e.g., dextran sulfate, formamide, DTT (dithiothreitol), SSC (NaCl plus sodium citrate), EDTA, etc. Other components can also be added to decrease the chance of nonspecific binding of the oligonucleotide probes, including, e.g., single-stranded DNA, tRNA acting as a carrier RNA, polyA, Denhardt's solution, etc. Exemplary hybridization conditions can be found in the art and/or determined empirically as well known in the art. See, e.g., U.S. patent application publication 2002/0172950, Player et al. (2001) J. Histochem. Cytochem. 49:603-611, and Kenny et al. (2002) J. Histochem. Cytochem. 50:1219-1227, which also describe fixation, permeabilization, and washing.

An additional prehybridization is optionally carried out to reduce background staining. Prehybridization involves incubating the fixed tissue or cells with a solution that is composed of all the elements of the hybridization solution, minus the probe.

### Washing

Following the labeling step, the cells are preferably washed to remove unbound probes or probes which have loosely bound to imperfectly matched sequences. Washing is generally started with a low stringency wash buffer such as 2 X SSC + 1 mM EDTA (1 X SSC is 0.15M NaCl, 0.015M Na-citrate), then followed by washing with higher stringency wash buffer such as 0.2 X SSC + 1 mM EDTA or 0.1 X SSC + 1 mM EDTA.

Washing is important in reducing background noise, improving signal to noise ratio of and quantification with the assay. Established washing procedures can be found, e.g., in Bauman and Bentvelzen (1988) "Flow cytometric detection of ribosomal RNA in suspended cells by fluorescent in situ hybridization" Cytometry 9(6):517-24 and Yu et al. (1992) "Sensitive detection of RNAs in single cells by flow cytometry" Nucleic Acids Res. 20(1):83-8.

Washing can be accomplished by executing a suitable number of washing cycles, i.e., one or more. Each cycle in general includes the following steps: mixing the cells with a suitable buffer solution, detaching non-specifically bound materials from the cells, and removing the buffer together with the waste. Each step is described in more detail below.

Mix the cells with wash buffer: In some assays, the cells are immobilized on the surface of a substrate before being washed. In such cases, the washing buffer is mixed together with the substrate surface. In many other embodiments, the cells to be washed are free-floating. The washing buffer is added to cell pellets or to the solution in which the cells are floating.

Detach non-specifically bound materials from cells: Any of a number of techniques can be employed here to reduce nonspecific binding after cell permeability treatment and probe hybridization to encourage non-specifically bound probes to detach from the cells and dissolve into the wash buffer. These include raising the temperature to somewhere just below the melting temperature of the specifically bound probes and employing agitation using a magnetic or mechanical stirrer or perturbation with sonic or ultrasonic waves. Agitation of the mixture can also be achieved by shaking the container with a rocking or vortex motion.

Remove buffer together with waste: Any convenient method can be employed to separate and remove the washing buffer and waste from the target cells in the sample. For example, the floating cells or substrates that the cells bound to are separated from the buffer and waste through centrifugation. After the spin, the cells or substrates form a pellet at the bottom of the container. The buffer and waste are decanted from the top.

As another example, the mixture is optionally transferred to (or formed in) a container the bottom of which is made of a porous membrane. The pore size of the membrane is chosen to be smaller than the target cells or the substrates that the cells are bound to but large enough to allow for debris and other waste materials to pass through. To remove the waste, the air or liquid pressure is optionally adjusted such that the pressure is higher inside the container than outside, thus driving the buffer and waste out of the container while the membrane retains the target cells inside. The waste can also be removed, e.g.,, by filtering the buffer and waste through the membrane driven by the force of gravity or by centrifugal force.

As yet another example, the cells can be immobilized on the surface of a large substrate, for example, a slide or the bottom of a container, through cell fixing or affinity attachment utilizing surface proteins. The buffer and waste can be removed directly by either using a vacuum to decant from the top or by turning the container upside down. As yet another example, the cells are optionally immobilized on magnetic beads, e.g., by either chemical fixing or surface protein affinity attachment. The beads can then be immobilized on the container by attaching a magnetic field on the container. The buffer and waste can then be removed directly without the loss of cells the same way as described in the previous example. As yet another example, the nonspecifically bound probes within cells are induced to migrate out of the cells by electrophoretic methods while the specifically bound probes remain.

As stated before, a washing cycle is completed by conducting each of the three steps above, and the washing procedure is accomplished by executing one or more (e.g., several) such washing cycles. Different washing buffers, detachment, or waste removal techniques may be used in different washing cycles.

### Detection

In the instant technology, the target cells that have signal-generating particles (labels) specifically hybridized to nucleic acid targets in them can be identified out of a large heterogeneous population after non-specifically bound probes and other wastes are removed through washing. Essentially any convenient method for the detection and identification can be employed.

In one embodiment, the suspension cells are immobilized onto a solid substrate after the labeling or washing step described above. The detection can be achieved using microscope based instruments. Specifically, in cases where the signal generated by the probes is chemiluminescent light, an imaging microscope with a CCD camera or a scanning microscope can be used to convert the light signal into digital information. In cases where the probe carries a label emitting a fluorescent signal, a fluorescent imaging or scanning microscope based instrument can be used for detection. In addition, since the target cells are, in general, rare among a large cell population, automatic event finding algorithms can be used to automatically identify and count the number of target cells in the population. Cells in suspension can be immobilized onto solid surfaces by any of a number of techniques. In one embodiment, a container with large flat bottom surface is used to hold the solution with the suspended cells. The container is then centrifuged to force the floating cells to settle on the bottom. If the surface is sufficiently large in comparison to the concentration of cells in the solution, cells are not likely to overlap on the bottom surface. In most cases, even if the cells overlap, the target cells will not because they are relatively rare in a large population. In another embodiment, suspended cells are cytospun onto a flat surface. After removal of fluids, the cells are immobilized on the surface by surface tension.

In preferred embodiments of this invention, cells are floating (in suspension) or are immobilized on floating substrates, such as beads, so that pre-detection procedures, such as hybridization and washing, can be carried out efficiently in solution. There are several methods to detect rare target cells out of a large floating cell population. The preferred method is to use a detection system based on the concept of flow cytometry, where the floating cells or substrates are streamlined and pass in front of excitation and detection optics one by one. The target cells are identified through the optical signal emitted by the probes specifically bound to the nucleic acid targets in the cells. The optical signal can, e.g., be luminescent light or fluorescent light of a specific wavelength.

### Advantages

In summary, the instant QMAGEX technology has a number of unique elements that enable multiplex nucleic acid detection in single cells and detection of target cells. These elements include the following.

Nucleic acid molecules immobilized inside cells are used as markers for the identification of CTC (or other cell types). Compared with protein based markers, nucleic acids are more stable, widely available, and provide better signal to noise ratio in detection. In addition, the detection technique can be readily applied to a wide range of tumors or even other applications related to cell identification or classification. As another advantage, nucleic acid molecules are quantifiably measured at an individual cell level, instead of in a mixed cell population. This feature ensures that the cell as a key functional unit in the biological system is preserved for study. In many applications involving a mixed population of cells, this feature can be very useful in extracting real, useful information out of the assay. (For example, a CTC can be identified based on detection of the presence or expression level(s) of a set of nucleic acid marker(s) in the cell; the presence or copy number of additional nucleic acids in the cell can then provide additional information useful in diagnosis, predicting outcome, or the like.)

Cells optionally remain in suspension or in pellets that can be re-suspended in all steps of the assay before final detection. This feature significantly improves assay kinetics, simplifies the process, enhances the reproducibility, and keeps the cell in its most functional relevant status. On the other hand, significant aspects of the invention, including probe selection and design, multiplexing, amplification and labeling, can be applied directly to in situ hybridization technique for the detection and enumeration of rare cells in tissue samples.

A unique indirect capture probe design approach is optionally employed to achieve exceptional target hybridization specificity, which results in better signal to noise ratio in detection.

The assays enable the detection of multiple target genes or multiple parameters on the same gene simultaneously. This feature benefits the detection of rare cells such as CTC in a number of ways. First, it can reduce the false positive rate, which is essential in cancer diagnostics. Second, it can provide additional, clinically important information related to the detected tumor cell, which may include the progression stage and/or original type and source of the primary tumor.

The invented technology incorporates a signal amplification scheme, which boosts the detection sensitivity and enables the detection of rare cells among a large number of normal cells with high confidence.

Detection can be implemented on FACS or flow cytometer based instruments or on microscope based platforms. The former can be fully automated and provides fast detection and the additional benefit of sorting out identified cells for further study, if desired. The latter platform is more widely available and has the benefit of allowing final manual identification through morphology.

### SYSTEMS

In one aspect, the invention provides systems and apparatus configured to carry out the procedures of the novel assays. The apparatus or system comprises one or more (and preferably all) of at least the following elements.

Fluid handling: The apparatus optionally includes a subsystem that can add reagents, and if required by the assay, decant fluids from the sample container (e.g., a removable or fixed, disposable or reusable container, for example a sample tube, multiwell plate, or the like). The subsystem can be based on a pipette style fluid transfer system where different fluids are handled by one pump head with disposable tips. As an alternative example, each reagent may have its own dedicated fluid channel.

Mixing and agitation: The apparatus optionally includes a device to mix different reagents in the sample solution and encourage any non-specifically bound material to detach from the cells. The device may have a mechanism to introduce a vortex or rocking motion to the holder of the sample container or to couple sound or ultrasound to the container. Alternatively, a magnetic stirrer can be put into the sample container and be driven by rotating magnetic field produced by an element installed in a holder for the container.

Temperature control: The temperature of the sample can be controlled to a level above the room temperature by installing a heater and a temperature probe to the chamber that holds the sample container. A peltier device can be used to control the temperature to a level above or below ambient. Temperature control is important, e.g., for performance of the hybridization and washing procedures in the assays.

Cell and waste fluid separation: The apparatus optionally includes a device that can remove waste fluid from the sample mixture while retaining cells for further analysis. The device may comprise a sample container that has a porous membrane as its bottom. The pore size of the membrane is smaller than the cells but larger than the waste material in the mixed solution. The space below the membrane can be sealed and connected to a vacuum pump. As an alternative example, the space above the membrane can be sealed and connected to a positive pressure source. In a different embodiment, the device can comprise a centrifuge. The container with the membrane bottom is loaded into the centrifuge, which spins to force the waste solution to filter out through the membrane. In another configuration of this device, the sample container has a solid bottom. Cells deposit at the bottom after centrifugation, and the waste solution is decanted from the top by the fluid handling subsystem described above.

This device can also perform a function that prepares the sample for final readout. In embodiments where the readout is by microscopy, the cells are typically deposited and attached to a flat surface. A centrifuge in the device can achieve this if the bottom of the container is flat. In another approach, a flat plate can spin within its plane, and the system can employ the fluid handling device to drop the solution containing the cells at the center of the spin. The cells will be evenly spun on the plate surface.

Detection: The detection element of the invented apparatus can be integrated with the rest of the system, or alternatively it can be separate from the rest of the subsystems described above. In one embodiment, the readout device is based on a microscope, which may be an imaging or scanning microscope. In another embodiment, the device is based on a fluorescent imaging or scanning microscope with multiple excitation and readout wavelengths for different probes. In a preferred embodiment, the readout device is based on flow cytometry. The cytometry approach is preferred because it can read floating cells directly out of fluid at multiple wavelengths thus greatly improving the efficiency of the assay.

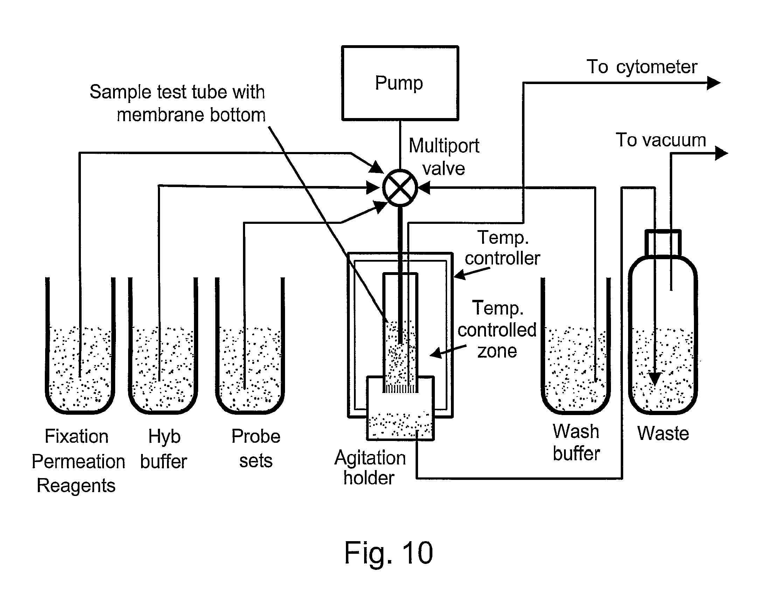

All of the above elements can be integrated into one instrument. Alternatively, these elements may be included in a number of instruments, which work together as a system to perform the assay. **Figure 10** illustrates one particular exemplary embodiment of the instrument configuration. In this particular configuration, the sample is held in a container (sample test tube) with a membrane bottom. Reagents are added from the top of the tube using a pump through a multiport valve. Waste is removed from bottom by vacuum. The holder for the sample container is fixed on an agitation table and the space around the sample is temperature controlled (temp controlled zone) by the temperature controller. The fluid handling element can introduce reagents (fixation and permeation reagents, hybridization buffer, probes sets, and wash buffer) into the sample tube, remove waste into a waste container, and feed cells to a flow cytometer for detection.

One class of embodiments provides a system comprising a holder configured to accept a sample container; a temperature controller configured to maintain the sample container at a selected temperature (e.g., a temperature selected by a user of the system or a preset temperature, different temperatures are optionally selected for different steps in an assay procedure); a fluid handling element fluidly connected to the sample container and configured to add fluid to and/or remove fluid from the sample container; a mixing element configured to mix (e.g., stir or agitate) contents of the sample container; and a detector for detecting one or more signals from within individual cells, wherein the detector is optionally fluidly connected to the sample container. One of more fluid reservoirs (e.g., for fixation or permeabilization reagents, wash buffer, probe sets, and/or waste) are optionally fluidly connected to the sample container.

A system of the invention optionally includes a computer. The computer can include appropriate software for receiving user instructions, either in the form of user input into a set of parameter fields, e.g., in a GUI, or in the form of preprogrammed instructions, e.g., preprogrammed for a variety of different specific operations. The software optionally converts these instructions to appropriate language for controlling the operation of components of the system (e.g., for controlling a fluid handling element and/or laser). The computer can also receive data from other components of the system, e.g., from a detector, and can interpret the data, provide it to a user in a human readable format, or use that data to initiate further operations, in accordance with any programming by the user.

### NUCLEIC ACID TARGETS

As noted, a nucleic acid target can be essentially any nucleic acid that is desirably detected in a cell. Choice of targets will obviously depend on the desired application, e.g., expression analysis, disease diagnosis, staging, or prognosis, target identification or validation, pathway analysis, drug screening, drug efficacy studies, or any of many other applications. Large numbers of suitable targets have been described in the art, and many more can be identified using standard techniques.

For detection of CTC, as just one example, a variety of suitable nucleic acid targets are known. For example, a multiplex panel of markers for CTC detection could include one or more of the following markers: epithelial cell-specific (e.g. CK19, Mucl, EpCAM), blood cell-specific as negative selection (e.g. CD45), tumor origin-specific (e.g. PSA, PSMA, HPN for prostate cancer and mam, mamB, her-2 for breast cancer), proliferating potential-specific (e.g. Ki-67, CEA, CA15-3), apoptosis markers (e.g. BCL-2, BCL-XL), and other markers for metastatic, genetic and epigenetic changes. As another example, targets can include HOXB13 and IL17BR mRNAs, whose ratio in primary tumor has been shown to predict clinical outcome of breast cancer patients treated with tamoxifen (Ma et al. (2004) "A two-gene expression ratio predicts clinical outcome in breast cancer patients treated with tamoxifen" Cancer Cell 5(6):607-16 and Goetz et al. (2006) "A Two-Gene Expression Ratio of Homeobox 13 and Interleukin-17B Receptor for Prediction of Recurrence and Survival in Women Receiving Adjuvant Tamoxifen" Clin Cancer Res 12:2080-2087). See also, e.g., Gewanter, R. M., A. E. Katz, et al. (2003) "RT-PCR for PSA as a prognostic factor for patients with clinically localized prostate cancer treated with radiotherapy" Urology 61(5):967-71; Giatromanolaki et al. (2004) "Assessment of highly angiogenic and disseminated in the peripheral blood disease in breast cancer patients predicts for resistance to adjuvant chemotherapy and early relapse" Int J Cancer 108(4):620-7; Halabi et al. (2003) "Prognostic significance of reverse transcriptase polymerase chain reaction for prostate-specific antigen in metastatic prostate cancer: a nested study within CALGB 9583" J Clin Oncol 21(3):490-5; Hardingham et al. (2000) "Molecular detection of blood-borne epithelial cells in colorectal cancer patients and in patients with benign bowel disease" Int J Cancer 89(1):8-13; Hayes et al. (2002) "Monitoring expression of HER-2 on circulating epithelial cells in patients with advanced breast cancer" Int J Oncol 21(5):1111-7; Jotsuka, et al. (2004) "Persistent evidence of circulating tumor cells detected by means of RT-PCR for CEA mRNA predicts early relapse: a prospective study in node-negative breast cancer" Surgery 135(4):419-26; Allen-Mersh T et al. (2003) "Colorectal cancer recurrence is predicted by RT-PCR detection of circulating cancer cells at 24 hours after primary excision" ASCO meeting, Chicago, May 2003; Shariat et al. (2003) "Early postoperative peripheral blood reverse transcription PCR assay for prostate-specific antigen is associated with prostate cancer progression in patients undergoing radical prostatectomy" Cancer Res 63(18):5874-8; Smith et al. (2000) "Response of circulating tumor cells to systemic therapy in patients with metastatic breast cancer: comparison of quantitative polymerase chain reaction and immunocytochemical techniques" J Clin Oncol 18(7):1432-9; Stathopoulou et al. (2002) "Molecular detection of cytokeratin-19-positive cells in the peripheral blood of patients with operable breast cancer: evaluation of their prognostic significance" J Clin Oncol 20(16):3404-12; and Xenidis et al. (2003) "Peripheral blood circulating cytokeratin-19 mRNA-positive cells after the completion of adjuvant chemotherapy in patients with operable breast cancer" Ann Oncol 14(6):849-55.

One preferred class of nucleic acid targets to be detected in the methods herein are those involved in cancer. Any nucleic acid that is associated with cancer can be detected in the methods of the invention, e.g., those that encode over expressed or mutated polypeptide growth factors (e.g., sis), overexpressed or mutated growth factor receptors (e.g., erb-B1), over expressed or mutated signal transduction proteins such as G-proteins (e.g., Ras), or non-receptor tyrosine kinases (e.g., abl), or over expressed or mutated regulatory proteins (e.g., myc, myb, jun, fos, etc.) and/or the like. In general, cancer can often be linked to signal transduction molecules and corresponding oncogene products, e.g., nucleic acids encoding Mos, Ras, Raf, and Met; and transcriptional activators and suppressors, e.g., p53, Tat, Fos, Myc, Jun, Myb, Rel, and/or nuclear receptors. p53, colloquially referred to as the "molecular policeman" of the cell, is of particular relevance, as about 50% of all known cancers can be traced to one or more genetic lesion in p53.

Taking one class of genes that are relevant to cancer as an example for discussion, many nuclear hormone receptors have been described in detail and the mechanisms by which these receptors can be modified to confer oncogenic activity have been worked out. For example, the physiological and molecular basis of thyroid hormone action is reviewed in Yen (2001) "Physiological and Molecular Basis of Thyroid Hormone Action" Physiological Reviews 81(3):1097-1142, and the references cited therein. Known and well characterized nuclear receptors include those for glucocorticoids (GRs), androgens (ARs), mineralocorticoids (MRs), progestins (PRs), estrogens (ERs), thyroid hormones (TRs), vitamin D (VDRs), retinoids (RARs and RXRs), and the peroxisome proliferator activated receptors (PPARs) that bind eicosanoids. The so called "orphan nuclear receptors" are also part of the nuclear receptor superfamily, and are structurally homologous to classic nuclear receptors, such as steroid and thyroid receptors. Nucleic acids that encode any of these receptors, or oncogenic forms thereof, can be detected in the methods of the invention. About 40% of all pharmaceutical treatments currently available are agonists or antagonists of nuclear receptors and/or oncogenic forms thereof, underscoring the relative importance of these receptors (and their coding nucleic acids) as targets for analysis by the methods of the invention.

One exemplary class of target nucleic acids are those that are diagnostic of colon cancer, e.g., in samples derived from stool. Colon cancer is a common disease that can be sporadic or inherited. The molecular basis of various patterns of colon cancer is known in some detail. In general, germline mutations are the basis of inherited colon cancer syndromes, while an accumulation of somatic mutations is the basis of sporadic colon cancer. In Ashkenazi Jews, a mutation that was previously thought to be a polymorphism may cause familial colon cancer. Mutations of at least three different classes of genes have been described in colon cancer etiology: oncogenes, suppressor genes, and mismatch repair genes. One example nucleic acid encodes DCC (deleted in colon cancer), a cell adhesion molecule with homology to fibronectin. An additional form of colon cancer is an autosomal dominant gene, hMSH2, that comprises a lesion. Familial adenomatous polyposis is another form of colon cancer with a lesion in the MCC locus on chromosome number 5. For additional details on colon cancer, see, Calvert et al. (2002) "The Genetics of Colorectal Cancer" Annals of Internal Medicine 137 (7): 603-612 and the references cited therein. For a variety of colon cancers and colon cancer markers that can be detected in stool, see, e.g., Boland (2002) "Advances in Colorectal Cancer Screening: Molecular Basis for Stool-Based DNA Tests for Colorectal Cancer: A Primer for Clinicans" Reviews In Gastroenterological Disorders Volume 2, Supp. 1 and the references cited therein. As with other cancers, mutations in a variety of other genes that correlate with cancer, such as Ras and p53, are useful diagnostic indicators for cancer.

Cervical cancer is another exemplary target for detection, e.g., by detection of nucleic acids that are diagnostic of such cancer in samples obtained from vaginal secretions. Cervical cancer can be caused by the papova virus (e.g., human papilloma virus) and has two oncogenes, E6 and E7. E6 binds to and removes p53 and E7 binds to and removes PRB. The loss of p53 and uncontrolled action of E2F/DP growth factors without the regulation of pRB is one mechanism that leads to cervical cancer.

Another exemplary target for detection by the methods of the invention is retinoblastoma, e.g., in samples derived from tears. Retinoblastoma is a tumor of the eyes which results from inactivation of the pRB gene. It has been found to transmit heritably when a parent has a mutated pRB gene (and, of course, somatic mutation can cause non-heritable forms of the cancer).

Neurofibromatosis Type 1 can be detected in the methods of the invention. The NF1 gene is inactivated, which activates the GTPase activity of the ras oncogene. If NF1 is missing, ras is overactive and causes neural tumors. The methods of the invention can be used to detect Neurofibromatosis Type 1 in CSF or via tissue sampling.

Many other forms of cancer are known and can be found by detecting associated genetic lesions using the methods of the invention. Cancers that can be detected by detecting appropriate lesions include cancers of the lymph, blood, stomach, gut, colon, testicles, pancreas, bladder, cervix, uterus, skin, and essentially all others for which a known genetic lesion exists. For a review of the topic, see, e.g., The Molecular Basis of Human Cancer Coleman and Tsongalis (Eds) Humana Press; ISBN: 0896036340; 1st edition (August 2001).

Similarly, nucleic acids from pathogenic or infectious organisms can be detected by the methods of the invention, e.g., for infectious fungi, e.g., *Aspergillus,* or *Candida* species; bacteria, particularly *E. coli,* which serves a model for pathogenic bacteria (and, of course certain strains of which are pathogenic), as well as medically important bacteria such as *Staphylococci* (e.g., *aureus),* or *Streptococci* (e.g., *pneumoniae*); protozoa such as sporozoa (e.g., *Plasmodia), rhizopods* (e.g., *Entamoeba*) and flagellates (*Trypanosoma*, *Leishmania*, *Trichomonas*, *Giardia,* etc.); viruses such as (+) RNA viruses (examples include Poxviruses e.g., *vaccinia;* Picornaviruses, e.g. *polio;* Togaviruses, e.g., *rubella;* Flaviviruses, e.g., HCV; and Coronaviruses), (-) RNA viruses (e.g., Rhabdoviruses, e.g., VSV; Paramyxovimses, e.g., RSV; Orthomyxovimses, e.g., influenza; Bunyaviruses; and Arenaviruses), dsDNA viruses (Reoviruses, for example), RNA to DNA viruses, i.e., Retroviruses, e.g., HIV and HTLV, and certain DNA to RNA viruses such as Hepatitis B.

As noted previously, gene amplification or deletion events can be detected at a chromosomal level using the methods of the invention, as can altered or abnormal expression levels. One preferred class of nucleic acid targets to be detected in the methods herein include oncogenes or tumor suppressor genes subject to such amplification or deletion. Exemplary nucleic acid targets include, but are not limited to, integrin (e.g., deletion), receptor tyrosine kinases (RTKs; e.g., amplification, point mutation, translocation, or increased expression), NF1 (e.g., deletion or point mutation), Akt (e.g., amplification, point mutation, or increased expression), PTEN (e.g., deletion or point mutation), MDM2 (e.g., amplification), SOX (e.g., amplification), RAR (e.g., amplification), CDK2 (e.g., amplification or increased expression), Cyclin D (e.g., amplification or translocation), Cyclin E (e.g., amplification), Aurora A (e.g., amplification or increased expression), P53 (e.g., deletion or point mutation), NBS1 (e.g., deletion or point mutation), Gli (e.g., amplification or translocation), Myc (e.g., amplification or point mutation), HPV-E7 (e.g., viral infection), and HPV-E6 (e.g., viral infection).

For embodiments in which a nucleic acid target is used as a reference, suitable reference nucleic acids have similarly been described in the art or can be determined. For example, a variety of genes whose copy number is stably maintained in various tumor cells is known in the art. Housekeeping genes whose transcripts can serve as references in gene expression analyses include, for example, 18S rRNA, 28S rRNA, GAPD, ACTB, and PPIB. Additional similar nucleic acids have been described in the art and can be adapted to the practice of the present invention.

### LABELS

A wide variety of labels are well known in the art and can be adapted to the practice of the present invention. For example, luminescent labels and light-scattering labels (e.g., colloidal gold particles) have been described. See, e.g., Csaki et al. (2002) "Gold nanoparticles as novel label for DNA diagnostics" Expert Rev Mol Diagn 2:187-93.

As another example, a number of fluorescent labels are well known in the art, including but not limited to, hydrophobic fluorophores (e.g., phycoerythrin, rhodamine, Alexa Fluor 488 and fluorescein), green fluorescent protein (GFP) and variants thereof (e.g., cyan fluorescent protein and yellow fluorescent protein), and quantum dots. See e.g., The Handbook: A Guide to Fluorescent Probes and Labeling Technologies, Tenth Edition or Web Edition (2006) from Invitrogen (available on the world wide web at probes.invitrogen.com/handbook), for descriptions of fluorophores emitting at various different wavelengths (including tandem conjugates of fluorophores that can facilitate simultaneous excitation and detection of multiple labeled species). For use of quantum dots as labels for biomolecules, see e.g., Dubertret et al. (2002) Science 298:1759; Nature Biotechnology (2003) 21:41-46; and Nature Biotechnology (2003) 21:47-51.

Labels can be introduced to molecules, e.g. polynucleotides, during synthesis or by postsynthetic reactions by techniques established in the art. For example, kits for fluorescently labeling polynucleotides with various fluorophores are available from Molecular Probes, Inc. ((www.) molecularprobes.com), and fluorophore-containing phosphoramidites for use in nucleic acid synthesis are commercially available. Similarly, signals from the labels (e.g., absorption by and/or fluorescent emission from a fluorescent label) can be detected by essentially any method known in the art. For example, multicolor detection and the like are well known in the art. Instruments for detection of labels are likewise well known and widely available, e.g., scanners, microscopes, flow cytometers, etc. For example, flow cytometers are widely available, e.g., from Becton-Dickinson ((www.) bd.com) and Beckman Coulter ((www.) beckman.com).

### MOLECULAR BIOLOGICAL TECHNIQUES

In practicing the present invention, many conventional techniques in molecular biology, microbiology, and recombinant DNA technology are optionally used. These techniques are well known and are explained in, for example, Berger and Kimmel, Guide to Molecular Cloning Techniques, Methods in Enzymology volume 152 Academic Press, Inc., San Diego, CA; Sambrook et al., Molecular Cloning - A Laboratory Manual (3rd Ed.), Vol. 1-3, Cold Spring Harbor Laboratory, Cold Spring Harbor, New York, 2000 and Current Protocols in Molecular Biology, F.M. Ausubel et al., eds., Current Protocols, a joint venture between Greene Publishing Associates, Inc. and John Wiley & Sons, Inc., (supplemented through 2006). Other useful references, e.g. for cell isolation and culture (e.g., for subsequent nucleic acid isolation) include Freshney (1994) Culture of Animal Cells, a Manual of Basic Technique, third edition, Wiley-Liss, New York and the references cited therein; Payne et al. (1992) Plant Cell and Tissue Culture in Liquid Systems John Wiley & Sons, Inc. New York, NY; Gamborg and Phillips (Eds.) (1995) Plant Cell, Tissue and Organ Culture; Fundamental Methods Springer Lab Manual, Springer-Verlag (Berlin Heidelberg New York) and Atlas and Parks (Eds.) The Handbook of Microbiological Media (1993) CRC Press, Boca Raton, FL.

### Making Polynucleotides

Methods of making nucleic acids (e.g., by in vitro amplification, purification from cells, or chemical synthesis), methods for manipulating nucleic acids (e.g., by restriction enzyme digestion, ligation, etc.) and various vectors, cell lines and the like useful in manipulating and making nucleic acids are described in the above references. In addition, methods of making branched polynucleotides (e.g., amplification multimers) are described in USPN 5;635,352, USPN 5,124,246, USPN 5,710,264, and USPN 5,849,481, as well as in other references mentioned above.

In addition, essentially any polynucleotide (including, e.g., labeled or biotinylated polynucleotides) can be custom or standard ordered from any of a variety of commercial sources, such as The Midland Certified Reagent Company ((www.) mcrc.com), The Great American Gene Company ((www.) genco.com), ExpressGen Inc. ((www.) expressgen.com), Qiagen (oligos.qiagen.com) and many others.

A label, biotin, or other moiety can optionally be introduced to a polynucleotide, either during or after synthesis. For example, a biotin phosphoramidite can be incorporated during chemical synthesis of a polynucleotide. Alternatively, any nucleic acid can be biotinylated using techniques known in the art; suitable reagents are commercially available, e.g., from Pierce Biotechnology ((www.) piercenet.com). Similarly, any nucleic acid can be fluorescently labeled, for example, by using commercially available kits such as those from Molecular Probes, Inc. ((www.) molecularprobes.com) or Pierce Biotechnology ((www.) piercenet.com) or by incorporating a fluorescently labeled phosphoramidite during chemical synthesis of a polynucleotide.

### REFERENCES :

Hess CJ, et al. Gene expression profiling of minimal residual disease in acute myeloid leukaemia by novel multiplex-PCR-based method. Leukemia. 2004 Dec;18(12):1981-8.
Vogel I et al. Detection and prognostic impact of disseminated tumor cells in pancreatic carcinoma. Pancreatology. 2002;2(2):79-88.
Gilbey AM et al. The detection of circulating breast cancer cells in blood. J Clin Pathol. 2004 Sep;57(9):903-11.
Molnar B et al. Molecular detection of circulating cancer cells. Role in diagnosis, prognosis and follow-up of colon cancer patients. Dig Dis. 2003;21(4):320-5.
Vlems FA et al. Detection and clinical relevance of tumor cells in blood and bone marrow of patients with colorectal cancer. Anticancer Res. 2003 Jan-Feb;23(1B):523-30.
Ma PC et al Circulating tumor cells and serum tumor biomarkers in small cell lung cancer. Anticancer Res. 2003 Jan-Feb;23(1A):49-62.
Mocellin S et al (2004) Molecular detection of circulating tumor cells in an independent prognostic factor in patients with high-risk cutaneous melanoma. Int J Cancer 111:741-745
Cristofanilli M. et al., (2004) Circulating tumor cells, disease progression, and survival in metastatic breast cancer. N Engl J Med. 2004 Aug 19;351(8):781-91.
Ito S et al., (2002) Quantitative detection of CEA expressing free tumor cells in the peripheral blood of colorectal cancer patients during surgery with the real-time RT-PCR on a Light Cycler, Cancer Letters, 183:195-203.
Hicks DG et al., In situ hybridization in the pathology laboratory: General principles, automation, and emerging research applications for tissue-based studies of gene expression. J Mol Histol. 2004 Aug;35(6):595-601.
Herzenberg LA et al. The history and future of the fluorescence activated cell sorter and flow cytometry: a view from Stanford. Clin Chem. 2002 Oct;48(10):1819-27.
Timm EA Jr et al. Amplification and detection of a Y-chromosome DNA sequence by fluorescence in situ polymerase chain reaction and flow cytometry using cells in suspension. Cytometry. 1995 Sep 15;22(3):250-5.
Bauman JG, Bentvelzen P. Flow cytometric detection of ribosomal RNA in suspended cells by fluorescent in situ hybridization. Cytometry. 1988 Nov;9(6):517-24.
Timm EA Jr, Stewart CC. Fluorescent in situ hybridization en suspension (FISHES) using digoxigenin-labeled probes and flow cytometry. Biotechniques. 1992 Mar;12(3):362-7.
Bains MA Flow cytometric quantitation of sequence-specific mRNA in hemopoietic cell suspensions by primer-induced in situ (PRINS) fluorescent nucleotide labeling. Exp Cell Res. 1993 Sep;208(1):321-6.
Patterson BK Detection of HIV-1 DNA and messenger RNA in individual cells by PCR-driven in situ hybridization and flow cytometry. Science. 1993 May 14;260(5110):976-9.
Rufer N Telomere length dynamics in human lymphocyte subpopulations measured by flow cytometry. Nat Biotechnol. 1998 Aug;16(8):743-7.
Hultdin M Telomere analysis by fluorescence in situ hybridization and flow cytometry. Nucleic Acids Res. 1998 Aug 15;26(16):3651-6.
Fava TA, et al Ectopic expression of guanylyl cyclase C in CD34+ progenitor cells in peripheral blood. J Clin Oncol. 2001 Oct 1;19(19):3951-9.
Kosman D, Mizutani CM, Lemons D, Cox WG, McGinnis W, Bier E. Multiplex detection of RNA expression in Drosophila embryos. Science. 2004 Aug 6;305(5685):846.
Player AN, Shen LP, Kenny D, Antao VP, Kolberg JA. Single-copy gene detection using branched DNA (bDNA) in situ hybridization. J Histochem Cytochem. 2001 May;49(5):603-12.
Schrock E, du Manoir S, Veldman T, Schoell B, Wienberg J, Ferguson-Smith MA, Ning Y, Ledbetter DH, Bar-Am I, Soenksen D, Garini Y, Ried T. Multicolor spectral karyotyping of human chromosomes. Science. 1996 Jul 26;273(5274):494-7.
Larsson C, Koch J, Nygren A, Janssen G, Raap AK, Landegren U, Nilsson M. In situ genotyping individual DNA molecules by target-primed rolling-circle amplification of padlock probes. Nat Methods. 2004 Dec;1(3):227-32. Epub 2004 Nov 18.

It is understood that the examples and embodiments described herein are for illustrative purposes only and that various modifications or changes in light thereof will be suggested to persons skilled in the art and are to be included within the spirit and purview of this application and scope of the appended claims.

While the foregoing invention has been described in some detail for purposes of clarity and understanding, it will be clear to one skilled in the art from a reading of this disclosure that various changes in form and detail can be made without departing from the true scope of the invention. For example, all the techniques and apparatus described above can be used in various combinations. All publications, patents, patent applications, and/or other documents cited in this application are incorporated by reference in their entirety for all purposes to the same extent as if each individual publication, patent, patent application, and/or other document were individually indicated to be incorporated by reference for all purposes.

Preferred items:
**1.** A method of detecting two or more nucleic acid targets in an individual cell, the method comprising:
   providing a sample comprising the cell, which cell comprises or is suspected of comprising a first nucleic acid target and a second nucleic acid target;
   providing a first label probe comprising a first label and providing a second label probe comprising a second label, wherein a first signal from the first label is distinguishable from a second signal from the second label;
   providing at least a first capture probe and at least a second capture probe;
   hybridizing, in the cell, the first capture probe to the first nucleic acid target, when present in the cell, and the second capture probe to the second nucleic acid target, when present in the cell;
   capturing the first label probe to the first capture probe and the second label probe to the second capture probe, thereby capturing the first label probe to the first nucleic acid target and the second label probe to the second nucleic acid target; and
   detecting the first signal from the first label and the second signal from the second label.
**2.** The method of item 1, wherein providing at least a first capture probe and at least a second capture probe comprises providing a single first capture probe and a single second capture probe; wherein capturing the first label probe to the first capture probe comprises hybridizing the first label probe to the first capture probe; and wherein capturing the second label probe to the second capture probe comprises hybridizing the second label probe to the second capture probe.
**3.** The method of item **1,** wherein providing at least a first capture probe comprises providing two or more first capture probes; wherein providing at least a second capture probe comprises providing two or more second capture probes; wherein providing a first label probe and a second label probe comprises providing a plurality of the first label probes and a plurality of the second label probes; wherein hybridizing the first capture probe to the first nucleic acid target and the second capture probe to the second nucleic acid target comprises hybridizing the two or more first capture probes to the first nucleic acid target and the two or more second capture probes to the second nucleic acid target; and wherein capturing the first label probe to the first capture probe and the second label probe to the second capture probe comprises hybridizing a single first label probe to each of the first capture probes and a single second label probe to each of the second capture probes.
**4.** The method of item **1,** wherein providing at least a first capture probe and at least a second capture probe comprises providing a single first capture probe and a single second capture probe; wherein providing a first label probe and a second label probe comprises providing a plurality of the first label probes and a plurality of the second label probes; wherein capturing the first label probe to the first capture probe comprises hybridizing a first amplifier to the first capture probe and to the plurality of first label probes; and wherein capturing the second label probe to the second capture probe comprises hybridizing a second amplifier to the second capture probe and to the plurality of second label probes.
**5.** The method of item **1,** wherein providing at least a first capture probe comprises providing two or more first capture probes; wherein providing at least a second capture probe comprises providing two or more second capture probes; wherein providing a first label probe and a second label probe comprises providing a plurality of the first label probes and a plurality of the second label probes; wherein hybridizing the first capture probe to the first nucleic acid target and the second capture probe to the second nucleic acid target comprises hybridizing the two or more first capture probes to the first nucleic acid target and the two or more second capture probes to the second nucleic acid target; wherein capturing the first label probe to the first capture probe comprises hybridizing a first amplifier to each of the first capture probes and hybridizing the plurality of first label probes to the first amplifiers; and wherein capturing the second label probe to the second capture probe comprises hybridizing a second amplifier to each of the second capture probes and hybridizing the plurality of second label probes to the second amplifiers.
**6.** The method of item **1,** wherein providing at least a first capture probe and at least a second capture probe comprises providing a single first capture probe and a single second capture probe; wherein providing a first label probe and a second label probe comprises providing a plurality of the first label probes and a plurality of the second label probes; wherein capturing the first label probe to the first capture probe comprises hybridizing a first preamplifier to the first capture probe, hybridizing a plurality of first amplifiers to the first preamplifier, and hybridizing the plurality of first label probes to the first amplifiers; and wherein capturing the second label probe to the second capture probe comprises hybridizing a second preamplifier to the second capture probe, hybridizing a plurality of second amplifiers to the second preamplifier, and hybridizing the plurality of second label probes to the second amplifiers.
**7.** The method of item **1,** wherein providing at least a first capture probe comprises providing two or more first capture probes; wherein providing at least a second capture probe comprises providing two or more second capture probes; wherein providing a first label probe and a second label probe comprises providing a plurality of the first label probes and a plurality of the second label probes; wherein hybridizing the first capture probe to the first nucleic acid target and the second capture probe to the second nucleic acid target comprises hybridizing the two or more first capture probes to the first nucleic acid target and the two or more second capture probes to the second nucleic acid target; wherein capturing the first label probe to the first capture probe comprises hybridizing a first preamplifier to each of the first capture probes, hybridizing a plurality of first amplifiers to each of the first preamplifiers, and hybridizing the plurality of first label probes to the first amplifiers; and wherein capturing the second label probe to the second capture probe comprises hybridizing a second preamplifier to each of the second capture probes, hybridizing a plurality of second amplifiers to each of the second preamplifiers, and hybridizing the plurality of second label probes to the second amplifiers.
**8.** The method of item **1,** wherein providing at least a first capture probe comprises providing two or more first capture probes, which first capture probes hybridize to nonoverlapping polynucleotide sequences in the first nucleic acid target; and wherein providing at least a second capture probe comprises providing two or more second capture probes, which second capture probes hybridize to nonoverlapping polynucleotide sequences in the second nucleic acid target.
**9.** The method of item **1,** wherein providing a first label probe and a second label probe comprises providing a plurality of the first label probes and a plurality of the second label probes;
   wherein capturing the first label probe to the first capture probe comprises
   a) producing a first amplified polynucleotide by rolling circle amplification of a first circular polynucleotide hybridized to the first capture probe, which first circular polynucleotide comprises at least one copy of a polynucleotide sequence identical to a polynucleotide sequence in the first label probe, and which first amplified polynucleotide comprises a plurality of copies of a polynucleotide sequence complementary to the polynucleotide sequence in the first label probe, and
   b) hybridizing the plurality of first label probes to the first amplified polynucleotide; and
   wherein capturing the second label probe to the second capture probe comprises
   a) producing a second amplified polynucleotide by rolling circle amplification of a second circular polynucleotide hybridized to the second capture probe, which second circular polynucleotide comprises at least one copy of a polynucleotide sequence identical to a polynucleotide sequence in the second label probe, and which second amplified polynucleotide comprises a plurality of copies of a polynucleotide sequence complementary to the polynucleotide sequence in the second label probe, and
   b) hybridizing the plurality of second label probes to the second amplified polynucleotide.
**10.** The method of item **1,** wherein the cell comprises or is suspected of comprising a third nucleic acid target; the method comprising: providing a third label probe comprising a third label, wherein a third signal from the third label is distinguishable from the first and second signals, providing at least a third capture probe, hybridizing in the cell the third capture probe to the third nucleic acid target, when present in the cell, capturing the third label probe to the third capture probe, and detecting the third signal from the third label.
**11.** The method of item **1,** wherein each hybridization or capture step is accomplished for all of the nucleic acid targets at the same time.
**12.** The method of item **1,** wherein the first nucleic acid target and the second nucleic acid target are independently selected from the group consisting of: a DNA, an RNA, a chromosomal DNA, an mRNA, a nucleic acid endogenous to the cell, and a nucleic acid introduced to or expressed in the cell by infection of the cell with a pathogen.
**13.** The method of item **1,** wherein the first nucleic acid target is a first mRNA and wherein the second nucleic acid target is a second mRNA.
**14.** The method of item **1,** wherein the first nucleic acid target comprises a first chromosomal DNA polynucleotide sequence and wherein the second nucleic acid target comprises a second chromosomal DNA polynucleotide sequence.
**15.** The method of item **1,** wherein the first nucleic acid target comprises a first region of an mRNA and wherein the second nucleic acid target comprises a second region of the same mRNA.
**16.** The method of item **1,** wherein the second nucleic acid target comprises a reference nucleic acid; the method comprising normalizing the first signal to the second signal.
**17.** The method of item **1**, wherein the first label is a first fluorescent label and the second label is a second fluorescent label.
**18.** The method of item **1,** wherein detecting the first signal and the second signal comprises measuring an intensity of the first signal and an intensity of the second signal; the method comprising correlating the intensity of the first signal and the second signal with a quantity of the corresponding nucleic acid target present in the cell.
**19.** The method of item **1**, wherein the cell is a circulating tumor cell.
**20.** The method of item **1**, wherein the sample comprising the cell is derived from a bodily fluid or from blood.
**21.** The method of item **1,** the method comprising identifying the cell as a desired target cell based on detection of the first and second signals from within the cell.
**22.** The method of item 1, wherein providing a sample comprising the cell comprises fixing and permeabilizing the cell.
**23.** The method of item 1, the method comprising washing the cell to remove materials not captured to one of the nucleic acid targets.
**24.** The method of item **1,** wherein the first nucleic acid target and/or the second nucleic acid target comprises a double-stranded DNA molecule, the method comprising denaturing the double-stranded DNA prior to hybridization of the first and second capture probes to the first and second nucleic acid targets.
**25.** The method of item **1,** wherein the cell is in suspension in the sample comprising the cell, and/or wherein the cell is in suspension during the hybridizing, capturing, and/or detecting steps.
**26.** The method of item **1,** wherein detecting the first signal from the first label and the second signal from the second label comprises detecting the first signal from the first label and the second signal from the second label in a single operation.
**27.** The method of item **1,** wherein detecting the first and second signals comprises performing flow cytometry.
**28.** A method of assaying a relative level of one or more target nucleic acids in an individual cell, the method comprising:
   providing a sample comprising the cell, which cell comprises or is suspected of comprising a first, target nucleic acid, and which cell comprises a second, reference nucleic acid;
   providing a first label probe comprising a first label and providing a second label probe comprising a second label, wherein a first signal from the first label is distinguishable from a second signal from the second label;
   capturing, in the cell, the first label probe to the first, target nucleic acid, when present in the cell, and the second label probe to the second, reference nucleic acid;
   detecting the first signal from the first label and the second signal from the second label in the individual cell, wherein detecting the first signal and the second signal comprises measuring an intensity of the first signal and an intensity of the second signal; and
   normalizing the intensity of the first signal to the intensity of the second signal.
**29.** The method of item **28,** wherein capturing the first label probe to the first, target nucleic acid comprises hybridizing the first label probe to the first, target nucleic acid.
**30.** The method of item **28,** wherein capturing the second label probe to the second, reference nucleic acid comprises hybridizing the second label probe to the second, reference nucleic acid.
**31.** The method of item **28,** wherein capturing the first label probe to the first, target nucleic acid and the second label probe to the second, reference nucleic acid comprises:
   providing at least a first capture probe and at least a second capture probe;
   hybridizing, in the cell, the first capture probe to the first, target nucleic acid and the second capture probe to the second, reference nucleic acid; and
   capturing the first label probe to the first capture probe and the second label probe to the second capture probe, thereby capturing the first label probe to the first, target nucleic acid and the second label probe to the second, reference nucleic acid.
**32.** The method of item **28,** wherein the cell comprises or is suspected of comprising a third, target nucleic acid; the method comprising:
   providing a third label probe comprising a third label, wherein a third signal from the third label is distinguishable from the first and second signals,
   capturing, in the cell, the third label probe to the third, target nucleic acid, when present in the cell,
   detecting the third signal from the third label, which detecting comprises measuring an intensity of the third signal, and
   normalizing the intensity of the third signal to the intensity of the second signal.
**33.** The method of item **28,** wherein the first, target nucleic acid and the second, reference nucleic acid are independently selected from the group consisting of: a DNA, an RNA, a chromosomal DNA, an mRNA, a nucleic acid endogenous to the cell, and a nucleic acid introduced to or expressed in the cell by infection of the cell with a pathogen.
**34.** The method of item **28,** wherein the first, target nucleic acid is a first mRNA and wherein the second, reference nucleic acid is a second mRNA.
**35.** The method of item **28,** wherein the first, target nucleic acid comprises a first chromosomal DNA polynucleotide sequence and wherein the second, reference nucleic acid comprises a second chromosomal DNA polynucleotide sequence.
**36.** The method of item **28,** wherein the first label is a first fluorescent label and the second label is a second fluorescent label.
**37.** The method of item **28,** the method comprising correlating the intensity of the first signal normalized to that of the second signal with a quantity of the first, target nucleic acid present in the cell.
**38.** The method of item **28,** wherein the cell is a circulating tumor cell.
**39.** The method of item **28,** wherein the sample comprising the cell is derived from a bodily fluid or from blood.
**40.** The method of item **28,** the method comprising identifying the cell as a desired target cell based on the normalized first signal.
**41.** The method of item **28,** wherein providing a sample comprising the cell comprises fixing and permeabilizing the cell.
**42.** The method of item **28,** the method comprising washing the cell to remove materials not captured to one of the target or reference nucleic acids.
**43.** The method of item **28,** wherein the first, target nucleic acid and/or the second, reference nucleic acid comprises a double-stranded DNA molecule, the method comprising denaturing the double-stranded DNA prior to capturing the first and second label probes to the first and second nucleic acids.
**44.** The method of item **28,** wherein the cell is in suspension in the sample comprising the cell, and/or wherein the cell is in suspension during the capturing and/or detecting steps.
**45.** The method of item **28,** wherein detecting the first signal from the first label and the second signal from the second label in the individual cell comprises detecting the first signal from the first label and the second signal from the second label in the individual cell in a single operation.
**46.** The method of item **28,** wherein detecting the first and second signals comprises performing flow cytometry.
**47.** A method of detecting an individual cell of a specified type, the method comprising:
   providing a sample comprising a mixture of cell types, which mixture comprises at least one cell of the specified type;
   providing a first label probe comprising a first label and providing a second label probe comprising a second label, wherein a first signal from the first label is distinguishable from a second signal from the second label;
   capturing, in the cell, the first label probe to a first nucleic acid target, when present in the cell, and the second label probe to a second nucleic acid target, when present in the cell;
   detecting the first signal from the first label and the second signal from the second label;
   correlating the first and second signals detected from the cell with the presence, absence, or amount of the first and second nucleic acid targets in the cell; and
   identifying the cell as being of the specified type based on detection of the presence, absence, or amount of both the first and second nucleic acid targets within the cell,
   wherein the specified type of cell is distinguishable from the other cell type(s) in the mixture on the basis of either the presence, absence, or amount of the first nucleic acid target or the presence, absence, or amount of the second nucleic acid target in the cell.
**48.** The method of item **47,** wherein the cell comprises the first nucleic acid target and the second nucleic acid target, the method comprising identifying the cell as being of the specified type based on detection of the presence or amount of both the first and second nucleic acid targets within the cell, wherein the specified type of cell is distinguishable from the other cell type(s) in the mixture on the basis of either the presence or amount of the first nucleic acid target or the presence or amount of the second nucleic acid target in the cell.
**49.** The method of item **47,** wherein capturing the first label probe to the first nucleic acid target comprises hybridizing the first label probe to the first nucleic acid target.
**50.** The method of item **47,** wherein capturing the second label probe to the second nucleic acid target comprises hybridizing the second label probe to the second nucleic acid target.
**51.** The method of item **47,** wherein capturing the first label probe to the first nucleic acid target and the second label probe to the second nucleic acid target comprises:
   providing at least a first capture probe and at least a second capture probe;
   hybridizing, in the cell, the first capture probe to the first nucleic acid target and the second capture probe to the second nucleic acid target; and
   capturing the first label probe to the first capture probe and the second label probe to the second capture probe, thereby capturing the first label probe to the first nucleic acid target and the second label probe to the second nucleic acid target.
**52.** The method of item **47,** the method comprising: providing a third label probe comprising a third label, wherein a third signal from the third label is distinguishable from the first and second signals, capturing in the cell the third label probe to a third nucleic acid target, when present in the cell, and detecting the third signal from the third label.
**53.** The method of item **52,** wherein the cell comprises the third nucleic acid target, the method comprising normalizing the first and/or second signal to the third signal.
**54.** The method of item. **53,** the method comprising identifying the cell as being of the specified type based on the normalized first and/or second signal.
**55.** The method of item **52,** the method comprising correlating the third signal detected from the cell with the presence, absence, or amount of the third nucleic acid target in the cell; wherein identifying the cell as being of the specified type based on detection of the presence, absence, or amount of both the first and second nucleic acid targets within the cell comprises identifying the cell as being of the specified type based on detection of the presence, absence, or amount of the first, second, and third nucleic acid targets within the cell, wherein the specified type of cell is distinguishable from the other cell type(s) in the mixture on the basis of either presence, absence, or amount of the first nucleic acid target, presence, absence, or amount of the second nucleic acid target, or presence, absence, or amount of the third nucleic acid target in the cell.
**56.** The method of item **47**, wherein the first nucleic acid target and the second nucleic acid target are independently selected from the group consisting of: a DNA, an RNA, a chromosomal DNA, an mRNA, a nucleic acid endogenous to the cell, and a nucleic acid introduced to or expressed in the cell by infection of the cell with a pathogen.
**57.** The method of item **47,** wherein the first nucleic acid target is a first mRNA and wherein the second nucleic acid target is a second mRNA.
**58.** The method of item **47,** wherein the first nucleic acid target comprises a first region of an mRNA and wherein the second nucleic acid target comprises a second region of the same mRNA.
**59.** The method of item **47,** wherein the first nucleic acid target comprises a first chromosomal DNA polynucleotide sequence and wherein the second nucleic acid target comprises a second chromosomal DNA polynucleotide sequence.
**60.** The method of item **47,** wherein the first label is a first fluorescent label and the second label is a second fluorescent label.
**61.** The method of item **47,** wherein detecting the first signal and the second signal comprises measuring an intensity of the first signal and an intensity of the second signal; the method comprising correlating the intensity of the first signal and the second signal with a quantity of the corresponding nucleic acid present in the cell.
**62.** The method of item **47**, wherein the cell is a circulating tumor cell.
**63.** The method of item **47,** wherein the sample comprising the cell is derived from a bodily fluid or from blood.
**64.** The method of item. **47,** wherein the ratio of cells of the specified type to cells of all other type(s) in the mixture is less than 1:1x10⁴, less than 1:1x10⁵, less than 1:1x10⁶, less than 1:1x10⁷, or less than 1:1x10⁸.
**65.** The method of item **47,** wherein providing a sample comprising a mixture of cell types comprises fixing and permeabilizing the cells constituting the mixture of cell types.
**66.** The method of item **47,** the method comprising washing the cell to remove materials not captured to one of the nucleic acid targets.
**67.** The method of item **47,** wherein the cell is in suspension in the sample and/or wherein the cell is in suspension during the capturing and/or detecting steps.
**68.** The method of item **47,** wherein detecting the first signal from the first label and the second signal from the second label comprises detecting the first signal from the first label and the second signal from the second label in a single operation.
**69.** The method of item **47,** wherein detecting the first and second signals comprises performing flow cytometry.
**70.** A composition, comprising:
   a fixed and permeabilized cell, which cell comprises or is suspected of comprising a first nucleic acid target and a second nucleic acid target;
   at least a first capture probe capable of hybridizing to the first nucleic acid target;
   at least a second capture probe capable of hybridizing to the second nucleic acid target; and
   a first label probe comprising a first label and a second label probe comprising a second label, wherein a first signal from the first label is distinguishable from a second signal from the second label.
**71.** The composition of item **70,** comprising a single first capture probe and a single second capture probe; wherein the first label probe is capable of hybridizing to the first capture probe; and wherein the second label probe is capable of hybridizing to the second capture probe.
**72.** The composition of item **70,** comprising two or more first capture probes, two or more second capture probes, a plurality of the first label probes, and a plurality of the second label probes; wherein a single first label probe is capable of hybridizing to each of the first capture probes and a single second label probe is capable of hybridizing to each of the second capture probes.
**73.** The composition of item **70,** comprising a single first capture probe, a single second capture probe, a plurality of the first label probes, a plurality of the second label probes, a first amplifier, and a second amplifier; wherein the first amplifier is capable of hybridizing to the first capture probe and to the plurality of first label probes; and wherein the second amplifier is capable of hybridizing to the second capture probe and to the plurality of second label probes.
**74.** The composition of item **70,** comprising two or more first capture probes, two or more second capture probes, a multiplicity of the first label probes, a multiplicity of the second label probes, a first amplifier, and a second amplifier; wherein the first amplifier is capable of hybridizing to one of the first capture probes and to a plurality of first label probes; and wherein the second amplifier is capable of hybridizing to one of the second capture probes and to a plurality of second label probes.
**75.** The composition of item **70,** comprising a single first capture probe, a single second capture probe, a multiplicity of the first label probes, a multiplicity of the second label probes, a plurality of first amplifiers, a plurality of second amplifiers, a first preamplifier, and a second preamplifier; wherein the first preamplifier is capable of hybridizing to the first capture probe and to the plurality of first amplifiers; wherein the second preamplifier is capable of hybridizing to the second capture probe and to the plurality of second amplifiers; wherein the first amplifier is capable of hybridizing to the first preamplifier and to a plurality of first label probes; and wherein the second amplifier is capable of hybridizing to the second preamplifier and to a plurality of second label probes.
**76.** The composition of item **70,** comprising two or more first capture probes, two or more second capture probes, a multiplicity of the first label probes, a multiplicity of the second label probes, a multiplicity of first amplifiers, a multiplicity of second amplifiers, a plurality of first preamplifiers, and a plurality of second preamplifiers; wherein the first preamplifier is capable of hybridizing to one of the first capture probes and to a plurality of first amplifiers; wherein the second preamplifier is capable of hybridizing to one of the second capture probes and to a plurality of second amplifiers; wherein the first amplifier is capable of hybridizing to the first preamplifier and to a plurality of first label probes; and wherein the second amplifier is capable of hybridizing to the second preamplifier and to a plurality of second label probes.
**77.** The composition of item **70,** comprising a plurality of the first label probes; a plurality of the second label probes; a first amplified polynucleotide produced by rolling circle amplification of a first circular polynucleotide hybridized to the first capture probe, which first circular polynucleotide comprises at least one copy of a polynucleotide sequence identical to a polynucleotide sequence in the first label probe, and which first amplified polynucleotide comprises a plurality of copies of a polynucleotide sequence complementary to the polynucleotide sequence in the first label probe; and a second amplified polynucleotide produced by rolling circle amplification of a second circular polynucleotide hybridized to the second capture probe, which second circular polynucleotide comprises at least one copy of a polynucleotide sequence identical to a polynucleotide sequence in the second label probe, and which second amplified polynucleotide comprises a plurality of copies of a polynucleotide sequence complementary to the polynucleotide sequence in the second label probe.
**78.** The composition of item **70,** wherein the cell comprises or is suspected of comprising a third nucleic acid target; the composition comprising: a third label probe comprising a third label, wherein a third signal from the third label is distinguishable from the first and second signals, and at least a third capture probe capable of hybridizing to the third nucleic acid target.
**79.** The composition of item **70,** wherein the first nucleic acid target and the second nucleic acid target are independently selected from the group consisting of: a DNA, an RNA, a chromosomal DNA, an mRNA, a nucleic acid endogenous to the cell, and a nucleic acid introduced to or expressed in the cell by infection of the cell with a pathogen.
**80.** The composition of item **70,** wherein the first nucleic acid target is a first mRNA and wherein the second nucleic acid target is a second mRNA.
**81.** The composition of item **70,** wherein the first nucleic acid target comprises a first chromosomal DNA polynucleotide sequence and wherein the second nucleic acid target comprises a second chromosomal DNA polynucleotide sequence.
**82.** The composition of item **70,** wherein the first nucleic acid target comprises a first region of an mRNA and wherein the second nucleic acid target comprises a second region of the same mRNA.
**83.** The composition of item **70,** wherein the second nucleic acid target comprises a reference nucleic acid.
**84.** The composition of item **70,** wherein the first label is a first fluorescent label and the second label is a second fluorescent label.
**85.** The composition of item **70,** wherein the cell is a circulating tumor cell.
**86.** The composition of item **70,** wherein the cell is derived from a bodily fluid or from blood.
**87.** The composition of item **70,** wherein the cell is of a specified type, the composition comprising one or more other types of cells.
**88.** The composition of item **87,** wherein the ratio of cells of the specified type to cells of all other type(s) in the composition is less than 1:1x10⁴, less than 1:1x10⁵, less than 1:1x10⁶, less than 1:1x10⁷, or less than 1:1x10⁸.
**89.** The composition of item **70,** wherein the cell is in suspension in the composition.
**90.** A kit for detecting a first nucleic acid target and a second nucleic acid target in an individual cell, comprising:
   at least one reagent for fixing and/or permeabilizing the cell;
   at least a first capture probe capable of hybridizing to the first nucleic acid target;
   at least a second capture probe capable of hybridizing to the second nucleic acid target; and
   a first label probe comprising a first label and a second label probe comprising a second label, wherein a first signal from the first label is distinguishable from a second signal from the second label;
   packaged in one or more containers.
**91.** A kit for detecting an individual cell of a specified type from a mixture of cell types by detecting a first nucleic acid target and a second nucleic acid target, the kit comprising:
   at least one reagent for fixing and/or permeabilizing the cell;
   a first label probe comprising a first label and a second label probe comprising a second label, wherein a first signal from the first label is distinguishable from a second signal from the second label;
   wherein the specified type of cell is distinguishable from the other cell type(s) in the mixture by presence, absence, or amount of the first nucleic acid target in the cell or by presence, absence, or amount of the second nucleic acid target in the cell;
   packaged in one or more containers.
**92.** A method of detecting two or more nucleic acid targets in an individual cell, the method comprising:
   providing a sample comprising the cell, which cell comprises or is suspected of comprising a first nucleic acid target and a second nucleic acid target;
   capturing, in the cell, a first label to the first nucleic acid target, when present in the cell, and a second label to the second nucleic acid target, when present in the cell, wherein a first signal from the first label is distinguishable from a second signal from the second label, wherein an average of at least one copy of the first label per nucleotide of the first nucleic acid target is captured to the first nucleic acid target over a region that spans at least 20 contiguous nucleotides of the first nucleic acid target, and wherein an average of at least one copy of the second label per nucleotide of the second nucleic acid target is captured to the second nucleic acid target over a region that spans at least 20 contiguous nucleotides of the second nucleic acid target; and
   detecting the first signal from the first label and the second signal from the second label.
**93.** The method of item **92,** wherein an average of at least four, eight, or twelve copies of the first label per nucleotide of the first nucleic acid target are captured to the first nucleic acid target over a region that spans at least 20 contiguous nucleotides of the first nucleic acid target, and wherein an average of at least four, eight, or twelve copies of the second label per nucleotide of the second nucleic acid target are captured to the second nucleic acid target over a region that spans at least 20 contiguous nucleotides of the second nucleic acid target.
**94.** The method of item **92,** wherein an average of at least sixteen copies of the first label per nucleotide of the first nucleic acid target are captured to the first nucleic acid target over a region that spans at least 20 contiguous nucleotides of the first nucleic acid target, and wherein an average of at least sixteen copies of the second label per nucleotide of the second nucleic acid target are captured to the second nucleic acid target over a region that spans at least 20 contiguous nucleotides of the second nucleic acid target.
**95.** A composition comprising: a cell, which cell comprises:
   a first nucleic acid target;
   a second nucleic acid target;
   a first label whose presence in the cell is indicative of the presence of the first nucleic acid target in the cell, wherein an average of at least one copy of the first label is present in the cell per nucleotide of the first nucleic acid target over a region that spans at least 20 contiguous nucleotides of the first nucleic acid target; and
   a second label whose presence in the cell is indicative of the presence of the second nucleic acid target in the cell, wherein an average of at least one copy of the second label is present in the cell per nucleotide of the second nucleic acid target over a region that spans at least 20 contiguous nucleotides of the second nucleic acid target;
   wherein a first signal from the first label is distinguishable from a second signal from the second label.
**96.** The composition of item **95,** wherein the copies of the first label are physically associated with the first nucleic acid target, and wherein the copies of the second label are physically associated with the second nucleic acid target.
**97.** A method of performing comparative gene expression analysis in single cells, the method comprising:
   providing a first mixed cell population comprising one or more cells of a specified type;
   providing a second mixed cell population comprising one or more cells of the specified type;
   measuring an expression level of one or more target nucleic acids relative to a reference nucleic acid in the cells of the specified type of the first population, to provide a first expression profile;
   measuring an expression level of the one or more target nucleic acids relative to the reference nucleic acid in the cells of the specified type of the second population, to provide a second expression profile; and
   comparing the first and second expression profiles.

## Claims

1. A method of detecting a nucleic acid target within a cell in a sample, the method comprising:
(a) providing
(i) one or more label probes, wherein each label probe comprises one or more labels;
(ii) one or more label probes, and one or more amplifiers, wherein each label probe comprises one or more labels, and wherein each amplifier is capable of hybridizing to said one or more label probes; or
(iii) one or more label probes, one or more amplifiers, and one or more preamplifiers, wherein each label probe comprises one or more labels, wherein each amplifier is capable of hybridizing to said one or more label probes, and wherein each preamplifier is capable of hybridizing to said one or more amplifiers;
(b) providing two or more capture probes, wherein each of said two or more capture probes comprises a section T complementary to a section on the nucleic acid target and a section L complementary to a section on (i) said label probe, or (ii) said amplifier, or (iii) said preamplifier;
(c) hybridizing, in the cell, the two or more capture probes to a single copy of the nucleic acid target, when present in the cell;
(d) capturing, in the cell, the label probe to the nucleic acid target, when present in the cell, by hybridizing:
(i) said two or more capture probes to a single copy of said label probe;
(ii) said two or more capture probes to a single copy of said amplifier and said one or more label probes to said amplifier; or
(iii) said two or more capture probes to a single copy of said preamplifier, said one or more amplifiers to said preamplifier and said one or more label probes to each of said one or more amplifiers;
(e) detecting a signal from the label; and
(f) correlating the signal detected from the label with the presence, absence, or amount of the nucleic acid target in the cell.

2. The method of claim 1, further comprising fixing and permeabilizing the cell.

3. The method of claim 1 or 2, further comprising identifying the cell as being of a desired type based on the presence, absence, or amount of the nucleic acid target in the cell.

4. The method of claim 3, wherein the cell of the desired type is contained in a mixture of cells, and wherein the ratio of cells of the desired type to cells of all other types in the mixture is preferably less than 1:1x10⁴, less than 1:1x10⁵, less than 1:1x10⁶, less than 1:1x10⁷, or less than 1:1x10⁸.

5. The method of any one of claims 1 to 4, wherein the cell is a tumor cell, preferably a circulating tumor cell or a residual tumor cell.

6. The method of any one of claims 1 to 5, wherein the cell is:
(i) in suspension, preferably wherein the cell is in suspension during the hybridizing, capturing, and/or detecting steps;
(ii) in solid tissue sample, preferably solid tissue section, further preferably formalin-fixed, paraffin embedded tissue sample; or
(iii) immobilized on a substrate, preferably on a slide.

7. The method of any one of claims 1 to 6, wherein the sample a bodily fluid, bodily waste, blood, tissue, biopsy, or tumor.

8. The method of any one of claims 1 to 7, wherein the nucleic acid target is:
(i) a DNA, a RNA, a chromosomal DNA, an mRNA, a microRNA, or a ribosomalRNA; and/or
(ii) a nucleic acid endogenous to the cell, or a nucleic acid introduced to or expressed in the cell by infection of the cell with a pathogen.

9. The method of any one of claims 1 to 8, wherein the melting temperature Tₘ of the T section of each of said two or more capture probes is above the hybridization temperature, and wherein the melting temperature Tₘ of the L section of each of said two or more capture probes is below the hybridization temperature.

10. The method of any one of claims 1 to 8, wherein the melting temperature Tₘ of the T section of each of said two or more capture probes is below the hybridization temperature, and wherein the melting temperature Tₘ of the L section of each of said two or more capture probes is above the hybridization temperature.

11. The method of any of the claims 1 to 10, wherein the method comprises detecting a plurality of nucleic acid targets within the cell, wherein each nucleic acid target has its associating label probe, two or more capture probes, and optionally preamplifier and/or amplifier; and each label probe comprises a label, and wherein said hybridizing or capturing step is preferably accomplished for all of the nucleic acid targets at the same time.

12. The method of claim 11, wherein the plurality of nucleic acid targets are different regions of a single nucleic acid molecule, or different nucleic acid molecules.

13. A kit for detecting a nucleic acid target in a cell, comprising in one or more containers:
(a) at least on reagent for fixing and/or premeabilizing the cell;
(b)
(i) one or more label probes, wherein each label probe comprises one or more labels,
(ii) one or more label probes, and one or more amplifiers, wherein each label probe comprises one or more labels, and wherein each amplifier is capable of hybridizing to said one or more label probes; or
(iii) one or more label probes, one or more amplifiers, and one or more preamplifiers, wherein each label probe comprises one or more labels, wherein each amplifier is capable of hybridizing to said one or more label probes, and wherein each preamplifier is capable of hybridizing to said one or more amplifiers;
(c) two or more capture probes, wherein each of said two or more capture probes comprises a section T complementary to a section on the nucleic acid target and a section L complementary to a section on (i) said label probe, or (ii) said amplifier, or (iii) said preamplifier.

14. A composition comprising a fixed and permeabilized call, wherein the cell comprises or is suspected of comprising a nucleic acid target and wherein the cell comprises:
(a)
(i) one or more label probes, wherein each label probe comprises one or more labels;
(ii) one or more label probes, and one or more amplifiers, wherein each label probe comprises one or more labels, and wherein each amplifier is capable of hybridizing to said one or more label probes; or
(iii) one or more label probes, one or more amplifiers, and one or more preamplifiers, wherein each label probe comprises one or more labels, wherein each amplifier is capable of hybridizing to said one or more label probes, and wherein each preamplifier is capable of hybridizing to said one or more amplifiers;
(b) two or more capture probes, wherein each of said two or more capture probes comprises a section T complementary to a section on the nucleic acid target and a section L complementary to a section on (i) said label probe, or (ii) said amplifier, or (iii) said preamplifier.

15. A tissue section comprising a fixed and permeabilized call, wherein the cell comprises or is suspected of comprising a nucleic acid target and wherein the cell comprises:
(a)
(i) one or more label probes, wherein each label probe comprises one or more labels,
(ii) one or more label probes, and one or more amplifiers, wherein each label probe comprises one or more labels, and wherein each amplifier is capable of hybridizing to said one or more label probes; or
(iii) one or more label probes, one or more amplifiers, and one or more preamplifiers, wherein each label probe comprises one or more labels, wherein each amplifier is capable of hybridizing to said one or more label probes, and wherein each preamplifier is capable of hybridizing to said one or more amplifiers;
(b) two or more capture probes, wherein each of said two or more capture probes comprises a section T complementary to a section on the nucleic acid target and a section L complementary to a section on (i) said label probe, or (ii) said amplifier, or (iii) said preamplifier.

16. The composition of claim 14, or the tissue section of claim 15, wherein the cell is as defined in claims 5 or 6.
